(19) Europäisches Patentamt
European Patent Office
Office européen des brevets

(11) Veröffentlichungsnummer: **0 273 239 B1**

(12) **EUROPÄISCHE PATENTSCHRIFT**

(45) Veröffentlichungstag der Patentschrift: **06.03.91**

(21) Anmeldenummer: **87118030.3**

(22) Anmeldetag: **05.12.87**

(51) Int. Cl.⁵: **C07D 471/04**, C07D 471/14, C07D 487/04, C07D 487/14, C07D 495/04, C07D 243/38, A61K 31/55, //(C07D471/04, 243:00,221:00)

# (54) Neue kondensierte Diazepinone, Verfahren zu ihrer Herstellung und diese Verbindungen enthaltende Arzneimittel.

(30) Priorität: **20.12.86 DE 3643666**

(43) Veröffentlichungstag der Anmeldung: **06.07.88 Patentblatt 88/27**

(45) Bekanntmachung des Hinweises auf die Patenterteilung: **06.03.91 Patentblatt 91/10**

(84) Benannte Vertragsstaaten: **AT BE CH DE ES FR GB GR IT LI LU NL SE**

(56) Entgegenhaltungen:
EP-A- 0 039 519
EP-A- 0 057 428
EP-A- 0 125 607
DD-A- 236 731
DE-A- 2 424 811
US-A- 3 660 380
US-A- 4 410 527
EP-A- 0 044 989
EP-A- 0 066 774
EP-A- 0 156 191
DE-A- 2 065 570
DE-A- 3 016 647
US-A- 3 691 159
US-A- 4 424 222

(73) Patentinhaber: **Dr. Karl Thomae GmbH
Postfach 1755
W-7950 Biberach 1(DE)**

(72) Erfinder: **Engel, Wolfhard, Dr. Dipl.-Chem.
Mozartstrasse 13
W-7950 Biberach 1(DE)**
Erfinder: **Eberlein, Wolfgang, Dr. Dipl.-Chem.
Obere Au 6
W-7950 Biberach 1(DE)**
Erfinder: **Mihm, Gerhard, Dr. Dipl.-Chem.
Nickeleshalde 5/1
W-7950 Biberach 1(DE)**
Erfinder: **Trummlitz, Günter, Dr., Dipl.Chem.
Buchenweg 27
W-7951 Warthausen(DE)**
Erfinder: **Mayer, Norbert, Dr.
Friedrich-Ebert-Strasse 66
W-7950 Biberach 1(DE)**
Erfinder: **De Jonge, Adriaan, Dr.
De Boomgaard 19
NL-3971 LD Driebergen(NL)**

**EP 0 273 239 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue kondensierte Diazepinone, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 ist beschrieben, daß durch Einführung neuartiger Aminoacylreste gegenüber den Verbindungen der obengenannten Publikationen völlig andersartige, wertvolle pharmakologische Eigenschaften induziert werden können. Gegenüber diesen Verbindungen zeichnen sich die erfindungsgemäßen kondensierten Diazepinone bei vergleichbarer oder verbesserter Selektivität und Resorption nach oraler Gabe durch eine wesentlich verstärke Wirkung und ausgeprägte Hydrolysestabilität aus.

Die neuen kondensierten Diazepinone besitzen die allgemeine Formel I,

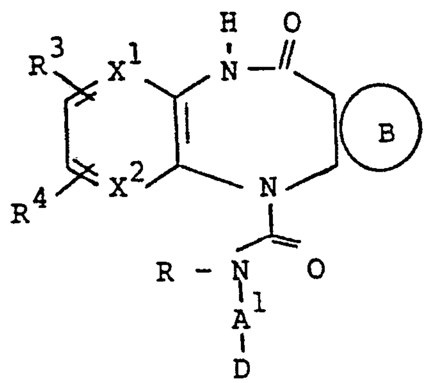

(I)

in der

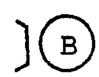

einen der zweiwertigen Reste

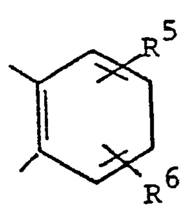 , 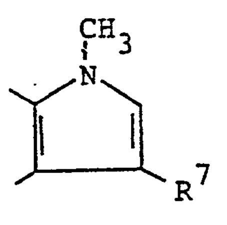 , 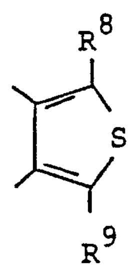 , 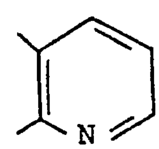 oder 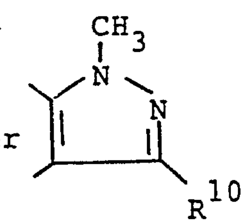

(S) (T) (U) (V) (W)

und D die Gruppen

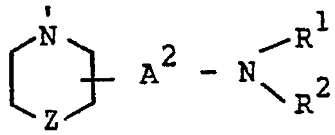 , 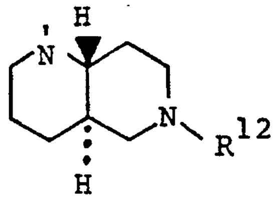 , 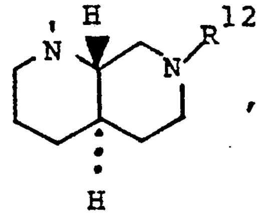 ,

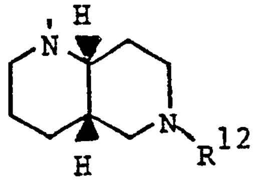 und 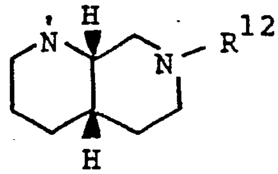

darstellen und

$X^1$, $X^2$, $A^1$, $A^2$, R, $R^1$ bis $R^{10}$, $R^{12}$ und Z die folgenden Bedeutungen besitzen:

$X^1$ und $X^2$ stellen eine =CH-Gruppe dar oder, sofern

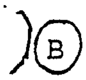

die Bedeutungen der oben genannten, zweiwertigen Reste S, U oder W annimmt, können beide oder nur $X^1$ oder nur $X^2$ auch ein Stickstoffatom darstellen;

$A^1$ ist ein geradkettiger oder verzweigter gesättigter Alkylenrest mit zwei bie sieben Kohlenstoffatomen;

$A^2$ ein geradkettiger oder verzweigter gesättigter Alkylenrest mit 1 bis 5 Kohlenstoffatomen oder, wenn er sich in 3-Stellung zum Stickstoff des gesättigten heterocyclischen Ringes befindet, auch eine Einfachbindung;

Z eine Einfachbindung, ein Sauerstoff- oder Schwefelatom, die Methylen- oder 1,2-Ethylengruppe;

R ein Wasserstoffatom oder eine Methylgruppe;

$R^1$ ein verzweigter oder unverzweigter Alkylrest mit 1 bis 4 Kohlenstoffatomen;

$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;

$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder durch die $N\text{-}CH_3$-Gruppe unterbrochen sein kann;

$R^3$ ist eine Alkylgruppe mit 1 bis 4 C-Atomen, ein Chloratom oder ein Wasserstoffatom;

$R^4$ ein Wasserstoffatom oder eine Methylgruppe;

$R^5$ und $R^6$ bedeuten jeweils ein Wasserstoffatom, ein Fluor-, Chlor- oder Bromatom oder eine Alkylgruppe mit 1 bis 4 C-Atomen;

$R^7$ ein Wasserstoff- oder Chloratom oder eine Methylgruppe;

$R^8$ ein Wasserstoffatom oder einen Alkylrest mit 1 bis 4 Kohlenstoffatomen;

$R^9$ ein Wasserstoff- oder Halogenatom oder eine Alkylgruppe mit 1 bis 4 Kohlenstoffatomen;

$R^{10}$ ein Wasserstoffatom oder eine Methylgruppe und

$R^{12}$ einen verzweigten oder unverzweigten Alkylrest mit 1 bis 6 Kohlenstoffatomen.

Für den Fall, daß

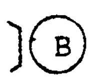

den zweiwertigen Rest T darstellt und $R^7$ ein Wasserstoffatom ist, können $R^3$ kein Chloratom und Z kein Schwefelatom bedeuten.

Bevorzugte Verbindungen der obigen allgemeinen Formel I sind solche, in denen

$X^1$ eine =CH-Gruppe,

$X^2$ entweder ein Stickstoffatom und

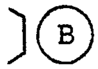

den zweiwertigen Rest S darstellt, mit der Maßgabe, daß $R^3$, $R^4$ und $R^5$ Wasserstoffatome sind und $R^6$ ein Wasserstoffatom, ein Chlor- oder Bromatom oder eine Methyl- oder Ethylgruppe in 8- oder 9-Stellung des Heterocyclus ist,

oder eine =CH-Gruppe ist, wenn

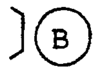

die Bedeutung des zweiwertigen Restes U annimmt, wobei $R^8$ ein Wasserstoffatom und $R^9$ eine Methylgruppe ist;

$A^1$ eine 1,2-Ethylengruppe;

$A^2$ eine geradkettige Alkylengruppe mit 1 bis 5 Kohlenstoffatomen;

Z eine Methylengruppe;

R ein Wasserstoffatom und

$R^1$ und $R^2$ Alkylgruppen mit 1 bis 3 Kohlenstoffatomen oder zusammen mit dem dazwischenliegenden Stickstoffatom den Piperidinylrest darstellen.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure, Amidosulfonsäure oder Cyclohexansulfaminsäure als geeignet erwiesen.

Zur Erläuterung des Erfindungsgegenstandes seien als Beispiele folgende Verbindungen genannt:

5,11-Dihydro-11-[[[2-[2-[(diethylamino)methyl]-1-piperidinyl]ethyl]amino]carbonyl]-6H-pyrido[2,3-b][1,4]-benzodiazepin-6-on

5,11-Dihydro-11-[[[2-[2-[[bis-(methylethyl)amino]methyl]-1-piperidinyl]ethyl]amino]carbonyl]-6H-pyrido-[2,3-b][1,4]benzodiazepin-6-on

5,11-Dihydro-11-[[[2-[2-[[bis-(2-methylpropyl)amino]methyl]-1-piperidinyl]ethyl]amino]carbonyl]-6H-pyrido[2,3-b][1,4]-benzodiazepin-6-on

5,11-Dihydro-11-[[[2-[2-[(1-piperidinyl)methyl]-1-piperidinyl]ethyl]amino]carbonyl]-6H-pyrido[2,3-b][1,4]-benzodiazepin-6-on-hydrochlorid

5,11-Dihydro-11-[[[2-[2-[(1-pyrrolidinyl)methyl]-1-piperidinyl]ethyl]amino]carbonyl]-6H-pyrido[2,3-b][1,4]-benzodiazepin-6-on

5,11-Dihydro-11-[[[2-[2-[(hexahydro-1H-1-azepinyl)methyl]-1-piperidinyl]ethyl]amino]carbonyl]-6H-pyrido[2,3-b][1,4]-benzodiazepin-6-on

5,11-Dihydro-11-[[[2-[2-[(4-morpholinyl)methyl]-1-piperidinyl]ethyl]amino]carbonyl]-6H-pyrido[2,3-b][1,4]-benzodiazepin-6-on

5,11-Dihydro-11-[[[2-[2-[(tetrahydro-4H-1,4-thiazin-4-yl)methyl]-1-piperidinyl]ethyl]amino]carbonyl]-6H-pyrido[2,3-b][1,4]-benzodiazepin-6-on

5,11-Dihydro-11-[[[2-[2-[(diethylamino)methyl]-1-piperidinyl]-1,1-dimethylethyl]amino]carbonyl]-6H-pyrido[2,3-b][1,4]-benzodiazepin-6-on

5,11-Dihydro-11-[[[2-[2-[(diethylamino)methyl]-1-piperidinyl]-2,2-dimethylethyl]amino]carbonyl]-6H-pyrido[2,3-b][1,4]-benzodiazepin-6-on

5,11-Dihydro-11-[[[3-[2-[(diethylamino)methyl]-1-piperidinyl]propyl]amino]carbonyl]-6H-pyrido[2,3-b]-[1,4]-benzodiazepin-6-on

5,11-Dihydro-11-[[[2-[2-[(dimethylamino)methyl]-1-piperidinyl]ethyl]amino]carbonyl]-6H-pyrido[2,3-b]-[1,4]-benzodiazepin-6-on

5,11-Dihydro-11-[[[3-[2-[(dimethylamino)methyl]-1-piperidinyl]propyl]amino]carbonyl]-6H-pyrido[2,3-b]-[1,4]-benzodiazepin-6-on

11-[[[2-[2-[2-(Diethylamino)ethyl]-1-piperidinyl]ethyl]amino]carbonyl]-5,11-dihydro-6H-pyrido[2,3-b][1,4]-benzodiazepin-6-on

11-[[[2-[2-[3-(Diethylamino)propyl]-1-piperidinyl]ethyl]amino]carbonyl]-5,11-dihydro-6H-pyrido[2,3-b]-[1,4]-benzodiazepin-6-on

5,11-Dihydro-11-[[[2-[2-[3-(dimethylamino)propyl]-1-piperidinyl]ethyl]amino]carbonyl]-6H-pyrido[2,3-b]-[1,4]-benzodiazepin-6-on

(R,S)-11-[[[2-[2-[(Diethylamino)methyl]-1-pyrrolidinyl]ethyl]amino]carbonyl]-5,11-dihydro-6H-pyrido[2,3-b][1,4]benzodiazepin-6-on

(R,S)-5,11-Dihydro-11-[[[2-[2-[(dipropylamino)methyl]-1-pyrrolidinyl]ethyl]amino]carbonyl]-6H-pyrido[2,3-b][1,4]-benzodiazepin-6-on

D,L-11-[[[4-[2-[(Diethylamino)methyl]-1-piperidinyl]butyl]amino]carbonyl]-5,11-dihydro-6H-pyrido[2,3-b]-[1,4]-benzodiazepin-6-on

D,L-5,11-Dihydro-11-[[[2-[2-[2-(dimethylamino)ethyl]-1-piperidinyl]ethyl]amino]carbonyl]-6H-pyrido[2,3-b][1,4]-benzodiazepin-6-on

D,L-11-[[[2-[2-[(Diethylamino)methyl]-1-piperidinyl]ethyl]methylamino]carbonyl]-5,11-dihydro-6H-pyrido-[2,3-b][1,4]benzodiazepin-6-on

D,L-5,11-Dihydro-11-[[[2-[2-[(dimethylamino)methyl]-1-piperidinyl]ethyl]methylamino]carbonyl]-6H-pyrido[2,3-b][1,4]benzodiazepin-6-on

(R,S)-11-[[[6-[2-[(Diethylamino)methyl]-1-piperidinyl]hexyl]amino]carbonyl]-5,11-dihydro-6H-pyrido[2,3-b][1,4]benzodiazepin-6-on

trans-5,11-Dihydro-11-[[[2-[2-[[(4-hydroxycyclohexyl)methylamino]methyl]-1-piperidinyl]ethyl]-methylamino]carbonyl]-6H-pyrido[2,3-b][1,4]benzodiazepin-6-on

5,11-Dihydro-11-[[[2-[3-[3-(1-piperidinyl)propyl]-1-piperidinyl]ethyl]amino]carbonyl]-6H-pyrido[2,3-b][1,4]-benzodiazepin-6-on

(R,S)-11-[[[2-[2-[2-(Diethylamino)ethyl]-1-pyrrolidinyl]ethyl]amino]carbonyl]-5,11-dihydro-6H-pyrido[2,3-b][1,4]benzodiazepin-6-on

D,L-11-[[[2-[2-[2-(Diethylamino)ethyl]-1-piperidinyl]ethyl]methylamino]carbonyl]-5,11-dihydro-6H-pyrido-[2,3-b][1,4]benzodiazepin-6-on

D,L-5,11-Dihydro-11-[[[4-[2-[(dimethylamino)methyl]-1-piperidinyl]butyl]amino]carbonyl]-6H-pyrido[2,3-b][1,4]benzodiazepin-6-on

trans-D,L-5,11-Dihydro-11-[[[2-[2-[[(4-hydroxycyclohexyl)methylamino]methyl]-1-piperidinyl]ethyl]amino]-carbonyl]-6H-pyrido[2,3-b][1,4]benzodiazepin-6-on

9-Chlor-11-[[[2-[2-[(diethylamino)methyl]-1-piperidinyl]ethyl]amino]carbonyl]-5,11-dihydro-6H-pyrido[2,3-b][1,4]benzodiazepin-6-on

5,11-Dihydro-11-[[[2-[2-[2-(dimethylamino)ethyl]-1-piperidinyl]ethyl]methylamino]carbonyl]-6H-pyrido-[2,3-b][1,4]benzodiazepin-6-on

11-[[[2-[2-[(Diethylamino)methyl]-1-piperidinyl]ethyl]amino]carbonyl]-5,11-dihydro-8-methyl-6H-pyrido-[2,3-b][1,4]-benzodiazepin-6-on

D,L-5,11-Dihydro-11-[[[4-[2-[(dimethylamino)methyl]-1-piperidinyl]butyl]methylamino]carbonyl]-6H-pyrido[2,3-b][1,4]-benzodiazepin-6-on

5,11-Dihydro-11-[[[2-[3-[(1-piperidinyl)methyl]-1-piperidinyl]ethyl]amino]carbonyl]-6H-pyrido[2,3-b][1,4]-benzodiazepin-6-on-dihydrochlorid

11-[[[2-[3-[(Diethylamino)methyl]-4-morpholinyl]ethyl]amino]carbonyl]-5,11-dihydro-6H-pyrido[2,3-b][1,4]-benzodiazepin-6-on

5,11-Dihydro-11-[[[2-[3-[(1-piperidinyl)methyl]-4-morpholinyl]ethyl]amino]carbonyl]-6H-pyrido[2,3-b][1,4]-benzodiazepin-6-on

11-[[[4-[2-[(Diethylamino)methyl]-1-piperidinyl]butyl]methylamino]carbonyl]-5,11-dihydro-6H-pyrido[2,3-b][1,4]-benzodiazepin-6-on

5,11-Dihydro-11-[[[2-[2-[(dipropylamino)methyl]-1-piperidinyl]ethyl]amino]carbonyl]-6H-pyrido[2,3-b]-[1,4]-benzodiazepin-6-on

8-Chlor-11-[[[2-[2-[(diethylamino)methyl]-1-piperidinyl]ethyl]amino]carbonyl]-5,11-dihydro-6H-pyrido[2,3-b][1,4]-benzodiazepin-6-on

11-[[[2-[2-[(Diethylamino)methyl]-1-piperidinyl]ethyl]amino]carbonyl]-8-ethyl-6H-pyrido[2,3-b][1,4]-benzodiazepin-6-on

8-Brom-11-[[[2-[2-[(diethylamino)methyl]-1-piperidinyl]ethyl]amino]carbonyl]-5,11-dihydro-6H-pyrido[2,3-b][1,4]benzodiazepin-6-on

11-[[[2-[2-[(Diethylamino)methyl]-1-piperidinyl]ethyl]amino]carbonyl]-5,11-dihydro-7-fluor-6H-pyrido[2,3-b][1,4]benzodiazepin-6-on

11-[[[2-[2-[(Diethylamino)methyl]-1-piperidinyl]ethyl]amino]carbonyl]-5,11-dihydro-8-fluor-6H-pyrido[2,3-b][1,4]benzodiazepin-6-on

11-[[[2-[2-[(Diethylamino)methyl]-1-piperidinyl]ethyl]amino]carbonyl]-5,11-dihydro-9-fluor-6H-pyrido[2,3-b][1,4]benzodiazepin-6-on

11-[[[2-[2-[(Diethylamino)methyl]-1-piperidinyl]ethyl]amino]carbonyl]-5,11-dihydro-10-fluor-6H-pyrido[2,3-b][1,4]benzodiazepin-6-on

11-[[[2-[2-[(Diethylamino)methyl]-1-piperidinyl]ethyl]amino]carbonyl]-5,11-dihydro-9-methyl-6H-pyrido-[2,3-b][1,4]benzodiazepin-6-on

11-[[[2-[2-[4-(Diethylamino)butyl]-1-piperidinyl]ethyl]amino]carbonyl]-5,11-dihydro-6H-pyrido[2,3-b][1,4]-benzodiazepin-6-on

6,11-Dihydro-11-[[[2-[2-[(dipropylamino)methyl]-1-piperidinyl]ethyl]amino]carbonyl]-5H-pyrido[2,3-b]-[1,5]benzodiazepin-5-on

11-[[[2-[2-[(Diethylamino)methyl]-1-piperidinyl]ethyl]amino]carbonyl]-6,11-dihydro-5H-pyrido[2,3-b][1,5]-benzodiazepin-5-on

5,11-Dihydro-11-[[[2-[2-[4-(1-piperidinyl)butyl]-1-piperidinyl]ethyl]amino]carbonyl]-6H-pyrido[2,3-b][1,4]-benzodiazepin-6-on

2-Chlor-11-[[[2-[2-[(diethylamino)methyl]-1-piperidinyl]ethyl]amino]carbonyl]-5,11-dihydro-6H-pyrido[2,3-b][1,4]benzodiazepin-6-on

11-[[[2-[2-[(Diethylamino)methyl]-1-piperidinyl]ethyl]amino]carbonyl]-5,11-dihydro-2-methyl-6H-pyrido-[2,3-b][1,4]benzodiazepin-6-on

11-[[[2-[2-[(Diethylamino)methyl]-1-piperidinyl]ethyl]amino]carbonyl]-5,11-dihydro-2,4,8-trimethyl-6H-pyrido[2,3-b][1,4]benzodiazepin-6-on

5-[[[2-[2-[(Diethylamino)methyl]-1-piperidinyl]ethyl]amino]carbonyl]-5,11-dihydro-10H-pyrido[3,2-b][1,4]-benzodiazepin-10-on

5,11-Dihydro-5-[[[2-[2-[(dipropylamino)methyl]-1-piperidinyl]ethyl]amino]carbonyl]-10H-pyrido[3,2-b]-[1,4]benzodiazepin10-on

4-[[[2-[2-[(Diethylamino)methyl]-1-piperidinyl]ethyl]amino]carbonyl]-1-methyl-1,4,9,10-tetrahydropyrazolo[4,3-e]pyrido[3,2-b][1,4]diazepin-10-on

4-[[[2-[2-[(Diethylamino)methyl]-1-piperidinyl]ethyl]amino]carbonyl]-1,3-dimethyl-1,4,9,10-tetrahydropyrazolo[4,3-e]pyrido[3,2-b][1,4]diazepin-10-on

4-[[[2-[2-[(Dipropylamino)methyl]-1-piperidinyl]ethyl]amino]carbonyl]-1-methyl-1,4,9,10-tetrahydropyrazolo[4,3-e]pyrido[3,2-b][1,4]diazepin-10-on

4-[[[2-[2-[(Diethylamino)methyl-1-piperidinyl]ethyl]amino]carbonyl]-1,3-dimethyl-1,4,9,10-tetrahydropyrazolo[4,3-b][1,5]benzodiazepin-10-on

4-[[[2-[2-[(Diethylamino)methyl]-1-piperidinyl]ethyl]amino]carbonyl]-1-methyl-1,4,9,10-tetrahydropyrazolo[4,3-b][1,5]benzodiazepin-10-on

11-[[[2-[2-[[(Cyclopentyl)methylamino]methyl]-1-piperidinyl]ethyl]amino]carbonyl]-5,11-dihydro-6H-pyrido[2,3-b][1,4]benzodiazepin-6-on

11-[[[2-[3-(Diethylamino)hexahydro-1H-1-azepinyl]ethyl]amino]carbonyl]-5,11-dihydro-6H-pyrido[2,3-b]-[1,4]benzodiazepin-6-on

11-[[[2-[3-[(Diethylamino)methyl]-1-piperidinyl]ethyl]amino]carbonyl]-5,11-dihydro-6H-pyrido[2,3-b][1,4]-benzodiazepin-6-on

5,11-Dihydro-11-[[[2-[3-[(dipropylamino)methyl]-1-piperidinyl]ethyl]amino]carbonyl]-6H-pyrido[2,3-b]-[1,4]-benzodiazepin-6-on

5,11-Dihydro-11-[[[2-[3-[(hexahydro-1H-1-azepinyl)methyl]-1-piperidinyl]ethyl]amino]carbonyl]-6H-pyrido[2,3-b][1,4]benzodiazepin-6-on

9-Chlor-11-[[[2-[3-[(diethylamino)methyl]-1-piperidinyl]ethyl]amino]carbonyl]-5,11-dihydro-6H-pyrido[2,3-b][1,4]benzodiazepin-6-on

11-[[[2-[4-[(Diethylamino)methyl]-1-piperidinyl]ethyl]amino]carbonyl]-5,11-dihydro-6H-pyrido[2,3-b][1,4]-benzodiazepin-6-on

5,11-Dihydro-11-[[[2-[4-[2-(1-piperidinyl)ethyl]-1-piperidinyl]ethyl]amino]carbonyl]-6H-pyrido[2,3-b][1,4]-benzodiazepin-6-on-hydrochlorid

11-[[[2-[4-[2-(Diethylamino)ethyl]-1-piperidinyl]ethyl]amino]carbonyl]-5,11-dihydro-6H-pyrido[2,3-b][1,4]-benzodiazepin-6-on

11-[[[2-[4-[3-(Diethylamino)propyl]-1-piperidinyl]ethyl]amino]carbonyl]-5,11-dihydro-6H-pyrido[2,3-b]-[1,4]-benzodiazepin-6-on

5,11-Dihydro-11-[[[2-[2-[(dipropylamino)methyl]-4-morpholinyl] ethyl]amino]carbonyl]-6H-pyrido[2,3-b]-[1,4]-benzodiazepin-6-on

5-[[[2-[2-[(Diethylamino)methyl]-1-piperidinyl]ethyl]amino]carbonyl]-5,10-dihydro-11H-dibenzo[b,e][1,4]-diazepin-11-on

5,10-Dihydro-5-[[[3-[2-[(dimethylamino)methyl]-1-piperidinyl]propyl]amino]carbonyl]-11H-dibenzo[b,e]-[1,4]diazepin-11-on

5-[[[3-[2-[(Diethylamino)methyl]-1-piperidinyl]propyl]amino]carbonyl]-5,10-dihydro-11H-dibenzo[b,e][1,4]-diazepin-11-on

5,10-Dihydro-5-[[[2-[2-[(dimethylamino)methyl]-1-piperidinyl]ethyl]amino]carbonyl]-11H-dibenzo[b,e]-[1,4]diazepin-11-on

(±)-5-[[[2-[2-[(Diethylamino)methyl]-1-pyrrolidinyl]ethyl]amino]carbonyl]-5,10-dihydro-11H-dibenzo[b,e]-[1,4]diazepin-11-on

4-[[[2-[2-[(Diethylamino)methyl]-1-piperidinyl]ethyl]amino]carbonyl]-4,9-dihydro-3-methyl-10H-thieno[3,4-b][1,5]benzodiazepin-10-on

4,9-Dihydro-4-[[[2-[2-[(1-pyrrolidinyl)methyl]-1-piperidinyl]ethyl]amino]carbonyl]-3-methyl-10H-thieno[3,4-b][1,5]benzodiazepin-10-on

3-Chlor-4-[[[2-[2-[(diethylamino)methyl]-1-piperidinyl]ethyl]amino]carbonyl]-4,9-dihydro-10H-thieno[3,4-b][1,5]benzodiazepin-10-on

1,3-Dimethyl-4-[[[2-[2-[(diethylamino)methyl]-1-piperidinyl]ethyl]amino]carbonyl]-4,9-dihydro-10H-thieno-[3,4-b][1,5]benzodiazepin-10-on

1,3-Dimethyl-4-[[[2-[2-[(dimethylamino)methyl]-1-piperidinyl]ethyl]amino]carbonyl]-4,9-dihydro-10H-thieno[3,4-b][1,5]benzodiazepin-10-on

(+)-5,11-Dihydro-11-[[[2-[2-[(diethylamino)methyl]-1-piperidinyl]ethyl]amino]carbonyl]-6H-pyrido[2,3-b]-[1,4]benzodiazepin-6-on

(-)-5,11-Dihydro-11-[[[2-[2-[(diethylamino)methyl]-1-piperidinyl]ethyl]amino]carbonyl]-6H-pyrido[2,3-b]-[1,4]benzodiazepin-6-on

(+)-4-[[[2-[2-[(Diethylamino)methyl]-1-piperidinyl]ethyl]amino]carbonyl]-4,9-dihydro-3-methyl-10H-thieno[3,4-b][1,5]benzodiazepin-10-on

(-)-4-[[[2-[2-[(Diethylamino)methyl]-1-piperidinyl]ethyl]amino]carbonyl]-4,9-dihydro-3-methyl-10H-thieno-[3,4-b][1,5]benzodiazepin-10-on

(+)-9-Chlor-11-[[[2-[2-[(diethylamino)methyl]-1-piperidinyl]ethyl]amino]carbonyl]-5,11-dihydro-6H-pyrido-[2,3-b][1,4]benzodiazepin-6-on

(-)-9-Chlor-11-[[[2-[2-[(diethylamino)methyl]-1-piperidinyl]ethyl]amino]carbonyl]-5,11-dihydro-6H-pyrido-[2,3-b][1,4]benzodiazepin-6-on

1,3-Dimethyl-4-[[[2-[2-[(dipropylamino)methyl]-1-piperidinyl]ethyl]amino]carbonyl]-1,4,9,10-tetrahydropyrrolo[3,2-b][1,5]benzodiazepin-10-on

1,3-Dimethyl-4-[[[2-[2-[(hexahydro-1H-1-azepinyl)methyl]-1-piperidinyl]ethyl]amino]carbonyl]-1,4,9,10-tetrahydropyrrolo[3,2-b][1,5]benzodiazepin-10-on

5-Chlor-4-[[[2-[2-[(diethylamino)methyl]-1-piperidinyl]ethyl]amino]carbonyl]-1,3-dimethyl-1,4,9,10-tetrahydropyrrolo[3,2-b][1,5]benzodiazepin-10-on

3-Chlor-1-methyl-4-[[[2-[2-[(dipropylamino)methyl]-1-piperidinyl]ethyl]amino]carbonyl]-1,4,9,10-tetrahydropyrrolo[3,2-b][1,5]benzodiazepin-10-on

1-Methyl-4-[[[2-[2-[(dipropylamino)methyl]-1-piperidinyl]ethyl]amino]carbonyl]-1,4,9,10-tetrahydropyrrolo-[3,2-b][1,5]benzodiazepin-10-on

4-[[[2-[2-[(Dibutylamino)methyl]-1-piperidinyl]ethyl]amino]carbonyl]-1-methyl-1,4,9,10-tetrahydropyrrolo-[3,2-b][1,5]benzodiazepin-10-on

6-Chlor-5-[[[2-[2-[(diethylamino)methyl]-1-piperidinyl]ethyl]amino]carbonyl]-5,10-dihydro-11H-dibenzo-[b,e][1,4]diazepin-11-on

Erfindungsgemäß erhält man die neuen basisch substituierten kondensierten Diazepinone der allgemeinen Formel I nach folgenden Verfahren:

a) Basisch substituierte kondensierte Diazepinone der allgemeinen Formel Ia

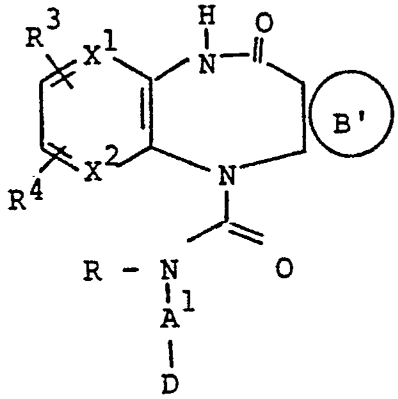

(Ia)

in der
R, $R^3$, $R^4$, $X^1$, $X^2$, $A^1$ und D die oben angegebenen Bedeutungen haben und

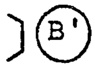

einen der zweiwertigen Reste S, U, V, W oder T′

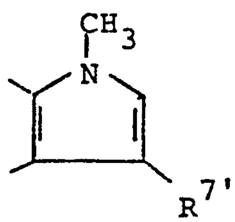

(T')

darstellt, wobei $R^{7'}$ ein Chloratom oder eine Methylgruppe ist,

erhält man durch Umsetzung von Kohlensäurederivaten der allgemeinen Formel II,

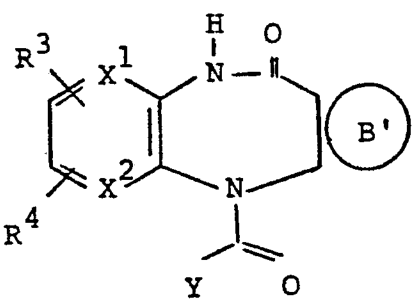

(II)

in der
$R^3$, $R^4$,

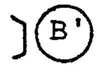

$X^1$, $X^2$ die angegebenen Bedeutungen haben und

Y ein Halogenatom, bevorzugt das Brom- oder Chloratom, oder den Rest $OR^{11}$ bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit Verbindungen der allgemeinen Formel III,

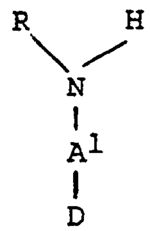

(III)

in der
R, $A^1$ und D wie vorstehend definiert sind.

Die Umsetzung wird ohne oder bevorzugt in Gegenwart von Lösungsmitteln, wie z. B. von Wasser, Toluol oder von Alkoholen, wie z. B Methanol, Ethanol oder Isopropanol, ganz bevorzugt jedoch in Gegenwart aprotischer polarer Lösungsmittel, z. B. von Tetrahydrofuran, 1,4-Dioxan, Acetonitril, N,N-Dimethylformamid, Dimethylsulfoxid, Hexamethylphosphorsäuretriamid, oder von Gemischen davon und bei Temperaturen zwischen -10° C und dem Siedepunkt des Reaktionsgemisches, bevorzugt zwischen 40 und 100° C durchgeführt. Bewährt hat sich die Verwendung zusätzlicher anorganischer oder organischer Basen, z. B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat; von tertiären Aminen, z. B. von Triethylamin, Ethyldiisopropylamin, N,N-Dimethylanilin, Pyridin oder von 4-(Dimethylamino)pyridin; sowie die Umsetzung in Gegenwart eines Überschusses einer Verbindung der allgemeinen Formel III.

Die Umsetzung kann jedoch auch mit einer Metallverbindung der allgemeinen Formel IIIa,

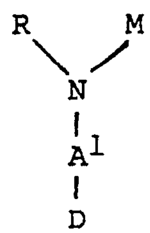

(IIIa)

in der
M ein Alkalimetallatom oder 1 Äquivalent eines Erdalkalimetallatoms bedeutet, durchgeführt werden. Dabei lassen sich Metallverbindungen der allgemeinen Formel IIIa aus III durch Umsetzung mit Alkali- oder Erdalkalimetallen, etwa mit Natrium, Kalium oder Barium, oder mit Alkali- oder Erdalkalihydriden, etwa mit Natrium-, Kalium oder Calciumhydrid, oder durch Reaktion mit Alkali- oder Erdalkaliorganylen, z. B. mit n-Butyllithium oder Phenyllithium, in situ leicht herstellen.

b.) Basisch substituierte kondensierte Diazepinone der allgemeinen Formel Ia lassen sich auch durch Umsetzung von Tricyclen der allgemeinen Formel IV,

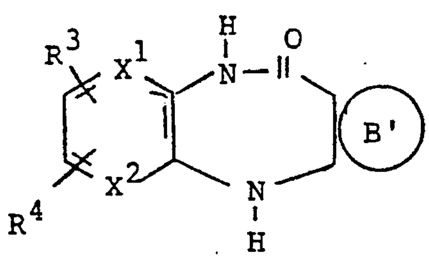

(IV)

in der
die Reste $R^3$, $R^4$, $X^1$, $X^2$ und

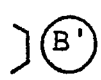

wie oben definiert sind, mit einem Chlorkohlensäurederivat der allgemeinen Formel V

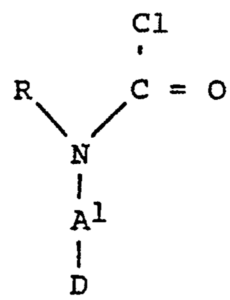

(V)

oder - sofern in Verbindungen der allgemeinen Formel Ia R die Bedeutung eines Wasserstoffatoms annimmt - einem Isocyanat der allgemeinen Formel Va,

$$O = C = N - A^1 - D \qquad (Va)$$

in denen die Reste R, $A^1$ und D die oben erwähnten Bedeutungen haben, erhalten.

Die Umsetzung wird vorzugsweise in inerten organischen Lösungsmitteln, beispielsweise in aromatischen Kohlenwasserstoffen wie Toluol, Xylol, in Äthern wie Diisopropyläther, Tetrahydrofuran oder Dioxan, in Ketonen wie 3-Pentanon, in chlorierten aliphatischen Kohlenwasserstoffen, wie 1,2-Dichlorethan oder in anderen Lösungsmitteln, wie Acetonitril oder Dimethylformamid oder in Gemischen davon, gegebenenfalls in Gegenwart tertiärer organischer Basen, wie Pyridin, und bei Temperaturen bis zum Siedepunkt des Reaktionsgemisches, bevorzugt bei Temperaturen zwischen +30 und +100 °C, durchgeführt.

c) Die unter die allgemeine Formel I fallenden neuen Pyrrolo-kondensierten Diazepinone der allgemeinen Formel Ib,

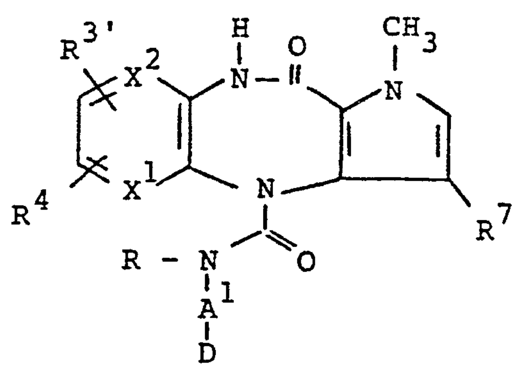

(Ib)

worin

$R^4$, $X^1$, $X^2$, $A^1$, und D die eingangs erwähnten Bedeutungen haben,

$R^{3'}$ eine Alkylgruppe mit 1 bis 4 C-Atomen oder ein Wasserstoffatom und

$R^7$ ein Wasserstoffatom darstellen und für die Gruppe

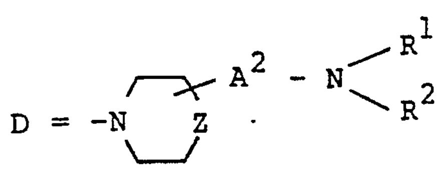

Z die eingangs genannten Bedeutungen mit Ausnahme eines Schwefelatoms hat, wobei $R^1$, $R^2$ und $A^2$ wie eingangs definiert sind, können auch durch Hydrogenolyse aus solchen Verbindungen der allgemeinen Formel Ib hergestellt werden, in denen $R^7$ ein Chloratom bedeutet.

Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von

0 ° C bis 130 ° C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110 ° C, sowie die Reduktion mit Triethylammoniumformiat in Gegenwart überschüssigen Triethylamins und von Palladium auf Tierkohle oder von Palladiumacetat und Triarylphosphinen, wie Triphenylphosphin, Tris-(o-tolyl)phosphin, Tris-(2,5-diisopropylphenyl)phosphin, bei Temperaturen zwischen 40 und 110 ° C, bewährt.

d) Basisch substituierte kondensierte Diazepinone der allgemeinen Formel Ia

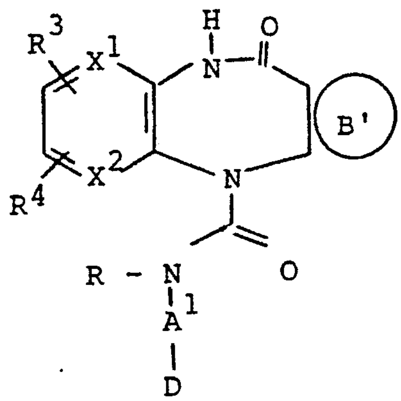

(Ia)

in der
R, $R^3$, $R^4$, $X^1$, $X^2$, $A^1$ und D die oben angegebenen Bedeutungen haben und

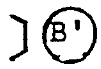

einen der zweiwertigen Reste S, U, V, W oder T'

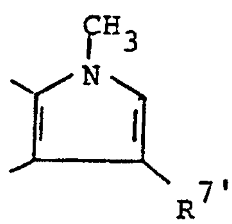

(T')

darstellt, wobei $R^{7'}$ ein Chloratom oder eine Methylgruppe ist,

erhält man durch Umsetzung von kondensierten Diazepinonen der allgemeinen Formel VIII,

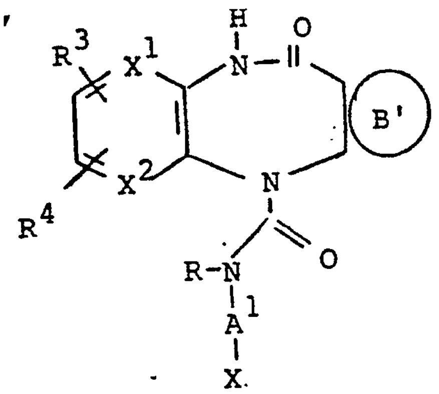

(VIII)

in der

R, $R^3$, $R^4$, $X^1$, $X^2$ und $A^1$ die angegebenen Bedeutungen haben und

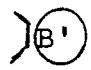

wie vorstehend definiert ist und X eine nucleofuge Gruppe darstellt, beispielsweise ein Chlor-, Brom- oder Iodatom , eine Alkansulfonyloxy-, Arensulfonyloxy-, die Trifluormethansulfonyloxy-oder auch die Hydroxygruppe, mit Diaminen der allgemeinen Formel IIIc,

H - D (IIIc)

in der D die eingangs genannte Bedeutung hat.

Die Aminierung erfolgt in einem inerten Lösungsmittel bei Temperaturen zwischen -10°C und der Siedetemperatur des Lösungsmittels, vorzugsweise entweder mit wenigstens 2 Molen sekundärem Amin der allgemeinen Formel IIIc oder mit 1 bis 2 Molen eines sekundären Amins der allgemeinen Formel IIIc und einer Hilfsbase. Als Lösungsmittel kommen beispielsweise chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone wie Aceton; Acetonitril, Dimethylformamid oder 1,3-Dimethyl-2-imidazolidinon in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall- oder Erdalkalimetallcarbonate oder -hydrogencarbonate, -hydroxide oder -oxide genannt. Gegebenenfalls kann die Reaktion durch Zusatz von Alkalimetalliodiden beschleunigt werden. Die Reaktionszeiten betragen je nach Menge und Art des eingesetzten Amins der allgemeinen Formel IIIc zwischen 15 Minuten und 80 Stunden. Verwendet man bei dieser Umsetzung nur 1 Mol des sekundären Amins der allgemeinen Formel IIIc auf 1 Mol einer Verbindung der allgemeinen Formel VIII, so erhält man direkt das Monohydrochlorid, Monohydrobromid, Monohydroiodid, Tosylat oder Mesylat der gesuchten Verbindung der allgemeinen Formel Ia, wenn X in der allgemeinen Formel VIII beispielsweise ein Chlor-, Brom-, Iodatom, die p-Toluolsulfonyloxy-oder Methansulfonyloxygruppe darstellt.

Ist in der allgemeinen Formel VIII X die Hydroxygruppe, so arbeitet man zweckmäßig in Gegenwart von Katalysatoren aus der VIII. Nebengruppe des Periodensystems der Elemente, beispielsweise von Palladiumschwarz oder Raney-Nickel, und unter Verwendung aprotischer inerter Lösungsmittel, zum Beispiel von offenkettigen oder cyclischen Ethern wie Diethylether, Tetrahydrofuran oder Dioxan, aromatischen Kohlenwasserstoffen wie Benzol, Toluol, Xylol oder Pyridin, von Acetonitril, Dimethylformamid oder 1,3-Dimethyl-2-imidazolidinon oder unter Verwendung überschüssigen sekundären Amins der allgemeinen Formel IIIc als Lösungsmittel. Umsetzungen in Gegenwart von Raney-Nickel können vorteilhaft auch unter Verwendung von Aluminium-t-butoxid als Cokatalysator durchgeführt werden. In Verbindungen der allgemeinen Formel VIII, in denen X die Hydroxygruppe bedeutet, kann diese Funktion in an sich bekannter Weise auch durch Überführung in geeignete Alkoxyphosphoniumsalze aktiviert werden, beispielsweise durch Umsetzung mit Tetrachlormethan und Hexamethylphosphorsäuretriamid, die nach anschließender Behandlung mit Ammoniumperchlorat die entsprechenden isolierbaren Alkoxyphosphoniumperchlorate liefert

$$(X = OP[N(CH_3)_2]_3^{(+)}\ ClO_4^{(-)});$$

Weitere Umsetzung mit sekundären Aminen der allgemeinen Formel IIIc ergibt die angestrebten basisch substituierten Diazepinone der allgemeinen Formel Ia. Eine besonders vorteilhafte Variante der Aktivierung des alkoholischen Hydroxyls in Verbindungen der allgemeinen Formel VIII, in der X die Hydroxygruppe bedeutet, besteht in der aufeinanderfolgenden Umsetzung mit Natriumhydrid in Benzol oder Dimethylformamid, dann mit (N-Methyl-N-phenylamino)triphenylphosphoniumiodid und einem sekundären Amin der allgemeinen Formel IIIc; bei diesem Vorgehen entfällt die Isolierung des intermediär auftretenden Phosphoniumsalzes und die Verwendung von Perchloraten.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Die erfindungsgemäßen aminoacylierten kondensierten Diazepinone der allgemeinen Formel I enthalten in der Regel ein asymmetrisches Kohlenstoffatom in der Seitenkette. Diese Verbindungen können deshalb jeweils als enantiomere (+)- und (-)-Formen auftreten. Die Erfindung umfaßt die einzelnen Isomeren ebenso wie ihre Racemate.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumhydroxid oder Kaliumhydroxid, neutralisiert und dadurch die entsprechende freie Verbindung in der (+)- oder (-)-Form erhalten.

Jeweils nur ein Enantiomeres wird auch dadurch erhalten, daß man die oben beschriebenen Synthesen mit nur einem Enantiomeren der allgemeinen Formel IIIa, V bzw. IIIc durchführt.

Die als Zwischenprodukte erforderlichen Kohlensäurederivate der allgemeinen Formel II erhält man in Analogie zu DE-A-32 04 169, DE-A-32 04 158 und DE-A-32 04 401 durch Umsetzung von Tricyclen der allgemeinen Formel IV mit einem Halogenkohlensäurederivat der allgemeinen Formel VI,

$$Hal - \underset{\underset{O}{\parallel}}{C} - Y \qquad (VI)$$

in der
Hal das Brom- oder Chloratom, bevorzugt das Chloratom, bedeutet und Y die oben angegebenen Bedeutungen hat.

Die Reaktion wird in inerten organischen Lösungsmitteln, beispielsweise aromatischen Kohlenwasserstoffen, wie Toluol, Chlorbenzol oder Xylol; offenkettigen oder cyclischen Ethern, wie Diisopropylether, Tetrahydrofuran oder Dioxan, offenkettigen oder cyclischen aliphatischen Ketonen, beispielsweise 3-Pentanon; chlorierten aliphatischen Kohlenwasserstoffen, wie 1,2-Dichlorethan oder anderen Lösungsmitteln, wie Acetonitril oder Dimethylformamid oder in Gemischen davon und bevorzugt in Gegenwart tertiärer organischer Basen, bevorzugt von Pyridin, und bei Temperaturen bis höchstens zum Siedepunkt des verwendeten Lösungsmittels oder Lösungsmittelgemisches, bevorzugt zwischen +30 und +80 °C, durchgeführt.

Die Ausgangsverbindungen der allgemeinen Formel III, die neu sind, können nach mehreren Verfahren hergestellt werden, unter denen die folgenden bevorzugt werden:

a.) Reduktion von Carbonsäureamiden der allgemeinen Formel III b,

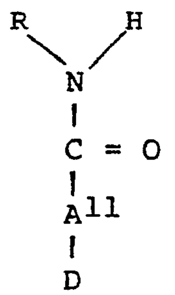

(IIIb)

in der

R und D die oben angegebenen Bedeutungen haben und $A^{11}$ ein geradkettiger oder verzweigter gesättigter Alkylenrest mit bis zu 6 Kohlenstoffatomen ist, der sich von entsprechenden Resten $A^1$ durch das Fehlen einer Methylengruppe unterscheidet.

Als Reduktionsmittel werden Metallhydride sowie komplexe Hydride bevorzugt, für die gutes Reduktionsvermögen gegenüber primären und sekundären aliphatischen Carbonsäureamiden literaturbekannt ist. Geeignete Reduktionsmittel sind beispielsweise: Lithiumaluminiumhydrid in etherischen Lösungsmitteln wie Diethylether, Tetrahydrofuran, Dioxan oder Diethylenglykoldimethylether (siehe z. B. N.G. Gaylord, "Reduction with Complex Metal Hydrides", Interscience Publishers Inc., New York, N.Y. 1956; W.G. Brown, Org. Reactions 6 , 469 (1951); R.F. Nystrom und W.G. Brown, J. Am. Chem. Soc. 69 , 2548 (1947)); Aluminiumhydrid in Tetrahydrofuran (in Analogie zu H.C. Brown und N.M. Yoon, J. Am. Chem. Soc. 88 , 1464 (1966)); Natrium-bis-(2-methoxyethoxy)aluminiumhydrid (RED-AL) in Benzol, Toluol oder Xylol (in Analogie zu: V. Bazant u.a., Tetrahedron Letters 1968 , 3303-3306; M. Cerny u.a., Coll. Czechoslov. Chem. Commun. 34 , 1033 (1969)); Lithium-trimethoxyaluminiumhydrid in Tetrahydrofuran (in Analogie zu H.C. Brown und P.M. Weissman, J. Am. Chem. Soc. 87 , 5614 (1965)); Natriumborhydrid in Dimethylsulfoxid und in Gegenwart von Methansulfonsäure oder Schwefelsäure (in Analogie zu: St. R. Wann, P.T. Thorsen und M.M. Kreevoy, J. Org. Chem. 46 , 2579 (1981)); Natriumborhydrid in Tetrahydrofuran und in Gegenwart von Ethandithiol oder Thiophenol (in Analogie zu: Y. Mabi u.a., Chem. Ind. 1976 , 332); Tetra-n-butylammoniumborhydrid in siedendem Dichlormethan (in Analogie zu T. Wakamatsu u.a., Heterocycles 14 , 1437 (1980)); Natrium-acyloxyborhydride, beispielsweise Natrium-acetoxyborhydrid oder Natrium-trifluoracetoxyborhydrid in siedendem Dioxan, Tetrahydrofuran oder Diethylenglykoldimethylether (in Analogie zu: N. Umino u.a., Tetrahedron Letters 1976 , 763); Diboran bzw. Boran-Tetrahydrofuran-Komplex, bevorzugt in Tetrahydrofuran als Lösungsmittel (in Analogie zu: H.C. Brown und P. Heim, J. Am. Chem. Soc. 86 , 3566 (1964); H.C. Brown und P. Heim, J. Org. Chem. 38 , 912 (1973); Z.B. Papanastassiou und R.J. Bruni, J. Org. Chem. 29 , 2870 (1964)); Boran-Dimethylsulfid-Komplex in Tetrahydrofuran (in Analogie zu: H.C. Brown, S. Narasimhan und Y.M. Loi, Synthesis 1981 , 441, ibid. 1981 , 996).

Die erforderlichen Carbonsäureamide der allgemeinen Formel IIIb, lassen sich in üblicher Weise aus Diaminen der allgemeinen Formel IIIc,

$$H - D \qquad (IIIc)$$

und ω-Halogenalkanamiden der allgemeinen Formel VII,

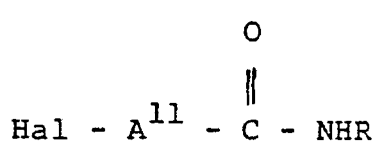

(VII)

in denen

R, $A^{11}$ und D die genannten Bedeutungen haben und Hal ein Chlor-, Brom- oder Jodatom ist, herstellen, beispielsweise unter Verwendung von Halogenwasserstoffacceptoren, wie Natrium- oder Kaliumhydrogencarbonat, Natrium-, Kalium- oder Bariumcarbonat, Diisopropylamin oder Triethylamin, und unter Verwendung polarer protischer oder aprotischer Lösungsmittel wie Methanol, Ethanol, Aceton, Acetonitril oder Dimethylformamid.

Die Herstellung der Zwischenverbindungen der allgemeinen Formel IIIc ist in DE-A-36 26 095 ausführlich beschrieben. Verbindungen der allgemeinen Formel VII sind literaturbekannt oder lassen sich aus gängigen Ausgangsmaterialien nach dem Fachmann geläufigen Verfahren leicht synthetisieren.

b.) Reduktion von Nitrilen der allgemeinen Formel IIId,

$$N \equiv C - A^{11} - D \qquad \text{(IIId)}$$

in der

die Reste $A^{11}$ und D die angegebenen Bedeutungen haben; bei dieser Verfahrensvariante entstehen Zwischenverbindungen der allgemeinen Formel III, in der R die Bedeutung eines Wasserstoffatoms hat. Zur Reduktion von Nitrilen der allgemeinen Formel IIId werden zwei Verfahrensvarianten bevorzugt.

1.) Die katalytische Hydrierung in Gegenwart von Metallen der VIII. Nebengruppe des Periodensystems der Elemente oder von deren Oxiden, z. B. von Raney-Nickel, Raney-Cobalt, Palladium auf Tierkohle, Palladium auf Bariumsulfat, Palladium auf Calciumcarbonat, Platin(IV)oxid, Rhodium auf Aluminiumoxid oder Rhodium in Gegenwart von Lithiumhydroxid. Die Hydrierung kann bei Normaldruck oder erhöhten Wasserstoffdrucken bis zu 1000 bar und bei Temperaturen zwischen -10° C und +200° C durchgeführt werden, wobei die Wahl der zweckmäßigen Hydriertemperatur von der Natur des Katalysators abhängt. Um die Bildung sekundärer Amine zu vermeiden, empfiehlt sich die Durchführung der Hydrierung in Gegenwart von wasserfreiem Ammoniak. Als Lösungsmittel werden Alkohole, wie z. B. Methanol, Ethanol, sowie Ether, wie z. B. Dioxan und Tetrahydrofuran, bevorzugt. Bei der Hydrierung von α-Aminonitrilen, also von solchen Verbindungen der allgemeinen Formel IIId, in der $A^{11}$ eine gegebenenfalls methylierte oder dimethylierte Methylengruppe darstellt, hat sich die Verwendung von Raney-Nickel als Katalysator und das Arbeiten ohne Lösungsmittel oder mit unpolaren Lösungsmitteln, wie z.B. Hexan oder Cyclohexan, bei Temperaturen zwischen +50° C und 100° C und Wasserstoffdrucken zwischen 50 und 200 bar besonders bewährt.

2.) Die Umsetzung mit Metallhydriden und komplexen Hydriden. Geeignete Reduktionsmittel sind zum Beispiel: Lithiumaluminiumhydrid in wasserfreien Ethern, wie Diethylether, Tetrahydrofuran, Dioxan oder Diethylenglykoldimethylether, oder auch in Pyridin und N-Methylmorpholin (siehe z. B.: N.G. Gaylord, "Reduction with Complex Metal Hydrides, Interscience Pulishers, Inc., New York, N.Y. 1956, W.G. Brown, Org. Reactions 6 , 469 (1951)); Lithiumaluminiumhydrid, das durch Zusatz von Aluminiumchlorid modifiziert worden ist (in Analogie zu R.F. Nystrom, J. Amer. Chem. Soc. 77 , 2544 (1955)) in Ethern, wie Diethylether oder Tetrahydrofuran; Lithiumtrimethoxyaluminiumhydrid in Tetrahydrofuran (in Analogie zu H.C. Brown und P.M. Weissman, J. Am. Chem. Soc. 87 , 5614 (1965)); Aluminiumhydrid in Tetrahydrofuran (in Analogie zu H.C. Brown und N.M. Yoon, J. Am. Chem. Soc. 88 , 1464 (1966)); Tetra-n-butylammoniumborhydrid in siedendem Dichlormethan oder Tetrahydrofuran (siehe auch T. Wakamatsu u.a., Heterocycles 14 , 1437 (1980)); Natriumborhydrid in Kombination mit Aluminiumchlorid und unter Verwendung von Ethern, z. B. von Diethylenglykoldimethylether als Lösungsmitteln (in Analogie zu: H.C. Brown und B.C. Subba Rao, J.Am. Chem. Soc. 78 , 2582 (1956)); Natriumborhydrid, kombiniert mit Übergangsmetallsalzen. z. B. Cobalt(II)-chlorid-hexahydrat, Nickel(II)-chlorid-hexahydrat, Nickel(II)-chlorid, Cobalt(II)-benzoat-tetrahydrat, Osmium-(IV)-chlorid-Iridium(III)-chlorid, Platin(II)-chlorid in hydroxylischen oder hydroxylfreien Solventien, beispielsweise in Methanol oder Toluol (in Analogie zu T. Satoh u.a., Tetrahedron Letters 1969 , 4555); Natrium-trifluoracetoxyborhydrid in Dioxan, Tetrahydrofuran oder Diethylenglykoldimethylether (in Analogie zu N. Umino u.a., Tetrahedron Letters 1976 , 2875); Natriumborhydrid in wässerigem Methanol und in Gegenwart von Raney-Nickel (in Analogie zu R.A. Egli, Helv. Chim. Acta 53 , 47 (1970)), Diboran bzw. Boran-Tetrahydrofuran-Komplex, bevorzugt in Tetrahydrofuran oder Diethylenglykoldimethylether als Lösungsmittel (in Analogie zu: H.C. Brown und B.C. Subba Rao, J. Am. Chem. Soc. 82 , 681 (1960); H.C. Brown, P. Heim und N.M. Yoon, J. Am. Chem. Soc. 92 , 1637 (1970)).

Nitrile der allgemeinen Formel IIId können in Analogie zu dem oben für Carbonsäureamide der allgemeinen Formel IIIb angegebenen Verfahren aus Diaminen der allgemeinen Formel IIIc und ω-Halogenalkannitrilen der allgemeinen Formel VIIa,

$$Hal - A^{11} - C \equiv N \qquad \text{(VIIa)}$$

in der

Hal und $A^{11}$ die oben genannten Bedeutungen haben, leicht synthetisiert werden. Nitrile der allgemeinen Formel VIIa sind käuflich oder literaturbekannt oder in Analogie zu literaturbekannten Verfahren erhältlich.

Zur Synthese von solchen Aminonitrilen der allgemeinen Formel IIId, in der $A^{11}$ eine gegebenenfalls alkylierte oder dialkylierte Methylengruppe darstellt, eignen sich auch die verschiedenen Varianten der α-Aminonitril-Synthese nach Strecker, beispielsweise kann man Diamine der allgemeinen Formel IIIc mit Aldehyden, etwa wässriger Formaldehyd-Lösung, und mit Natriumcyanid in Gegenwart von Natriumhydrogensulfit zur Reaktion bringen oder mit Cyanhydrinen entsprechender Aldehyde oder Ketone in Gegenwart von Schleppmitteln, wie etwa Benzol, Toluol oder Xylol, umsetzen.

Die Tricyclen der allgemeinen Formel IV sind aus der Patentliteratur bekannt oder können in enger Anlehnung an publizierte Verfahren aus gängigen Ausgangsmaterialien synthetisiert werden.

Chlorkohlensäurederivate der allgemeinen Formel V und Isocyanate der allgemeinen Formel Va können in Analogie zu literaturbekannten Verfahren erhalten werden (siehe beispielsweise W. Sifken, Liebigs. Ann. Chem. 562, 75 (1949); Houben-Weyl 8 , 117, 119; Ullmann V, 72 (1954); H.H. Saunders und R.J. Slocombe, Chem. Rev. 43 , 203 (1948); R.J. Slocombe, E.E. Hardy, J.H. Saunders und R.L. Jenkins, J. Am. Chem. Soc. 72 , 1888 (1950); H. Habad und A.G. Zeiler, Chem. Rev. 73 , 75 (1973); H.J. Tritchett, Chem. Soc. Rev. 3 , 209 (1979) R. Appel, K. Warning, K.-D. Ziehn und A. Gilak, Chem. Ber. 107 , 2671-4 (1974)).

Halogenkohlensäurederivate der allgemeinen Formel VI sind bekannt.

Die Ausgangsverbindungen der allgemeinen Formel VIII, in der X das Chlor-, Brom- oder Iodatom oder die Hydroxygruppe bedeutet, erhält man durch Aminolyse von Kohlensäurederivaten der allgemeinen Formel II,

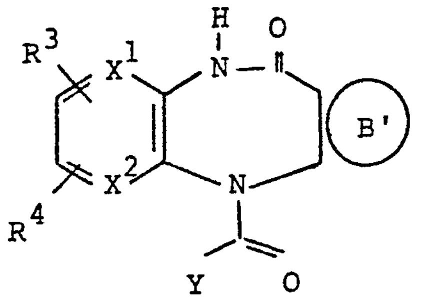

(II)

in der
$R^3$, $R^4$,

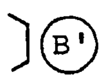

, $X^1$, $X^2$ die angegebenen Bedeutungen haben und

Y ein Halogenatom, bevorzugt das Brom- oder Chloratom, oder den Rest $OR^{11}$ bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit Verbindungen der allgemeinen Formel IX,

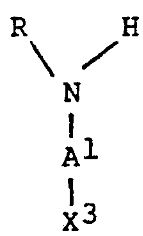

(IX)

in der
R und $A^1$ wie eingangs definiert sind und $X^3$ das Chlor-, Brom- oder Iodatom oder die Hydroxygruppe bedeutet.

Die Umsetzung wird ohne oder bevorzugt in Gegenwart von Lösungsmitteln, wie z. B. von Wasser, Toluol oder von Alkoholen, wie z. B. Methanol, Ethanol oder Isopropanol, ganz bevorzugt jedoch in

Gegenwart aprotischer polarer Lösungsmittel, z. B. von Tetrahydrofuran, 1,4-Dioxan, Acetonitril, N,N-Dimethylformamid, Dimethylsulfoxid, Hexamethylphosphorsäure triamid, oder von Gemischen davon und bei Temperaturen zwischen -10° C und dem Siedepunkt des Reaktionsgemisches, bevorzugt zwischen 40 und 100° C, durchgeführt. Bewährt hat sich die Verwendung zusätzlicher anorganischer oder organischer Basen, z. B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat, Natriumhydrogencarbonat; von tertiären Aminen, z. B. von Triethylamin, Ethyldiisopropylamin, N,N-Dimethylanilin, Pyridin oder von 4-(Dimethylamino)pyridin.

Verbindungen der allgemeinen Formel VIII, in der X die oben genannten Bedeutungen mit Ausnahme einer Hydroxygruppe annimmt, erhält man in Anlehnung an literaturbekannte Verfahren aus Verbindungen der allgemeinen Formel VIII, in denen X die Hydroxygruppe ist. Beispielsweise kann man die Verbindungen der allgemeinen Formel VIII, in denen X die Hydroxygruppe darstellt, durch Reaktion mit Triaryl- oder Trialkylphosphinen und Tetrachlormethan leicht in solche Verbindungen der allgemeinen Formel VIII umwandeln, in denen X das Chloratom bedeutet. Hieraus erhält man wiederum in üblicher Weise durch Umsetzung mit Natriumbromid bzw. Natriumiodid in geeigneten Lösungsmitteln die Verbindungen der allgemeinen Formel VIII, in der X das Brom- bzw. Iodatom darstellt.

Verbindungen der allgemeinen Formel IX schließlich sind käuflich oder können nach literaturbekannten Methoden aus käuflichen Materialien leicht hergestellt werden.

Ein weiterer Gegenstand der Erfindung sind Arzneimittel, die ein oder mehrere basisch substituierte Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,01 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften; insbesondere besitzen sie günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon und Blase.

Solche Verbindungen der allgemeinen Formel I, die aufgrund geringer Lipophilie schlecht resorbiert werden, eignen sich zur inhalativen Anwendung gegen Bronchospasmen, beispielsweise bei asthmatischen Erkrankungen; gegenüber bisher in dieser Indikation eingesetzten quaternären Ammoniumsalzen, beispielsweise Ipratropiumbromid, besitzen die Verbindungen der allgemeinen Formel I den Vorteil der größeren Selektivität, insbesondere sind sie praktisch frei von einer Hemmung exokriner Drüsen.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Untersuchung auf funktionelle Selektivität der anti muscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vitro" Organpräparationen

Dissoziationskonstanten ($K_B$-Werte) wurden "in vitro" am Ileum und spontan schlagenden Vorhof des Meerschweinchens ermittelt. Das Ileum wurde entnommen und im Organbad in Krebs-Henseleit-Lösung inkubiert. Kontraktionen wurden derart durch steigende Konzentrationen von Methacholin (M) hervorgerufen, daß volle Konzentrations-Wirkungskurven aufgenommen werden konnten. Danach wurde M ausgewaschen,

die zu untersuchende Substanz beigefügt und 30 Minuten lang in Kontakt gelassen, und wiederum eine Konzentrations-Wirkungskurve mit M aufgenommen.

Aus dem Dosisverhältnis (DR), das ist das Ausmaß der Verschiebung der Konzentrations-Wirkungskurve, wurde die Dissoziationskonstante nach Arunlakshana und Schild (Brit. J. Pharmacol. 14, 48, 1959) berechnet.

Am isolierten, spontan schlagenden rechten Vorhof reduzierte M konzentrationsabhängig die Herzfrequenz. Durch Zugabe eines Antimuscarinicums wurde dieser Effekt wieder aufgehoben. Dissoziationskonstanten für die muscarinischen Rezeptoren des Vorhofes wurden auf die gleiche Art und Weise wie oben beschrieben gewonnen. Der Vergleich der in beiden Geweben ermittelten Dissoziationskonstanten erlaubte die Identifizierung von cardioselektiven Substanzen. Die Ergebnisse sind in der Tabelle IV enthalten.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

1. Tachycarde Wirkung am wachen Hund,
2. $M_1$-/$M_2$-Selektivität an der Ratte und
3. Speichselsekretionshemmende Wirkung an der Ratte

untersucht.

1. Herzfrequenz steigernde Wirkung am wachen Hund

Die Substanzen wurden entweder intravenös injiziert oder oral verabreicht und die Herzfrequenz mit Hilfe eines Tachygraphen gemessen. Nach einem Kontrollzeitraum wurden steigende Dosen der Verbindung appliziert, um die Herzfrequenz zu erhöhen. Es wurde jeweils dann die nächste Dosis appliziert, wenn der Effekt der vorangegangenen nicht mehr sichtbar war. Die Dosis einer Substanz, die eine Erhöhung um 50 Schläge/Minute ($ED_{50}$) bewirkte, wurde graphisch ermittelt. Jede Substanz wurde an 3 bis 5 Hunden untersucht. Die Ergebnisse sind aus der Tabelle II zu ersehen.

2. $M_1$-/$M_2$-Selektivität an der Ratte

Die angewandte Methode wurde von Hammer und Giachetti (Life Sciences 31, 2991-2998 (1982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle III.

3. Speichselsekretionshemmende Wirkung an der Ratte

Nach Lavy und Mulder (Arch. int. Pharmacodyn. 178, 437-445, (1969)) erhielten mit 1,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle III.

B Bindungsstudien an muscarinischen Rezeptoren:

1.) in vitro: Bestimmung des $IC_{50}$-Wertes

Als Organspender dienten männliche Sprague-Dawley-Ratten mit 180-220 g Körpergewicht. Nach Entnahme von Herz und Submandibularis wurden alle weiteren Schritte in eiskaltem HepesHCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 400 |
| Submandibularis | 1: 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30° C. Die Inkubationsdauer betrug 45 Minuten. Als Radioligand wurde 0,3 n molar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle 1 zu ersehen.

2.) in vivo: Bestimmung der $ID_{50}$-Werte

Für diese Experimente wurden weibliche Ratten mit einem Körpergewicht von ca. 200 g verwendet. Vor Versuchsbeginn wurden die Tiere mit einer Dosis von 1,25 g/kg Urethan narkotisiert. Die Tiere erhielten jeweils die vorgesehene Dosis der Prüfsubstanz durch i.v. Injektion. Nach Ablauf von 15 Minuten wurde auf gleiche Weise 113 ng/kg $^3$H-N-Methylscopolamin ($^3$H-NMS) gegeben. Nach weiteren 15 Minuten wurden die Tiere getötet, das Herz, die Bronchien und die Tränendrüsen entnommen. Diese Organe wurden in Soluene R aufgelöst und die Radioaktivität bestimmt. Diese Messwerte wurden als Totalbindung angenommen. Der Anteil an unspezifischer Bindung wurde als jene Radioaktivität, die durch Gabe von 2 mg/kg Atropin nicht verdrängbar war, definiert. Aus diesen Ver suchen wurden für die einzelnen Organe $ID_{50}$-Werte ermittelt. Die $ID_{50}$-Werte sind Dosen der Prüfsubstanzen, die 50% der spezifischen Bindung von $^3$H-NMS an die muskarinischen Rezeptoren der jeweiligen Organe hemmten. Die Ergebnisse sind in der Tabelle V enthalten.

Nach den vorstehenden Angaben wurden beispielsweise die folgenden Verbindungen untersucht:

A = 5,11-Dihydro-11-[[[2-[2-[(dipropylamino)methyl]-1-piperidinyl]ethyl]amino]carbonyl]-6H-pyrido-[2,3-b][1,4]benzodiazepin-6-on

B = 4-[[[2-[2-[(Diethylamino)methyl]-1-piperidinyl]ethyl]amino]carbonyl]-4,9-dihydro-3-methyl-10H-thieno[3,4-b][1,5]benzodiazepin-10-on

C = 9-Chlor-11-[[[2-[2-[(diethylamino)methyl]-1-piperidinyl]ethyl]amino]carbonyl]-5,11-dihydro-6H-pyrido[2,3-b][1,4]benzodiazepin-6-on

und als Vergleichssubstanzen

X = 11-[[2-[(Diethylamino)methyl]-1-piperidinyl]acetyl]-5,11-dihydro-6H-pyrido[2,3-b][1,4]-benzodiazepin-6-on (siehe US-Patent Nr. 4 550 107)

Y = 5,11-Dihydro-11-[(4-methyl-1-piperazinyl)acetyl]-6H-pyrido[2,3-b][1,4]benzodiazepin-6-on (Pirenzepin, siehe US-Patent Nr. 3 660 380)

und

Z = Atropin

Die folgenden Tabellen enthalten die dabei gefundenen Ergebnisse:

Tabelle I:

Rezeptor-Bindungs-Tests, in vitro:

| Substanz | Rezeptor-Bindungs-Tests $IC_{50}[nMl^{-1}]$ Herz | Submandibularis | Selektivitätsfaktor: Verhältnis $IC_{50}$ Submandibularis zu $IC_{50}$ Herz |
|---|---|---|---|
| A | 20 | 200 | 10 |
| B | 3 | 15 | 5 |
| C | 30 | 150 | 5 |
| X | 150 | 5000 | 33 |
| Y | 1500 | 200 | 0,13 |
| Z | 4 | 4 | 1 |

Tabelle II:

Herzfrequenz steigernde Wirkung am wachen Hund:

| Substanz | Tachycardie (Hund) $ED_{50}[\mu g/kg]$ intravenös | oral | Verhältnis $ED_{50}$ p.o./ $ED_{50}$ i.v. |
|---|---|---|---|
| A | 42 | 300 | 7 |
| X | 120 | 1750 | 15 |

Tabelle III:

$M_1/M_2$-Selektivität und speichelsekretionshemmende Wirkung an der Ratte:

| Substanz | $M_2$-Aktivität (Ratte) $ED_{50}$[µg/kg] i.v. | $M_1$-Aktivität (Ratte) $ED_{50}$[µg/kg] i.v. | Speichelsekretionshemmung (Ratte) $ED_{50}$[µg/kg] i.v. | Verhältnis Speichelsekretionshemmung zu $M_2$-Aktivität |
|---|---|---|---|---|
| A | 19 | 562 | 458 | 24,05 |
| B | 8 | 33 | 78 | 9,75 |
| C | 58 | 360 | 1399 | 24,12 |
| X | 160 | 988 | 4215 | 26,34 |
| Y | 883 | 40 | 84 | 0,1 |
| Z | 4 | 16 | 9 | 2,25 |

Tabelle IV:

Dissoziationskonstanten ($K_B$-Werte) am Ileum und spontan schlagenden Vorhof des Meerschweinchens:

| Substanz | $K_B$ [mol/l] Herz | Ileum |
|---|---|---|
| A | $2,45 \times 10^{-9}$ | $2,57 \times 10^{-8}$ |
| B | $2,40 \times 10^{-10}$ | $3,63 \times 10^{-9}$ |
| C | $4,90 \times 10^{-9}$ | $8,32 \times 10^{-8}$ |
| X | $1,05 \times 10^{-7}$ | $6,17 \times 10^{-7}$ |
| Y | $2,4 \times 10^{-7}$ | $1,55 \times 10^{-7}$ |
| Z | $1,41 \times 10^{-9}$ | $8,13 \times 10^{-10}$ |

Tabelle V:

Rezeptor-Bindungstests, in viro:

| Substanz | $ID_{50}$ [mg/kg] Herz | | | | Verhältnis der $ID_{50}$ Tränendrüse zu $ID_{50}$ des Atrium |
|---|---|---|---|---|---|
| | Atrium | Ventrikel | Bronchien | Tränendrüsen | |
| A | 0,2 | 0,15 | 0,7 | > 10,0 | > 50 |
| B | 0,025 | 0,006 | 0,15 | > 1,0 | > 40 |
| C | 0,20 | 0,09 | 3,0 | 10,0 | 50 |
| X | 1,0 | 0,6 | 15,0 | > 30,0 | > 30 |
| Y | 5,0 | 1,0 | 10,0 | 10,0 | 2 |
| Z | 0,08 | 0,03 | 0,1 | 0,2 | 1,5 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Submandibularis.

Aus den pharmakologischen Daten der vorstehenden Tabelle III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichselsekretion beobachtet wird. Das Verhältnis von $ED_{50}$ der Speichelsekretionshemmung zu $ED_{50}$ der $M_2$-Aktivität zeigt einen ausreichenden Sicherheitsabstand zur Nebenwirkung Mundtrockenheit für die Verbindungen A bis X. Damit ist bewiesen, daß die Substanzen A bis C bei Steigerung der Wirksamkeit (vgl. Tabelle V) eine ausreichende, mit der Substanz X vergleichbare therapeutisch nutzbare Selektivität aufweisen.

Außerdem deuten die pharmakologischen Daten der vorstehenden Tabelle IV auf ein überraschend großes Unterscheidungsvermögen zwischen Herz und glatter Muskulatur. A, B und C sind deutlich wirksamer als X und Y, wobei eine klare Selektivität zugunsten des Herzens sichtbar wird und die Verbindungen A, B und C deutliche Wirksamkeitsvorteile gegenüber X und Y aufweisen. Atropin (= Z) ist ein bekanntermaßen nichtselektives Antimuscarinikum und zeigt unter diesen Modellbedingungen ein umgekehrtes Selektivitätsverhältnis. Die genannten Verbindungen werden hervorragend resorbiert, da sie ein kleines Dosisverhältnis p.o. zu i.v. aufweisen. Je kleiner das Verhältnis $ED_{50}$ p.o. zu $ED_{50}$ i.v. ist, desto besser ist die Resorbierkarkeit der Wirksubstanz.

Die Tabelle V zeigt die bevorzugte Bindung an Rezeptoren des Herzens (Atrium/Ventrikel). Die Substanzen A, B und C zeigen eine entscheidende Verbesserung der Wirkungsstärke gegenüber den Substanzen X und Y am Herzen. Dies ist für die Schaffung eines vagalen Schrittmachers ein wichtiger therapeutischer Vorteil. Diese Effektivitätssteigerung gelang unter Beibehaltung des nutzbaren Selektivitätsabstandes zu den Effekten an den exokrinen Drüsen, wie sich auch aus dem Verhältnis der $ID_{50}$-Werte der Tränendrüse zu den $ID_{50}$-Werten des Atrium ergibt. Das Atropin (= Z) ist dagegen nicht selektiv.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Alle Substanzen der allgemeinen Formel I zeichnen sich durch eine ausgesprochene Hydrolyse-Stabilität aus. Dadurch wird es möglich, lagerbeständige Lösungen zur parenteralen Applikation herzustellen.

Die nachfolgenden Beispiele sollen die Erfindung näher erläutern:

"Fp." beudeutet "Schmelzpunkt", "Z." bedeutet "Zersetzung". Für alle Verbindungen liegen befriedigende Elementaranalysen, IR-, UV-, $^1$H-NMR-, häufig auch Massenspektren vor.

## Herstellung der Ausgangsmaterialien

### Beispiel A

5,11-Dihydro-8-ethyl-6H-pyrido[2,3-b][1,4]benzodiazepin-6-on

Zu der Lösung von 155,7 g (1,21 Mol) 2-Chlor-3-pyridinamin in 417 ml trockenem Dimethylsulfoxid gab man 150,0 g (1,34 Mol) Kalium-tert.-butanolat und rührte 15 Minuten bei einer Reaktionstemperatur von 40 °C. In die entstandene dunkle Lösung wurde innerhalb von 10 Minuten eine Lösung von 218,0 g (1,22 Mol) 2-Amino-5-ethylbenzoesäuremethylester in 50 ml Dimethylsulfoxid eingetropft, die Mischung anschließend noch 40 Minuten auf 50 °C erwärmt. Man ließ erkalten, rührte in 1 Liter Eiswasser ein und stellte durch Zugabe von 20proz. wässriger Salzsäure auf pH 6. Der entstandene Kristallbrei wurde abgesaugt, anschließend in 1 Liter 1proz. wässrigem Ammoniak suspendiert und erneut abgenutscht. Nach dem Trocknen im Umlufttrockenschrank: Farblose Kristalle vom Fp. 145-147 °C, die ohne weitere Reinigung direkt weiter umgesetzt wurden, $R_F$ 0,9 (Macherey-Nagel, Polygram® SIL G/$UV_{254}$, pre-coated plastic sheets for TLC, Fließmittel: Dichlormethan/Essigsäureethylester/Methanol/Cyclohexan/wässriges Ammoniak 59/25/7,5/7,5/1 v/v).

Ausbeute: 270,5g (81 % der Theorie).

160,0 g (0,58 Mol) des erhaltenen 2-Amino-5-ethyl-N-(2-chlor-3-pyridinyl)benzamids wurden in 256 ml Sulfolan gelöst und unter Rühren 20 Minuten auf 170 °C erhitzt. Man ließ erkalten, verrührte mit 1 Liter

Acetonitril, nutschte den entstandenen Kristallbrei ab und wusch ihn nacheinander mit 50 ml Acetonitril und 100 ml konz. Ammoniak. Man kristallierte aus heißer 70proz. Essigsäure um und erhielt nach dem Trocknen im Umluftrockenschrank 114,0 g (48% der Theorie) an fahlgelben Kristallen vom Fp. 232-234 °C, $R_F$ 0,78 (Macherey-Nagel, Polygram® SIL G/$UV_{254}$, pre-coated plastic sheets for TLC, Fließmittel: Dichlormethan/Essigsäureethylester 1/1 v/v).

Beispiel B

8-Brom-5,11-dihydro-6H-pyrido[2,3-b][1,4]benzodiazepin-6-on

Die Mischung aus 8,2 g (0,0251 Mol) 2-Amino-5-brom-2-(2-chlor-3-pyridinyl)benzamid (Fp. 187-188 °C nach Umkristallisieren aus Acetonitril; hergestellt Analog Beispiel A aus 2-Chlor-3-pyridinamin und 2-Amino-5-brombenzoesäuremethylester in einer Ausbeute von 83% der Theorie), 16 ml Sulfolan und 0,3 g konz. Schwefelsäure wurde 3 Stunden unter Durchleiten von Stickstoff und Rühren auf eine Reaktionstemperatur von 140 °C erhitzt. Nach dem Erkalten wurde die Mischung in 150 ml Eiswasser eingetragen, das ausgefallene Reaktionsprodukt anschließend abfiltriert und unter Verwendung von Dichlormethan/Cyclohexan/Essigsäureethylester 17/66/17 (v/v) zum Eluieren chromatographisch an Kieselgel (35-70 mesh) von Nebenprodukten befreit. Nach dem Umkristallisieren aus Dimethylacetamid und Acetonitril erhielt man 4,4 g (60% der Theorie) an schwach gelblichen Kristallen vom Fp. 338-340 °C.

Beispiel C

5,11-Dihydro-9-methyl-6H-pyrido[2,3-b][1,4]benzodiazepin-6-on

127,0 g (0,485 Mol) analog Beispiel A aus 2-Chlor-3-pyridinamin und 2-Amino-4-methylbenzoesäuremethylester (siehe: F. Mayer und R. Schulze, Ber. dtsch. chem. Ges. 58 , 1465 [1925]) hergestelltes 2-Amino-N-(2-chlor-3-pyridinyl)-4-methylbenzamid (Fp. 150-152 °C) wurden in 500 ml Sulfolan suspendiert und nach Zusatz von 1,4 ml konz. Schwefelsäure 3 Stunden lang unter Rühren auf 130 °C erhitzt. Man ließ auf 80 °C abkühlen, versetzte mit 500 ml Toluol und ließ die Mischung über Nacht bei Zimmertemperatur stehen. Die entstandenen Kristalle wurden abgenutscht, in 0,75 Liter Dichlormethan suspendiert, abermals abgenutscht und gründlich mit Dichlormethan gewaschen. Nach dem Trocknen im Umlufttrockenschrank erhielt man 70,2 g (64% der Theorie) an ganz schwach gelben Kristallen vom Fp. 286-288 °C, die ohne weitere Reinigung weiter umgesetzt wurden.

Beispiel D

4-(Chlorcarbonyl)-4,9-dihydro-3-methyl-10H-thieno [3,4-b][1,5]benzodiazepin-10-on

8,2 g (0,036 Mol) 4,9-Dihydro-3-methyl-10H-thieno[3,4-b][1,5]benzodiazepin-10-on wurden in 365 ml Dichlormethan suspendiert und nach Zugabe von 40 ml (0,08 Mol) einer 20proz. Phosgen-Lösung in Toluol 7 Tage bei Zimmertemperatur gerührt. Die entstandene Lösung wurde dreimal mit je 80 ml Wasser gewaschen, über Natriumsulfat getrocknet und im Vakuum vom Lösungsmittel befreit. Die gesuchte Verbindung wurde säulenchromatographisch an MN-Kieselgel 60 (70-230 mesh ASTM) unter Verwendung von Dichlormethan/Essigsäureethylester/Petrolether 5/5/4 (v/v) als Eluens von Begleitern befreit. Man erhielt 6,1 g (58% der Theroie) an farblosen Kristallen vom Fp. 238-239 °C, $R_F$ 0,77 (Macherey-Nagel, Polygram® SIL G/$UV_{254}$, pre-coated plastic sheets for TLC, Fließmittel: Dichlormethan/Essigsäureethylester/Petrolether 5/5/4 v/v).

Entsprechend erhielt man:

4-(Chlorcarbonyl)-4,9-dihydro-10H-thieno[3,4-b][1,5]benzodiazepin-10-on vom Fp. 244-245 °C (Z.) (Acetonitril);

4-(Chlorcarbonyl)-4,9-dihydro-1-methyl-10H-thieno[3,4-b] [1,5]benzodiazepin-10-on vom Fp. 235-236 °C (aus Chloroform);

4-(Chlorcarbonyl)-4,9-dihydro-1,3-dimethyl-10H-thieno[3,4-b][1,5]benzodiazepin-10-on als amorphes, schaumiges Produkt, das ohne Reinigung weiter umgesetzt wurde;

3-Chlor-4-(chlorcarbonyl)-4,9-dihydro-10H-thieno[3,4-b][1,5]benzodiazepin-10-on vom Fp. 238-240° C (Ethanol);

aus 4,9-Dihydro-3-methyl-10H-thieno[3,4-b][1,5]benzodiazepin-10-on und Chlorkohlensäuremethylester das 4,9-Dihydro-4-(methoxycarbonyl)-3-methyl-10H-thieno[3,4-b] [1,5]benzodiazepin-10-on;

aus 4,9-Dihydro-3-methyl-10H-thieno[3,4-b][1,5]benzodiazepin-10-on und Chlorkohlensäurebenzylester das 4-(Benzyloxycarbonyl)-4,9-dihydro-3-methyl-10H-thieno[3,4-b][1,5]benzodiazepin-10-on.

## Beispiel E

### 11-(Chlorcarbonyl)-5,11-dihydro-6H-pyrido[2,3-b][1,4] benzodiazepin-6-on

Zu der Suspension von 158,3 g (0,75 Mol) 5,11-Dihydro-6H-pyrido[2,3-b][1,4]benzodiazepin-6-on in 3 Liter Dioxan tropfte man innerhalb von 45 Minuten 730 ml (1,46 Mol) einer 20proz. Lösung von Phosgen in Toluol und rührte anschließend noch 2 Stunden bei Raumtemperatur. Man versetzte mit 1,5 l Wasser, rührte nochmals 2 Stunden bei Zimmertemperatur und nutschte den entstandenen Niederschlag ab. Man wusch das Produkt gründlich mit Wasser und trocknete es im Umlufttrockenschrank. Farblose Kristalle vom Fp. 239-240° C.

Ausbeute: 178,8 g (87% der Theorie).

## Beispiel F

### 9-Chlor-11-(chlorcarbonyl)-5,11-dihydro-6H-pyrido[2,3-b] [1,4]benzodiazepin-6-on

11,6 g (0,0472 Mol) 9-Chlor-5,11-dihydro-6H-pyrido[2,3-b][1,4]benzodiazepin-6-on, suspendiert in einem Gemisch aus 450 ml wasserfreiem Dioxan und 6,4 ml (0,08 Mol) Pyridin, wurden bei 45° C innerhalb von 20 Minuten mit 47,1 ml (0,094 Mol) einer 20proz. Lösung von Phosgen in Toluol versetzt. Man rührte das Reaktionsgemisch 2 Stunden bei 45° C und anschließend 4 Stunden bei 60° C. Die Mischung wurde noch heiß filtriert, das Filtrat im Vakuum eingedampft, der verbliebene kristallisierende Rückstand erneut in 100 ml Dioxan suspendiert, 2 Stunden unter Rückfluß gekocht, abermals eingedampft, mit viel Wasser verrührt und abgenutscht. Das Produkt hatte nach dem Trocknen im Umlufttrockenschrank Fp. 265-267° C, $R_F$ 0,70 (Macherey-Nagel, Polygram® SIL G/$UV_{254}$, pre-coated plastic sheets for TLC, Fließmittel: Dichlormethan/Essigsäureethylester 1/1 v/v)

Ausbeute: 12,0 g (83% der Theorie).

Entsprechend wurden hergestellt:

11-(Chlorcarbonyl)-7-fluor-5,11-dihydro-6H-pyrido[2,3-b][1,4]benzodiazepin-6-on;

11-(Chlorcarbonyl)-8-fluor-5,11-dihydro-6H-pyrido[2,3-b][1,4]benzodiazepin-6-on;

11-(Chlorcarbonyl)-9-fluor-5,11-dihydro-6H-pyrido[2,3-b][1,4]benzodiazepin-6-on;

11-(Chlorcarbonyl)-8-methyl-5,11-dihydro-6H-pyrido[2,3-b][1,4]benzodiazepin-6-on in einer Ausbeute von 92% der Theorie; Fp. 209-210° C (Z); $R_F$ 0,70 (Macherey-Nagel, Polygram® SIL G/$UV_{254}$, pre-coated plastic sheets for TLC, Fließmittel: Dichlormethan/Essigsäureethylester 1/1 v/v);

8-Chlor-11-(chlorcarbonyl)-5,11-dihydro-6H-pyrido[2,3-b][1,4]benzodiazepin-6-on in einer Ausbeute von 72% der Theorie; Fp. 216-220° C; $R_F$ 0,78 (Macherey-Nagel, Polygram® SIL G/$UV_{254}$, pre-coated plastic sheets for TLC, Fließmittel: Dichlormethan/Essigsäureethylester 1/1 v/v)

11-(Chlorcarbonyl]-8-ethyl-5,11-dihydro-6H-pyrido[2,3-b][1,4]benzodiazepin-6-on in einer Ausbeute von 80% der Theorie; Fp. 206-208° C; $R_F$ 0,69 (Macherey-Nagel, Polygram® SIL G/$UV_{254}$, pre-coated plastic sheets for TLC, Fließmittel: Dichlormethan/Essigsäureethylester/Methanol/Cyclohexan/konz. Ammoniak 54,5/37/4/4/0,5 v/v);

11-(Chlorcarbonyl)-9-methyl-5,11-dihydro-6H-pyrido[2,3-b][1,4]benzodiazepin-6-on mit $R_F$ 0,70 (Macherey-Nagel, Polygram® SIL G/$UV_{254}$, pre-coated plastic sheets for TLC, Fließmittel: Dichlormethan/Essigsäureethylester 1/1/ v/v);

8-Brom-9-(chlorcarbonyl)-5,11-dihydro-6H-pyrido[2,3-b][1,4]benzodiazepin-6-on mit $R_F$ 0,75 (Macherey-Nagel, Polygram® SIL G/$UV_{254}$, pre-coated plastic sheets for TLC, Fließmittel: Dichlormethan/Essigsäureethylester 1/1 v/v) bzw. 0,80 (Fließmittel: Dichlormethan/Essigsäureethylester/Methanol/Cyclohexan/konz. Ammoniak 59/25/7,5/7,5/1 v/v);

4-(Chlorcarbonyl)-1,3-dimethyl-1,4,9,10-tetrahydropyrazolo[4,3-e]pyrido[3,2-b][1,4]diazepin-10-on vom Fp. 208-212 °C (Acetonitril) in einer Ausbeute von 70% der Theorie;

11-(Chlorcarbonyl)-6,11-dihydro-5H-pyrido[2,3-b][1,5]benzodiazepin-5-on vom Fp. 246-247 °C in einer Ausbeute von 85% der Theorie;

5-(Chlorcarbonyl-5,11-dihydro-10H-pyrido[3,2-b][1,4]benzodiazepin-10-on,

4-(Chlorcarbonyl)-1,3-dimethyl-1,4,9,10-tetrahydropyrrolo[3,2-b][1,5]benzodiazepin-10-on;

3-Chlor-4-(chlorcarbonyl)-1-methyl-1,4,9,10-tetrahydropyrrolo[3,2-b][1,5]benzodiazepin-10-on;

4-(Chlorcarbonyl)-1-methyl-1,4,9,10-tetrahydropyrazolo[4,3-e]pyrido[3,2-b][1,4]diazepin-10-on;

4-(Chlorcarbonyl)-1,3-dimethyl-1,4,9,10-tetrahydropyrazolo[4,3-b][1,5]benzodiazepin-10-on;

6-Chlor-5-(chlorcarbonyl)-5,10-dihydro-11H-dibenzo[b,e][1,4]diazepin-11-on.

## Beispiel G

### 2-[2-[(Diethylamino)methyl]-1-piperidinyl]acetonitril

Die Mischung aus 236,5 ml (ca. 1,1 Mol) einer 37 proz. wässerigen Natriumhydrogensulfit-Lösung und 110 ml (ca. 1,1 Mol) einer 40 proz. Formalin-Lösung wurde 30 Minuten auf 60 °C erwärmt. Man ließ auf 30 °C abkühlen, tropfte die Lösung von 170,3 g (1,0 Mol) 2-[(Diethylamino)methyl]piperidin in 70 ml Wasser zu, wobei sich die Mischung von selbst auf 40 °C erwärmte, und rührte weitere 2 Stunden ohne äußere Wärmezufuhr. Dann gab man die Lösung von 53,9 g (1,1 Mol) Natriumcyanid in 172 ml Wasser zu und rührte nochmals 2 Stunden bei Zimmertemperatur. Die obere organische Schicht wurde in 500 ml Cyclohexan aufgenommen, die wäßrige Phase noch viermal mit je 125 ml Cyclohexan extrahiert. Die vereinigten Extrakte wurden über Natriumsulfat getrocknet, eingedampft und im Vakuum destilliert. Man erhielt das gewünschte 2-[2-[(Diethylamino)methyl]-1-piperidinyl]acetonitril als farbloses Öl vom Sdp.$_{19\ mm\ Hg}$ 155-156 °C in einer Ausbeute von 172,0 g (82% der Theorie).

Entsprechend wurden erhalten:

2-[2-[3-(Dimethylamino)-1-propyl]-1-piperidinyl]acetonitril, $R_F$ 0,75 (Macherey-Nagel, Polygram® SIL G/UV$_{254}$, pre-coated plastic sheets for TLC, Fließmittel: Dichlormethan/Methanol/Cyclohexan/konz. Ammoniak 68/15/15/2 v/v) in einer Ausbeute von 99% der Theorie;

2-[2-[2-(Dimethylamino)ethyl]-1-piperidinyl]acetonitril von Sdp.$_{15\ mm\ Hg}$ 140-142 °C in einer Ausbeute von 61% der Theorie;

2-[3-[(Diethylamino)methyl]-4-morpholinyl]acetonitril, $R_F$ 0,80 (Macherey-Nagel, Polygram® SIL G/UV$_{254}$, pre-coated plastic sheets for TLC, Fließmittel: Dichlormethan/Essigsäureethylester/Cyclohexan/Methanol/konz. Ammoniak 58/25/8/8/1 v/v) in einer Ausbeute von 88% der Theorie;

2-[2-[(Dipropylamino)methyl]-1-piperidinyl]acetonitril vom Sdp.$_{18\ mm\ Hg}$ 162-170 °C, $R_F$ 0,95 (Macherey-Nagel, Polygram® SIL G/UV$_{254}$, pre-coated plastic sheets for TLC, Fließmittel: Dichlormethan/Methanol/Cyclohexan/konz. Ammoniak 68/15/15/2 v/v) in einer Ausbeute von 55% der Theorie;

2-[2-[4-(Diethylamino)butyl]-1-piperidinyl]acetonitril vom Sdp.$_{0,1\ mm\ Hg}$ 103-113 °C in einer Ausbeute von 71% der Theorie;

2-[2-[(1-Piperidinyl)methyl]-1-piperidinyl]acetonitril vom Sdp.$_{0,2\ mm\ Hg}$ 110-113 °C in einer Ausbeute von 66% der Theorie;

2-[3-(Diethylamino)-hexahydro-1H-azepin-1-yl]acetonitril vom Sdp.$_{20\ mm\ Hg}$ 164-170 °C in einer Ausbeute von 83% der Theorie;

2-[2-[(4-Morpholinyl)methyl]-1-piperidinyl]acetonitril vom Sdp.$_{0,4\ mm\ Hg}$ 134-139 °C in einer Ausbeute von 84% der Theorie;

2-[2-[(1-Pyrrolidinyl)methyl]-1-piperidinyl]acetonitril vom Sdp.$_{20\ mm\ Hg}$ 168-170 °C in einer Ausbeute von 67% der Theorie;

2-[2-[[Bis-(methylethyl)amino]methyl]-1-piperidinyl]acetonitril;

2-[2-[(Butylethylamino)methyl]-1-piperidinyl]acetonitril in einer Aubeute von 83 % der Theorie; farbloses Öl vom Sdp.$_{15\ mm\ Hg}$ 168-171 °C;

2-[2-[(Hexahydro-1H-azepin-1-yl)methyl]-1-piperidinyl]acetonitril in einer Ausbeute von 74 % der Theorie; farbloses Öl vom Sdp.$_{0,3\ mm\ Hg}$ 120-124 °C;

(+)-2-[2-[(Diethylamino)methyl]-1-piperidinyl]acetonitril vom Sdp.$_{19\ mm\ Hg}$ 155-157 °C in einer Ausbeute von 79% der Theorie;

(-)-2-[2-[(Diethylamino)methyl]-1-piperidinyl]acetonitril vom Sdp.$_{19\ mm\ Hg}$ 154-156 °C in einer Ausbeute von 84% der Theorie;

(+)-2-[2-[(Dipropylamino)methyl]-1-piperidinyl]acetonitril vom Sdp.$_{18\ mm\ Hg}$ 163-168 °C in einer Ausbeute von 58% der Theorie;

(-)-2-[2-[(Dipropylamino)methyl]-1-piperidinyl]acetonitril vom Sdp.$_{18\ mm\ Hg}$ 161-169 °C in einer Ausbeute von 53% der Theorie;

2-[3-[(Diethylamino)methyl]-1-pyrrolidinyl]acetonitril;

2-[3-[(Dipropylamino)methyl]-1-pyrrolidinyl]acetonitril;

2-[3-[(1-Piperidinyl)methyl]-1-pyrrolidinyl]acetonitril;

2-[3-[[(Cyclohexyl)methylamino]methyl]-1-pyrrolidinylacetonitril, das roh weiterverarbeitet wurde, in einer Ausbeute von 91 % der Theorie;

2-[2-[[(Cyclopentyl)methylamino]methyl]-1-piperidinyl]acetonitril in einer Ausbeute von 95 % der Theorie; farbloses, leicht bewegliches Öl vom $R_F$ 0,95 (Macherey-Nagel, Polygram® SIL G/$UV_{254}$, pre-coated plastic sheets for TLC; Fließmittel: Dichlormethan/Essigsäureethylester/Methanol/Cyclohexan/konz. Ammoniak 59/25/7,5/7,5/1 v/v/v/v/v/);

2-[3-[(Diethylamino)methyl]-1-piperidinyl]acetonitril in einer Ausbeute von 90% der Theorie; $R_F$ 0,51 (Macherey-Nagel, Polygram® SIL G/$UV_{254}$, pre-coated plastic sheets for TLC, Fließmittel: Dichlormethan/Essigsäureethylester/Cyclohexan/Methanol/konz. Ammoniak 58/25/8/8/1 v/v);

2-[3-[(Dipropylamino)methyl]-1-piperidinyl]acetonitril in einer Ausbeute von 76% der Theorie; $R_F$ 0,545 (MachereyNagel, Polygram® SIL G/$UV_{254}$, pre-coated plastic sheets for TLC, Fließmittel: Dichlormethan/Methanol/konz. Ammoniak 89/10/1 v/v);

2-[3-[(Hexahydro-1H-azepin-1-yl)methyl]-1-piperidinyl]acetonitril in einer Ausbeute von 34% der Theorie; $R_F$ 0,515 (Macherey-Nagel, Polygram® SIL G/$UV_{254}$, pre-coated plastic sheets for TLC, Fließmittel: Dichlormethan/Essigsäureethylester/Cyclohexan/Methanol/konz. Ammoniak 58/15/8/8/1 v/v);

2-[3-[3-(1-Piperidinyl)propyl]-1-piperidinyl]acetonitril in einer Ausbeute von 62% der Theorie;

2-[3-[(1-Piperidinyl)methyl]-1-piperidinyl]acetonitril in einer Ausbeute von 94% der Theorie;

2-[3-[2-(1-Piperidinyl)ethyl]-1-piperidinyl]aceonitril in einer Ausbeute von 72 % der Theorie; $R_F$ 0,49 (Merck DC-Fertigplatten Kieselgel 60 $F_{254}$; Fließmittel: Dichlormethan/Methanol/konz. Ammoniak 90/10/1 v/v/v);

2-[3-[4-(Dimethylamino)butyl]-1-piperidinyl]acetonitril in einer Ausbeute von 93 % der Theorie: $R_F$ 0,39 (DC-Untersuchung wie vorstehend);

2-[3-[4-(1-Pyrrolidinyl)butyl]-1-piperidinyl]acetonitril in einer Ausbeute von 90 % der Theorie, farbloses Öl vom $R_F$ 0,29 (DC-Untersuchung wie vorstehend);

2-[3-[4-(Diethylamino)butyl]-1-piperidinyl]acetonitril in einer Ausbeute von 94 % der Theorie; farbloses, dünnflüssiges Öl vom $R_F$ 0,60 (DC-Untersuchung wie vorstehend);

2-[2-[4-(1-Piperidinyl)butyl]-1-piperidinyl]acetonitril in einer Ausbeute von 83 % der Theorie; farbloses Öl vom Sdp.$_{0,1\ mm\ Hg}$ 149-150 °C;

2-[2-[[Bis-(methylethyl)amino]methyl]-1-piperidinyl]acetonitril in einer Ausbeute von 67 % der Theorie; farbloses Öl vom Sdp.$_{15\ mm\ Hg}$ 163-165 °C;

2-[2-[(Ethylpropylamino)methyl]-1-piperidinyl]acetonitril in einer Ausbeute von 71 % der Theorie; farbloses Öl vom Sdp.$_{10\ mm\ Hg}$ 150-152 °C.

Beispiel H

3-[2-[(Diethylamino)methyl]-1-piperidinyl]propannitril

In einem 500 ml Dreihalskolben mit Rührer, Tropftrichter, Rückflußkühler und Innenthermometer löste man 42,5 g (0,25 Mol) 2-[(Diethylamino)methyl]piperidin in 250 ml Ethanol. Zu dieser Lösung wurden unter Rühren 17,8 ml (0,267 Mol) Acrylnitril getropft, wobei man die Innentemperatur unter 30 °C hielt. Nach Zugabe von 6 Tropfen einer methanolischen Triton-B-Lösung rührte man noch 24 Stunden bei Raumtemperatur. Das Lösungsmittel wurde bei vermindertem Druck abgezogen, der Rückstand in Vakumm destilliert. Farbloses Öl vom Sdp.$_{0,3\ mm\ Hg}$ 110-113 °C.

Ausbeute: 40,3 g (72% der Theorie).

Entsprechend wurde erhalten:

3-[2-[(Dimethylamino)methyl]-1-piperidinyl]propannitril, Sdp.$_{0,3\ mm\ Hg}$ 90-92 °C, in einer Ausbeute von 78% der Theorie.

Beispiel I

2-[2-[3-(Dimethylamino)propyl]-1-piperidinyl]acetonitril

Die Mischung aus 17,0 g (0,1 Mol) 2-[3-(Dimethylamino)propyl]piperidin, 15,2 ml (0,11 Mol) Triethylamin, 100 ml wasserfreiem Tetrahydrofuran und 6,9 ml (0,11 Mol) Chloracetonitril wurde 8 Stunden unter Rückfluß gekocht. Die erkaltete Mischung wurde filtriert, das Filtrat im Vakuum eingedampft. Man erhielt 3,2 g (15% der Theorie) der obigen Verbindung vom $R_F$ 0,75 (Macherey-Nagel, Polygram® SIL G/UV$_{254}$, pre-coated plastic sheets for TLC, Fließmittel: Dichlormethan/Methanol/Cyclohexan/konz. Ammoniak 68/15/15/2.

Entsprechend wurden erhalten:

(±)-2-[2-[2-(Diethylamino)ethyl]-1-pyrrolidinyl]acetonitril in einer Ausbeute von 98% der Theorie;

(±)-2-[2-[(Diethylamino)methyl]-1-pyrrolidinyl]acetonitril vom Sdp.$_{12\ mm\ Hg}$ 122-125 °C in einer Ausbeute von 73% der Theorie.

Beispiel K

4-[2-[(Diethylamino)methyl]-1-piperidinyl]butannitril

Die Mischung aus 17,0 g (0,1 Mol) 2-[(Diethylamino)methyl]piperidin, 16,3 g (0,11 Mol) 4-Brombutannitril, 12,7 g (0,12 Mol) Natriumcarbonat und 100 ml Tetrahydrofuran wurde 6 Stunden rührend unter Rückfluß gekocht. Nach dem Erkalten wurde vom Unlöslichen abfiltriert, das Lösungsmittel abgetrieben und der Rückstand an Kieselgel (35-70 mesh ASTM) unter Verwendung von Dichlormethan/Essigsäureethylester/Cyclohexan/Methanol/konz. Ammoniak 3,5/1,5/0,46/0,46/0,06 zum Eluieren chromatographisch gereinigt. Man erhielt 20,9 g (88% der Theorie) eines farblosen Öls mit $R_F$ 0,5 (Macherey-Nagel, Polygram® SIL G/UV$_{254}$, pre-coated plastic sheets for TLC, Fließmittel: wie oben).

Entsprechend wurden erhalten:

Aus 2-[(Diethylamino)methyl]piperidin und 6-Bromhexannitril in einer Ausbeute von 84% der Theorie das 6-[2-[(Diethylamino)methyl]-1-piperidinyl]hexannitril vom Sdp.$_{0,2\ mm\ Hg}$ 135-136 °C;

aus 2-[(Dimethylamino)methyl]piperidin und 4-Brombutannitril in einer Ausbeute von 47% der Theorie das 4-[2-[(Dimethylamino)methyl]-1-piperidinyl]butannitril vom $R_F$ 0,45 (DC-Untersuchung wie oben), das nach längerem Stehen kristallisierte. Fp. 137-139 °C;

aus 2-[(Dipropylamino)methyl]piperidin und 2-Bromacetonitril das (±)-2-[2-[(Dipropylamino)methyl]-1-piperidinyl]acetonitril, $R_F$ 0,9 (Macherey-Nagel, Polygram® SIL G/UV$_{254}$, pre-coated plastic sheets for TLC, Fließmittel: Dichlormethan/Essigsäureethylester 50:50 v/v).

Beispiel L

2-[2-[(Dimethylamino)methyl]-1-piperidinyl]acetamid

28,0 g (0,197 Mol) 2-[(Dimethylamino)methyl]piperidin wurden in 200 ml Ethanol gelöst und nach Zusatz von 22,4 g (0,24 Mol) Chloracetamid und 20,1 g (0,24 Mol) Natriumhydrogencar bonat 48 Stunden unter Rückfluß gekocht. Die erkaltete Mischung wurde filtriert, das Filtrat im Vakuum eingedampft. Man erhielt 22,0 g (56% der Theorie) eines hellgelben Öls vom $R_F$ 0,50 (Macherey-Nagel, Polygram® SIL G/UV$_{254}$, pre-coated plastic sheets for TLC, Fließmittel: Dichlormethan/Methanol/Cyclohexan/konz. Ammoniak 68/15/15/2 v/v), das ohne weitere Reinigung weiterverarbeitet wurde.

Entsprechend wurden erhalten:

Aus 2-[2-(Diethylamino)ethyl]piperidin und Chloracetamid in einer Ausbeute von 79% der Theorie das 2-[2-[2-(Diethylamino)ethyl]-1-piperidinyl]acetamid, $R_F$ 0,50 (DC-Untersuchung wie oben);

aus 2-[3-(Diethylamino)propyl]piperidin und Chloracetamid in einer Ausbeute von 65% der Theorie das

2-[2-[3-(Diethylamino)propyl]-1-piperidinyl]acetamid, $R_F$ 0,50 (DC-Unter suchung wie oben);

aus 2-[(Diethylamino)methyl]piperidin und N-Methyl-chloracetamid in einer Ausbeute von 81% der Theorie das 2-[2-[(Diethylamino)methyl]-1-piperidinyl]-N-methyl-acetamid, $R_F$ 0,85 (DC-Untersuchung wie oben);

aus 2-[(Dimethylamino)methyl]piperidin und N-Methyl-chloracetamid in einer Ausbeute von 70% der Theorie das 2-[2-[(Dimethylamino)methyl]-1-piperidinyl]-N-methyl-acetamid, $R_F$ 0,80 (DC-Untersuchung wie oben);

aus trans-2-[[(4-Hydroxycyclohexyl)methylamino]methyl]piperidin und N-Methyl-chloracetamid in einer Ausbeute von 48% der Theorie das trans-2-[2 [[(4-Hydroxycyclohexyl)methyl amino]methyl]-1-piperidinyl]-N-methyl-acetamid vom Fp. 104-105°C;

aus 2-[2-(Diethylamino)ethyl]piperidin und N-Methyl-chloracetamid in einer Ausbeute von 94% der Theorie das 2-[2-[2-(Diethylamino)ethyl]-1-piperidinyl]-N-methyl-acetamid, $R_F$ 0,80 (DC-Untersuchung wie oben);

aus 4-[2-(Diethylamino)ethyl]piperidin und Iodacetamid in einer Ausbeute von 95% der Theorie das 2-[4-[2-(Diethylamino)ethyl]-1-piperidinyl]acetamid;

aus 4-[(Diethylamino)methyl]piperidin und Iodacetamid in einer Ausbeute von 92% der Theorie das 2-[4-[(Diethylamino)methyl]-1-piperidinyl]acetamid;

aus 4-[2-(1-Piperidinyl)ethyl]piperidin und Iodacetamid in einer Ausbeute von 89% der Theorie das 2-[4-[2-(1-Piperidinyl)ethyl]-1-piperidinyl]acetamid;

aus 4-[4-(1-Piperidinyl)butyl]piperidin und Iodacetamid in einer Ausbeute von 60% der Theorie das 2-[4-[4-(1-Piperidinyl)butyl]-1-piperidinyl]acetamid vom Fp. 112-116°C;

aus N,N-Bis(methylethyl)-hexahydro-4-pyridinpropanamid und Iodacetamid das 2-[4-[3-[Bis-(methylethyl)amino]-3-oxopropyl]-1-piperidinyl]acetamid, das roh weiterverarbeitet wurde, in einr Ausbeute von 30 % der Theorie;

aus 4-[3-(Dipropylamino)propyl]piperidin und Iodacetamid das 2-[4-[3-(Dipropylamino)propyl]-1-piperidinyl]acetamid, das ohne Reinigung weiterverarbeitet wurde, in einer Ausbeute von 43 %, $R_F$ 0,50 (DC-Untersuchung wie oben);

aus N,N-Diethyl-hexahydro-4-pyridinpropanamid und Iodacetamid das kristalline 2-[4-[3-(Diethylamino)-3-oxopropyl]- 1-piperidinyl]acetamid, das roh weiterverarbeitet wurde, in einr Ausbeute von 81 % der Theorie;

aus 4-[3-(1-Piperidinyl)propyl]piperidin und Iodacetamid in einer Ausbeute von 96% der Theorie das 2-[4-[3-(1-Piperidinyl)propyl]-1-piperidinyl]acetamid vom Fp. 94-97°C.

## Beispiel M

[2-[2-[2-(Dimethylamino)ethyl]-1-piperidinyl]ethyl]carb aminsäure-O-ethylester

Zu dem Gemisch aus 14,9 g (0,075 Mol) 2-[2-[2-(Dimethylamino)ethyl]-1-piperidinyl]ethylamin, 12,7 ml (0,091 Mol) Triethylamin und 150 ml wasserfreiem Ethanol tropfte man 8,6 ml (0,09 Mol) Chlorkohlensäureethylester und rührte die Mischung anschließend noch 2 Stunden bei Raumtemperatur. Die flüchtigen Bestandteile wurden im Wasserstrahlvakuum abdestilliert, der Rückstand in 30 ml Wasser aufgenommen; die erhaltene Lösung wurde mit Kaliumcarbonat gesättigt und mit Essigsäureethylester erschöpfend extrahiert. Die vereinigten Extrakte wurden über Natriumsulfat getrocknet, filtriert und erneut eingedampft. Der verbliebene Rückstand ergab nach der Destillation im Feinvakuum ($Sdp._{0,2\ mm\ Hg}$ 141-143°C) ein farbloses Öl in einer Ausbeute von 14,0 g (69 % der Theorie).

Entsprechend wurden erhalten:

[4-[2-[(Dimethylamino)methyl]-1-piperidinyl]butyl]carbaminsäure-O-ethylester, $R_F$ 0,3 (Macherey-Nagel, Polygram® SIL G/$UV_{254}$, pre-coated plastic sheets for TLC, Fließmittel: Dichlormethan/Essigsäureethylester/Cyclohexan/Methanol/konz. Ammoniak 58/25/8/8/1), in einer Ausbeute von 83% der Theorie;

[4-[2-[(Diethylamino)methyl]-1-piperidinyl]butyl]carbaminsäure-O-methylester, farbloses Öl, in einer Ausbeute von 93% der Theorie.

## Beispiel N

2-[2-[(Diethylamino)methyl]-1-piperidinyl]ethanamin

In einen 500 ml-Dreihalskolben mit Rührer, Zweihalsaufsatz, Tropftrichter und Rückflußkühler mit Calciumchloridrohr gab man 200 ml wasserfreien Ether und 10,7 g (0,282 Mol) Lithiumaluminiumhydrid und tropfte unter ständigem Rühren eine Lösung von 43,0 g (0,189 Mol) 2-[2-[(Diethylamino)methyl]-1-piperidinyl]acetamid in 60 ml absolutem Ether so zu, daß der Ether mäßig siedete. Nach Beendigung des Zutropfens rührte man noch 6 Stunden unter gleichzeitigem Rückflußkochen, zersetzte das überschüssige Lithiumaluminiumhydrid anschließend durch aufeinanderfolgende tropfenweise Zugabe von 11 ml Wasser, 11 ml 15-proz. wässeriger Natronlauge und 33 ml Wasser und filtrierte das ausgefallene Aluminiumoxidhydrat ab. Das Filtrat wurde über Natriumsulfat getrocknet, vom Lösungsmittel befreit und im Feinvakuum destilliert. Man erhielt 12,7 g (31% der Theorie) eines farblosen Öls vom $Sdp._{0,2\ mm\ Hg}$ 80-82 °C, $R_F$ 0,5 (Macherey-Nagel, Polygram® SIL G/$UV_{254}$, pre-coated plastic sheets for TLC, Fließmittel: Dichlormethan/Methanol/Cyclohexan/konz. Ammoniak 68/15/15/2).

Entsprechend wurden erhalten:

Aus 3-[2-[(Diethylamino)methyl]-1-piperidinyl]propannitril in einer Ausbeute von 72% der Theorie das 3-[2-[(Diethylamino)methyl]-1-piperidinyl]propanamin vom $Sdp._{0,2\ mm\ Hg}$ 92-93C;

aus 2-[2-[(Diethylamino)methyl]-1-piperidinyl]acetonitril in einer Ausbeute von 87% der Theorie das 2-[2-[(Diethylamino)methyl]-1-piperidinyl]ethanamin vom $Sdp._{15\ mm\ Hg}$ 135-138C;

aus 2-[2-[(Dimethylamino)methyl]-1-piperidinyl]acetamid in einer Ausbeute von 42% der Theorie das 2-[2-[(Dimethylamino)methyl]-1-piperidinyl]ethanamin vom $Sdp._{0,15\ mm\ Hg}$ 62-65C;

aus 3-[2-[(Dimethylamino)methyl]-1-piperidinyl]propannitril in einer Ausbeute von 53% der Theorie das 3-[2-[(Dimethylamino)methyl]-1-piperidinyl]propanamin vom $Sdp._{0,3\ mm\ Hg}$ 80-82C;

aus 2-[2-[2-(Diethylamino)ethyl]-1-piperidinyl]acetamid in einer Ausbeute von 47% der Theorie das 2-[2-[2-(Diethylamino)ethyl]-1-piperidinyl]ethanamin vom $Sdp._{15\ mm\ Hg}$ 155-161C; $R_F$ 0,3 (DC-Untersuchung wie oben);

aus 2-[2-[3-(Dimethylamino)propyl]-1-piperidinyl]acetonitril in einer Ausbeute von 75% der Theorie das 2-[2-[3-(Dimethylamino)propyl]-1-piperidinyl]ethanamin vom $Sdp._{26\ mm\ Hg}$ 153-154C;

aus 2-[2-[3-(Diethylamino)propyl]-1-piperidinyl]acetamid in einer Ausbeute von 38% der Theorie das 2-[2-[3-(Diethylamino)propyl]-1-piperidinyl]ethanamin vom $Sdp._{15\ mm\ Hg}$ 169-173C;

aus 4-[2-[(Diethylamino)methyl]-1-piperidinyl]butannitril in einer Ausbeute von 36% der Theorie das 4-[2-[(Diethylamino)methyl]-1-piperidinyl]butanamin vom $R_F$ 0,2 (DC-Bedingungen wie oben, jedoch Fließmittel: Dichlormethan/Cyclohexan/Methanol/konz. Ammoniak 4,08/0,92/0,92/0,12);

aus 2-[2-[2-(Dimethylamino)ethyl]-1-piperidinyl]acetonitril in einer Ausbeute von 67% der Theorie das 2-[2-[2-(Dimethylamino)ethyl]-1-piperidinyl]ethanamin vom $Sdp._{18\ mm\ Hg}$ 131-134 °C;

aus 2-[2-[(Diethylamino)methyl]-1-piperidinyl]-N-methylacetamid in einer Ausbeute von 33% der Theorie das 2-[2-[(Diethylamino)methyl]-1-piperidinyl]-N-methyl-ethanamin vom $Sdp._{15\ mm\ Hg}$ 148-152 °C;

aus 6-[2-[(Diethylamino)methyl]-1-piperidinyl]hexannitril in einer Ausbeute von 44% der Theorie das 6-[2-[(Diethylamino)methyl]-1-piperidinyl]hexanamin vom $Sdp._{0,2\ mm\ Hg}$ 149-151 °C;

aus 2-[2-[(Dimethylamino)methyl]-1-piperidinyl]-N-methylacetamid in einer Ausbeute von 39% der Theorie das 2-[2-[(Dimethylamino)methyl]-1-piperidinyl]-N-methyl-ethanamin vom $Sdp._{18\ mm\ Hg}$ 127 -32 °C;

aus trans 2-[2-[[(4-Hydroxycyclohexyl)methylamino]methyl]-1-piperidinyl]-N-methyl-acetamid in einer Ausbeute von 90% der Theorie das trans-2-[2-[[(4-Hydroxycyolchexyl)methylamino]methyl]-1-piperidinyl]-N-methyl-ethanamin, $R_F$ 0,1 (DC-Bedingungen wie oben), das nach längerem Stehenlassen zu kristallisieren beginnt;

aus 2-[2-[2-(Diethylamino)ethyl]-1-piperidinyl]-N-methylacetamid in einer Ausbeute von 31% der Theorie das 2-[2-[2-(Diethylamino)ethyl]-1-piperidinyl]-N-methyl-ethanamin vom $Sdp._{18\ mm\ Hg}$ 155-159 °C;

aus 4-[2-[(Dimethylamino)methyl]-1-piperidinyl]butannitril in einer Ausbeute von 86% der Theorie das 4-[2-[(Dimethylamino)methyl]-1-piperidinyl]butanamin, $R_F$ 0,15 (DC-Untersuchung wie vorstehend);

aus trans-2-[2-[[(4-Hydroxycyclohexyl)methylamino]methyl]-1-piperidinyl]acetamid in einer Ausbeute von 57% der Theorie das trans-2-[2-[[(4-Hydroxycyclohexyl)methylamino]methyl]-1-piperidinyl]ethanamin, $R_F$ 0,15 (DC-Untersuchung wie vorstehend);

aus 2-[4-[2-(1-Piperidinyl)-2-oxoethyl]-1-piperidinyl]acetamid in einer Ausbeute von 83% der Theorie das 2-[4-[2-(1-Piperidinyl)ethyl]-1-piperidinyl]ethanamin, das ohne weitere Reinigung verwendet wurde;

aus [2-[2-[2-(Dimethylamino)ethyl]-1-piperidinyl]ethyl]carbaminsäure-O-ethylester in einer Ausbeute von 67% der Theorie das 2-[2-[2-(Dimethylamino)ethyl]-1-piperidinyl]-N-methylethanamin vom $Sdp._{20\ mm\ Hg}$ 148-151 °C;

aus [4-[2-[(Dimethylamino)methyl]-1-piperidinyl]butyl]carbaminsäure-O-ethylester in einer Ausbeute von 84% der Theorie das 4-[2-[(Dimethylamino)methyl]-1-piperidinyl]-N-methylbutanamin, $R_F$ 0,1 (DC-Untersuchung wie oben);

aus [4-[2-[(Diethylamino)methyl]-1-piperidinyl]butyl]carbaminsäure-O-ethylester in einer Ausbeute von 84% der Theorie das 4-[2-[(Diethylamino)methyl]-1-piperidinyl]-N-methylbutanamin, vom $Sdp._{0,1\ mm\ Hg}$ 103-110 °C;

aus 2-[2-[2-(Diethylamino)ethyl]-1-pyrrolidinyl]acetonitril in einer Ausbeute von 56% der Theorie das 2-[2-[2-(Diethylamino)ethyl]-1-pyrrolidinyl]ethanamin vom $Sdp._{20\ mm\ Hg}$ 125-130 °C;

aus 2-[2-[(Diethylamino)methyl]-1-pyrrolidinyl]acetonitril in einer Ausbeute von 58% der Theorie das 2-[2-[(Diethylamino)methyl]-1-pyrrolidinyl]ethanamin vom $Sdp._{12\ mm\ Hg}$ 114-117 °C;

aus 2-[4-[(Diethylamino)methyl]-1-piperidinyl]acetamid in einer Ausbeute von 32% der Theorie das 2-[4-[(Diethylamino)methyl]-1-piperidinyl]ethanamin vom $Sdp._{12\ mm\ Hg}$ 132-138 °C;

aus 2-[4-[3-(Diethylamino)-3-oxopropyl]-1-piperidinyl]acetamid in einer Ausbeute von 74% der Theorie das 2-[4-[3-(Diethylamino)propyl]-1-piperidinyl]ethanamin, das ohne weitere Reinigung verwendet wurde; $R_F$ 0,2 (Macherey-Nagel, Polygram® SIL G/$UV_{254}$, pre-coated plastic sheets for TLC, Fließmittel: Dichlormethan/Cyclohexan/Methanol/konz. Ammoniak 68/15/15/2 v/v/v/v);

aus 2-[4-[3-(Dimethylamino)propyl]-1-piperidinyl]acetamid das 2-[4-[3-(Dimethylamino)propyl]-1-piperidinyl]ethanamin;

aus 2-[3-[2-(1-Piperidinyl)ethyl]-1-piperidinyl]acetonitril in einer Ausbeute von 90 % der Therorie das 2-[3-[2-(1-Piperidinyl)ethyl]-1-piperidinyl]ethanamin;

aus 2-[3-[4-(Dimethylamino)butyl]-1-piperidinyl]acetonitril in einer Ausbeute von 94 % der Theorie das 2-[3-[4-(Dimethylamino)butyl]-1-piperidinyl]ethanamin;

aus 2-[3-[4-(1-Pyrrolidinyl)butyl]-1-piperidinyl]acetonitril in einer Ausbeute von 96 % der Therorie das 2-[3-[4-(1-Pyrrolidinyl)butyl]-1-piperidinyl]ethanamin;

aus 2-[3-[4-Diethylamino)butyl]-1-piperidinyl]acetonitril in einer Ausbeute von 86 % der Theorie das 2-[3-[4-(Diethylamino)butyl]-1-piperidinyl]ethanamin;

aus 2-[4-[4-(Diethylamino)butyl]-1-piperidinyl]acetamid das 2-[4-[4-(Diethylamino)butyl]-1-piperidinyl]-ethanamin;

aus 2-[4-[2-(Diethylamino)ethyl]-1-piperidinyl]acetamid in einer Ausbeute von 94% der Theorie das 2-[4-[2-(Diethylamino)ethyl]-1-piperidinyl]ethanamin;

aus 2-[3-[(Dipropylamino)methyl]-1-piperidinyl]acetonitril in einer Ausbeute von 98% das 2-[3-[(Dipropylamino)methyl]-1-piperidinyl]ethanamin vom $Sdp._{0,035\ mm\ Hg}$ 95-97 °C;

aus 2-[3-[(Hexahydro-1H-azepin-1-yl)methyl]-1-piperidinyl]acetonitril in einer Ausbeute von 93% der Theorie das 2-[3-[(Hexahydro-1H-azepin-1-yl)methyl]-1-piperidinyl]ethanamin vom $Sdp._{0,03\ mm\ Hg}$ 109-110 °C;

aus 2-[3-[3-(1-Piperidinyl)propyl]-1-piperidinyl]acetonitril in einer Ausbeute von 98% der Theorie das 2-[3-[3-(1-Piperidinyl)propyl]-1-piperidinyl]ethanamin;

aus 2-[3-[(1-Piperidinyl)methyl]-1-piperidinyl]acetonitril in einer Ausbeute von 72% der Theorie das 2-[3-[(1-Piperidinyl)methyl]-1-piperidinyl]ethanamin;

aus 2-[3-[(Diethylamino)methyl]-1-piperidinyl]acetonitril in einer Ausbeute von 95% der Theorie das 2-[3-[(Diethylamino)methyl]-1-piperidinyl]ethanamin vom $Sdp._{0,3\ mm\ Hg}$ 84-85 °C;

aus 2-[3-[(1-Piperidinyl)methyl]-1-pyrrolidinyl]acetonitril in einer Ausbeute von 66% der Theorie das 2-[3-[(1-Piperidinyl)methyl]-1-pyrrolidinyl]ethanamin vom $Sdp._{12\ mm\ Hg}$ 135-137 °C;

aus 2-[3-[(Dipropylamino)methyl]-1-pyrrolidinyl]acetonitril in einer Ausbeute von 61% der Theorie das 2-[3-[(Dipropylamino)methyl]-1-pyrrolidinyl]ethanamin vom $Sdp._{12\ mm\ Hg}$ 133-136 °C;

aus 2-[3-[[(Cyclohexyl)methylamino]methyl]-1-pyrrolidinyl]acetonitril in einer Ausbeute von 54 % der Theorie das 2-[3-[[(Cyclohexyl)methylamino]methyl]-1-pyrrolidinyl]ethanamin vom $Sdp._{5\ mm\ Hg}$ 160-165 °C;

aus 2-[4-[4-(1-Piperidinyl)butyl]-1-piperdidinyl]acetamid das 2-[4-[4-(1-Piperidinyl)butyl]-1-piperidinyl]-ethanamin, das ohne weitere Reinigung umgesetzt wurde; $R_F$ 0,3 (Macherey-Nagel, Polygram® SIL G/$UV_{254}$, pre-coated plastic sheets for TLC; Fließmittel: Dichlormethan/Cyclohexan/Methanol/konz. Ammoniak 68/15/15/2 v/v/v/v/);

aus 2-[4-[4-(Dipropylamino)butyl]-1-piperidinyl]acetamid das 2-[4-[4-(Dipropylamino)butyl]-1-piperidinyl]-ethanamin

aus 2-[4-[3-[Bis-(methylethyl)amino]propyl]-1-piperidinyl]acetamid das 2-[4-[3-[Bis-(methylethyl)amino]-propyl]-1-piperidinyl]ethanamin;

aus 2-[4-[3-(1-Piperidinyl)propyl]-1-piperidinyl]acetamid das 2-[4-[3-(1-Piperidinyl)propyl]-1-piperidinyl]-ethanamin;

aus 2-[4-[3-(Dipropylamino)propyl]-1-piperidinyl]acetamid das 2-[4-[3-(Dipropylamino)propyl]-1-piperidinyl]ethanamin.

Beispiel O

2-[2-[(Diethylamino)methyl]-1-piperidinyl]ethanamin

172 g (0,822 Mol) 2-[2-[(Diethylamino)methyl]-1-piperidinyl]acetonitril wurden in 1300 ml n-Hexan gelöst und nach Zusatz von 57,0 g Raney-Nickel und 300 ml flüssigem Ammoniak bei einem Wasserstoffdruck von 70 bar und 90 °C in einem Schüttelautoklav bis zur Beendigung der Wasserstoffaufnahme (ca. 12 Stunden) katalytisch hydriert. Nach Abkühlen und Entspannen des Autoklaven wurde vom Katalysator abfiltriert und das Lösungsmittel abgedampft. Der verbliebene Rückstand wurde durch Vakuumdestillation gereinigt. Man erhielt 152,0 g (87% der Theorie) eines farblosen Öls vom $Sdp._{15\ mm\ Hg}$ 135-138 °C.

Entsprechend wurden erhalten:

aus 2-[2-[(4-Morpholinyl)methyl]-1-piperidinyl]acetonitril in einer Ausbeute von 55 % der Theorie das 2-[2-[(4-Morpholinyl)methyl]-1-piperidinyl]ethanamin, $Sdp._{0,4\ mm\ Hg}$ 123-124 °C;

aus 2-[2-[4-(1-Piperidinyl)butyl]-1-piperidinyl]acetonitril in einer Ausbeute von 42 % der Theorie das 2-[2-[4-(1-Piperidinyl)butyl]-1-piperidinyl]ethanamin, $Sdp._{0,1\ mm\ Hg}$ 130-133 °C;

aus 2-[2-[[(Cyclopentyl)methylamino]methyl]-1-piperidinyl]acetonitril in einer Ausbeute von 76 % der Thorie das 2-[2-[[(Cyclopentyl)methylamino]methyl]-1-piperidinyl]ethanamin, $Sdp._{0,7\ mm\ Hg}$ 136-140 °C;

aus 2-[2-[[Bis-(methylethyl)amino]methyl]-1-piperidinyl]acetonitril in einer Ausbeute von 43 % der Theorie das 2-[2-[[Bis-(methylethyl)amino]methyl]-1-piperidinyl]ethanamin, $Sdp._{21\ mm\ Hg}$ 158-159 °C;

aus 2-[2-[(Butylethylamino)methyl]-1-piperidinyl]acetonitril in einer Ausbeute von 61 % der Theorie das 2-[2-[(Butylethylamino)methyl]-1-piperidinyl]ethanamin, $Sdp._{15\ mm\ Hg}$ 158-160 °C;

aus 2-[2-[(Hexahydro-1H-azepin-1-yl)methyl]-1-piperidinyl]acetonitril in einer Ausbeute von 54 % der Theorie das 2-[2-[(Hexahydro-1H-azepin-1-yl)methyl]-1-piperidinyl]ethanamin, $Sdp._{0,3\ mm\ Hg}$ 105-107 °C;

aus 2-[2-[(Ethylpropylamino)methyl]-1-piperidinyl]acetonitril in einer Ausbeute von 57 % der Thorie das 2-[2-[(Ethylpropylamino)methyl]-1-piperidinyl]ethanamin, $Sdp._{10\ mm\ Hg}$ 144-147 °C;

aus 2-[3-[(Diethylamino)methyl]-4-morpholinyl]acetonitril in einer Ausbeute von 78% der Theorie das 2-[3-[(Diethylamino)methyl]-4-morpholinyl]ethanamin, $R_F$ 0,2 (Macherey-Nagel, Polygram® SIL G/$UV_{254}$, pre-coated plastic sheets for TLC, Fließmittel: Dichlormethan/Cyclohexan/Methanol/konz. Ammoniak/Essigsäureethylester 63/11,5/11,5/1,5/12,5 v/v);

aus 4-[2-[(Diethylamino)methyl]-1-piperidinyl]butannitril in einer Ausbeute von 99% der Theorie das 4-[2-[(Diethylamino)methyl]-1-piperidinyl]butanamin, $R_F$ 0,19 (Macherey-Nagel, Polygram® SIL G/$UV_{254}$, pre-coated plastic sheets for TLC, Fließmittel: Dichlormethan/Cyclohexan/Methanol/konz. Ammoniak 102/23/23/3;

aus 2-[2-[4-(Diethylamino)butyl]-1-piperidinyl]acetonitril in einer Ausbeute von 54% der Theorie das 2-[2-[4-(Diethylamino)butyl]-1-piperidinyl]ethanamin vom $Sdp._{0,2\ mm\ Hg}$ 97-99 °C;

aus 2-[2-[(1-Piperidinyl)methyl]-1-piperidinyl]acetonitril in einer Ausbeute von 38% der Theorie das 2-[2-[(1-Piperidinyl)methyl]-1-piperidinyl]ethanamin vom $Sdp._{0,1\ mm\ Hg}$ 87-88 °C;

aus 2-[2-[(Dipropylamino)methyl]-1-piperidinyl]acetonitril in einer Ausbeute von 40% der Theorie das 2-[2-[(Dipropylamino)methyl]-1-piperidinyl]ethanamin vom $Sdp._{18\ mm\ Hg}$ 154-156 °C;

aus (-)-2-[2-[(Diethylamino)methyl]-1-piperidinyl]acetonitril in einer Ausbeute von 51% der Theorie das (-)-2-[2-[(Diethylamino)methyl]-1-piperidinyl]ethanamin vom $Sdp._{18\ mm\ Hg}$ 143-145 °C; $[\alpha]_D^{20}$ = - 80,06° (Ethanol);

aus (+)-2-[2-[(Diethylamino)methyl]-1-piperidinyl]acetonitril in einer Ausbeute von 63% der Theorie das (+)-2-[2- [(Diethylamino)methyl]-1-piperidinyl]ethanamin vom $Sdp._{16\ mm\ Hg}$ 142-144 °C; $[\alpha]_D^{20}$ = + 80,56° (Ethanol);

aus 2-[3-(Diethylamino)-hexahydro-1H-azepin-1-yl]acetonitril in einer Ausbeute von 61% der Theorie das 2-[3-(Diethylamino)hexahydro-1H-azepin-1-yl]ethylamin vom $Sdp._{17\ mm\ Hg}$ 143-147 °C;

aus 2-[(Diethylamino)methyl]-1-(2-methyl-2-nitropropyl)piperidin (hergestellt in Analogie zu H.G. Johnson, J. Am. Chem. Soc. 68 , 12 [1946] und G.H. Butler und F.N. Mc Millan, ibid. 72 , 2978 [1950]) in einer Ausbeute von 22% der Theorie das 2-[2-[(Diethylamino)methyl]-1-piperidinyl]-1,1-dimethylethanamin, $Sdp._{0,3\ mm\ Hg}$ 85-92 °C;

aus 2-[2-[(1-Pyrrolidinyl)methyl]-1-piperidinyl]acetonitril in einer Ausbeute von 64% der Theorie das 2-[2-[(1-Pyrrolidinyl)methyl]-1-piperidinyl]ethanamin vom $Sdp._{20\ mm\ Hg}$ 156-158 °C.

Beispiel P

11-[[[2-Bromethyl]amino]carbonyl]-5,11-dihydro-6H-pyrido [2,3-b][1,4]benzodiazepin-6-on

Zu der Mischung aus 5,0 g (0,0183 mol) 11-(Chlorcarbonyl)5,11-dihydro-6H-pyrido[2,3-b][1,4]-benzodiazepin-6-on, 50 ml Tetrahydrofuran, 4,08 g (0,02 mol) 2-Bromethylammoniumbromid und 7,0 g (0,051 mol) wasserfreiem Kaliumcarbonat gab man 10 ml Wasser, wodurch sich der Ansatz von selbst auf 30 bis 35°C erwärmte, und rührte anschließend noch 30 Minuten ohne äußere Wärmezufuhr. Während anfangs vorübergehend alle Feststoffe in Lösung gegangen waren, hatte sich gegen Ende der Umsetzung ein farbloser Niederschlag gebildet. Man rührte die Mischung in 400 ml Wasser ein, rühre abermals 30 Minuten bei Zimmertemperatur und nutschte das entstandene Produkt ab. Man wusch gründlich mit Wasser und trocknete über Nacht im Umlufttrockenschrank bei 40°C. Ausbeute 6,15 g (93 % der Theorie) an farblosen Kristallen vom Fp. 217-220°C (Z.).

Entsprechend wurden erhalten:

11-[[[2-Bromethyl]amino]carbonyl]-9-chlor-5,11-dihydro-6H-pyrido[2,3-b][1,4]benzodiazepin-6-on in einer Ausbeute von 67 % der Theorie; das Produkt wurde ohne Reinigung weiter umgesetzt; $R_F$ 0,72 (Macherey-Nagel, Polygram® SIL G/UV$_{254}$, pre-coated plastic sheets for TLC, Fließmittel: Dichlormethan/Cyclohexan/Methanol/konz. Ammoniak 68/15/15/2 v/v/v/v);

11-[[[3-Brompropyl]amino]carbonyl]-5,11-dihydro-6H-pyrido[2,3-b][1,4]benzodiazepin in einer Ausbeute von 96 % der Theorie; farblose Kristalle vom Fp. 139-140°C; $R_F$ 0,8 (Macherey-Nagel, Polygram® SIL G/UV$_{254}$, pre-coated plastic sheets for TLC, Fließmittel: Dichlormethan/Cyclohexan/Methanol/konz. Ammoniak 68/15/15/2 v/v/v/v);

4-[[[2-Bromethyl]amino]carbonyl]-4,9-dihydro-3-methyl-10H-thieno[3,4-b][1,5]benzodiazepin-10-on in einer Ausbeute von 54 % der Theorie; farblose Kristalle vom Fp. 218°C; $R_F$ 0,70 (Macherey-Nagel, Polygram® SIL G/UV$_{254}$, pre-coated plastic sheets for TLC, Fließmittel: Essigsäureethylester/Cyclohexan/Methanol/konz. Ammoniak 80/10/10/1 v/v/v/v).

Herstellung der Endprodukte:

Beispiel 1

11-[[[2-[2-[(Diethylamino)methyl]-1-piperidinyl]ethyl] amino]carbonyl]-5,11-dihydro-6H-pyrido[2,3-b][1,4]-benzo diazepin-6-on-hydrochlorid

Die Mischung aus 33,4 g (0,122 Mol) 11-(Chlorcarbonyl)-5,11-dihydro-6H-pyrido[2,3-b][1,4]-benzodiazepin-6-on, 28,0 g (0,131 Mol) 2-[2-[(Diethylamino)methyl]-1-piperidinyl]ethanamin und 730 ml wasserfreiem Acetonitril wurde 3 Stunden bei einer Reaktionstemperatur von 45°C gerührt. Nach einstündigem Stehen im Eisbad wurde der entstandene Niederschlag abgenutscht und mit eiskaltem Acetonitril gewaschen. Man löste in 400 ml heißem Ethanol, fügte 4,6 g Aktivkohle zu, filtrierte noch heiß und engte das Filtrat im Vakuum ein. Der verbliebene Rückstand wurde in 150 ml heißem 2-Propanol gelöst und nach vorsichtiger Zugabe von 800 ml Essigsäureethylester 2 Stunden bei einer Temperatur von 75°C gerührt. Man ließ anschließend 2 Stunden bei Eisbadtemperatur stehen, nutschte die entstandenen Kristalle ab und wusch sie dreimal gründlich mit je 20 ml Essigsäureethylester. Man erhielt 37,0 g (62% der Thorie) an farblosen Kristallen vom Fp. 200-202°C.

Beispiel 2

11-[[[2-[2-[(Diethylamino)methyl]-1-piperidinyl]ethyl] amino]carbonyl]-5,11-dihydro-6H-pyrido[2,3-b][1,4]-benzo diazepin-6-on

3,2 g (0,0117 Mol) 11-(Chlorcarbonyl)-5,11-dihydro-6H-pyrido[2,3-b][1,4]benzodiazepin-6-on und 1,27 g (0,012 Mol) wasserfreies Natriumcarbonat wurden in 100 ml trockenem Aceto nitril zusammen mit 3,0 g (0,014 Mol) 2-[2-[(Diethylamino)methyl]-1-piperidinyl]ethanamin unter Rühren 4 Stunden auf 60°C erhitzt. Es wurde heiß filtriert, das Filtrat wurde abgekühlt und die auskristallisierte Substanz abgenutscht. Man erhielt 2,7 g (51% der Theorie) an farblosen Kristallen vom Fp. 175-176°C.

Beispiel 3

11-[[[3-[2-[(Diethylamino)methyl]-1-piperidinyl]propyl] amino]carbonyl]-5,11-dihydro-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 3-[2-[(Diethylamino)methyl]-1-piperidinyl]propanamin in einer Ausbeute von 47% der Theorie. Farblose Kristalle vom Fp. 160-161 °C (Acetonitril).

Beispiel 4

5,11-Dihydro-11-[[[2-[2-[(dimethylamino)methyl]-1-piperi dinyl]ethyl]amino]carbonyl]-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 2-[2-[(Dimethylamino)methyl]-1-piperidinyl]ethanamin in einer Ausbeute von 53% der Theorie. Farblose Kristalle vom Fp. 189-190 (Acetonitril).

Beispiel 5

5,10-Dihydro-5-[[[3-[2-[(dimethylamino)methyl]-1-piperi dinyl]propyl]amino]carbonyl]-11H-dibenzo[b,e][1,4]-diazepin-11-on

Hergestellt analog Beispiel 2 aus 5-(Chlorcarbonyl)-5,10-dihydro-11H-dibenzo[b,e][1,4]diazepin-11-on und 3-[2-[(Dimethylamino)methyl]-1-piperidinyl]propanamin in einer Ausbeute von 80% der Theorie. Farblose Kristalle vom Fp. 131-133 °C (Diisopropylether)

Beispiel 6

5,11-Dihydro-11-[[[3-[2-[(dimethylamino)methyl]-1-piperi dinyl]propyl]amino]carbonyl-6H-pyrido[2,3-b][1,4]-benzo diazepin-6-on

Hergestellt analog Beispiel 2 aus 11-Chlorcarbonyl-5,11-dihydro-6H-pyrido[2,3-b][1,4]benzodiazepin-6-on und 3-[2-[(Dimethylamino)methyl]-1-piperidinyl]propanamin in einer Ausbeute von 54% der Theorie. Farblose Kristalle vom Fp. 160-162 °C (Acetonitril).

Beispiel 7

5-[[[2-[2-[(Diethylamino)methyl]-1-piperidinyl]ethyl] amino]carbonyl]-5,10-dihydro-11H-dibenzo[b,e][1,4] diazepin-11-on

Hergestellt analog Beispiel 2 aus 5-(Chlorcarbonyl)-5,10-dihydro-11H-dibenzo[b,e][1,4]diazepin-11-on und 2-[2-[(Diethylamino)methyl]-1-piperidinyl]ethanamin in einer Ausbeute von 74% der Theorie. Farblose Kristalle vom Fp. 123-125 °C (Diisopropylether).

Beispiel 8

5-[[[3-[2-[(Diethylamino)methyl]-1-piperidinyl]propyl] amino]carbonyl]-5,10-dihydro-11H-dibenzo[b,e][1,4]dia zepin-11-on

Hergestellt analog Beispiel 2 aus 5-Chlorcarbonyl-5,10-dihydro-11H-dibenzo[b,e][1,4]diazepin-11-on und 3-[2-[(Diethylamino)methyl]-1-piperidinyl]propanamin in einer Ausbeute von 54% der Theorie. Fp. 148,0-149,5 °C (Diisopropylether). Löslich in Wasser.

Beispiel 9

5,10-Dihydro-5-[[[2-[2-[(dimethylamino)methyl]-1-piperi dinyl]ethyl]amino]carbonyl]-11H-dibenzo[b,e][1,4]-diazepin-11-on

Hergestellt analog Beispiel 2 aus 5-(Chlorcarbonyl)-5,10-dihydro-11H-dibenzo[b,e][1,4]diazepin-11-on und 2-[2-[(Dimethylamino)methyl]-1-piperidinyl]ethanamin in einer Ausbeute von 73% der Theorie. Farblose Kristalle vom Fp. 152-154 °C (Diisopropylether).

Beispiel 10

11-[[[2-[2-[2-(Diethylamino)ethyl]-1-piperidinyl]ethyl] amino]carbonyl]-5,11-dihydro-6H-pyrido[2,3-b][1,4]ben zodiazepin-6-on

Hergestellt analog Beispiel 2 aus 11-(Chlorcarbonyl)-5,11-dihydro-6H-pyrido[2,3-b][1,4]benzodiazepin-6-on und 2-[2- [2-(Diethylamino)ethyl]-1-piperidinyl]ethanamin in einer Ausbeute von 65% der Theorie. Farblose Kristalle vom Fp. 161-162 °C (Acetonitril).

Beispiel 11

11-[[[2-[2-[3-(Diethylamino)propyl]-1-piperidinyl]ethyl] amino]carbonyl]-5,11-dihydro-6H-pyrido[2,3-b][1,4]ben zodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[3-(Diethylamino)propyl]-1-piperidinyl]ethanamin in einer Ausbeute von 58% der Theorie. Farblose Kristalle vom Fp. 157-158 °C (Acetonitril).

Beispiel 12

5,11-Dihydro-11-[[[2-[2-[3-(dimethylamino)propyl]-1-piperi dinyl]ethyl]amino]carbonyl]-6H-pyrido[2,3-b][1,4]-benzodi azepin-6-on

Hergestellt analog Beispiel 2 aus 11-(Chlorcarbonyl)-5,11-dihydro-6H-pyrido[2,3-b][1,4]benzodiazepin-6-on und 2-[2-[3-(Dimethylamino)propyl]-1-piperidinyl]ethanamin in einer Ausbeute von 61% der Theorie. Farblose Kristalle vom Fp. 154-155 °C (Acetonitril).

Beispiel 13

D,L-11-[[[4-[2-[(Diethylamino)methyl]-1-piperidinyl]butyl] amino]carbonyl]-5,11-dihydro-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 4-[2-[(Diethylamino)methyl]-1-piperidinyl]butanamin in einer Ausbeute von 90% der Theorie. Farblose Kristalle vom Fp. 136-137° C (Acetonitril).

Beispiel 14

D,L-5,11-Dihydro-11-[[[2-[2-[2-(dimethylamino)ethyl]-1-piperidinyl]ethyl]amino]carbonyl]-6H-pyrido[2,3-b]-[1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[2-(Dimethylamino)ethyl]-1-piperidinyl]ethanamin in einer Ausbeute von 48% der Theorie. Farblose Kristalle vom Fp. 158-159° C (Acetonitril).

Beispiel 15

D,L-11-[[[2-[2-[(Diethylamino)methyl]-1-piperidinyl] ethyl]methylamino]carbonyl]-5,11-dihydro-6H-pyrido [2,3-b][1,4]benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[(Diethylamino)methyl]-1-piperidinyl]-N-methyl-ethanamin in einer Ausbeute von 54% der Theorie. Farblose Kristalle vom Fp. 68-70° C (Petrolether).

Beispiel 16

D,L-5,11-Dihydro-11-[[[2-[2-[dimethylamino)methyl]-1-pi peridinyl]ethyl]methylamino]carbonyl]-6H-pyrido-[2,3-b][1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[(Dimethylamino)methyl]-1-piperidinyl]-N-methyl-ethanamin in einer Ausbeute von 19% der Theorie. Farblose Kristalle vom Fp. 64-67° C (Petrolether).

Beispiel 17

(R,S)-11-[[[6-[2-[(Diethylamino)methyl]-1-piperidinyl] hexyl]amino]carbonyl]-5,11-dihydro-6H-pyrido[2,3-b]-[1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 6-[2-[(Diethylamino)methyl]-1-piperidinyl]hexanamin in einer Ausbeute von 56% der Theorie. Farblose Kristalle vom Fp. 111-112° C (Acetonitril).

Beispiel 18

trans-5,11-Dihydro-11-[[[2-[2-[[(4-hydroxycyclohexyl)methyl amino]methyl]-1-piperidinyl]ethyl]methylamino]-carbonyl]-6H-pyrido[2,3-b][1,4]benzodiazepin-6-on

Hergestellt analog Beispiel 2 aus 11-(Chlorcarbonyl)-5,11- dihydro-6H-pyrido[2,3-b][1,4]benzodiazepin-6-on und trans-2-[2-[[(4-Hydroxycyclohexyl)methylamino]methyl]-1-piperidinyl] -N-methyl-ethanamin in einer Ausbeute von 24% der Theorie. Farblose, weitgehend amorphe Festsubstanz mit dem unscharfen Fp. 105-120° C. Fp. des Hydrochlorids 210-213° C (Z.) (Aceton/Isopropanol).

Beispiel 19

D,L-11-[[[2-[2-[2-(Diethylamino)ethyl]-1-piperidinyl] ethyl]methylamino]carbonyl]-5,11-dihydro-6H-pyrido [2,3-b][1,4]benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[2-(Diethylamino)ethyl]-1-piperidinyl]-N-methyl-ethanamin in einer Ausbeute von 37% der Theorie. Farblose Kristalle vom Fp. 111-113° C (nach Umkristallisieren aus Diisopropylether/Acetonitril 1/1 v/v sowie aus Acetonitril).

Beispiel 20

D,L-5,11-Dihydro-11-[[[4-[2-[(dimethylamino)methyl]-1-pi peridinyl]butyl]amino]carbonyl]-6H-pyrido[2,3-b]-[1,4]benzo diazepin-6-on

Hergestellt analog Beispiel 2 aus 11-(Chlorcarbonyl)-5,11-dihydro-6H-pyrido[2,3-b][1,4]benzodiazepin-6-on und 4-[2-[(Dimethylamino)methyl]-1-piperidinyl]butanamin in einer Ausbeute von 14% der Theorie. Farblose Kristalle vom Fp. 126-128° C (Essigsäureethylester/Diisopropylether 1/1 v/v) Löslich in Wasser.

Beispiel 21

D,L-trans-5,11-Dihydro-11-[[[2-[2-[[(4-hydroxycyclohexyl) methylamino]methyl]-1-piperidinyl]ethyl]amino]-carbonyl]-6H-pyrido[2,3-b][1,4]benzodiazepin-6-on

Hergestellt analog Beispiel 2 aus 11-(Chlorcarbonyl)-5,11-dihydro-6H-pyrido[2,3-b][1,4]benzodiazepin-6-on und trans-2-[2-[[(4-Hydroxycyclohexyl)methylamino]methyl]-1-piperidinyl] ethanamin in einer Ausbeute von 75% der Theorie. Farblose Kristalle vom Fp. 130-133° C (Acetonitril/Essigsäureethylester 1/1 v/v).

Beispiel 22

4-[[[2-[2-[(Diethylamino)methyl]-1-piperidinyl]ethyl]amino] carbonyl]-4,9-dihydro-3-methyl-10H-thieno[3,4-b]-[1,5]benzo diazepin-10-on

Hergestellt analog Beispiel 2 aus 4-(Chlorcarbonyl)-4,9-dihydro-3-methyl-10H-thieno[3,4-b][1,5]-benzodiazepin-10-on und 2-[2-[(Diethylamino)methyl]-1-piperidinyl]ethanamin in einer Ausbeute von 39% der Theorie. Farblose Kristalle vom Fp. 138-140° C (Acetonitril).

Beispiel 23

9-Chlor-11-[[[2-[2-[(diethylamino)methyl]-1-piperidinyl] ethyl]amino]carbonyl]-5,11-dihydro-6H-pyrido[2,3-b]-[1,4] benzodiazepin-6-on

Hergestellt analog Beispiel 2 aus 9-Chlor-11-(chlorcarbonyl)-5,11-dihydro-6H-pyrido[2,3-b][1,4]-benzodiazepin-6-on und 2-[2-[(Diethylamino)methyl]-1-piperidinyl]ethanamin in einer Ausbeute von 60% der Theorie. Farblose Kristalle vom Fp. 171-173° C (Acetonitril).

Beispiel 24

5,11-Dihydro-11-[[[2-[2-[2-(dimethylamino)ethyl]-1-piperi dinyl]ethyl]methylamino]carbonyl]-6H-pyrido[2,3-b]-[1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[2-(Dimethylamino)ethyl]-1-piperidinyl]-N-methyl-ethanamin in einer Ausbeute von 52% der Theorie. Farblose Kristalle vom Fp. 114-116° C (nach dem Umkristallisieren aus Diisopropylether und Acetonitril und Auskochen mit Wasser).

Beispiel 25

11-[[[2-[2-[(Diethylamino)methyl]-1-piperidinyl]ethyl] amino]carbonyl]-5,11-dihydro-8-methyl-6H-pyrido[2,3-b] [1,4]benzodiazepin-6-on

Hergestellt analog Beispiel 2 aus 11-(Chlorcarbonyl)-5,11-dihydro-8-methyl-6H-pyrido[2,3-b][1,4]-benzodiazepin-6-on und 2-[2-[(Diethylamino)methyl]-1-piperidinyl]ethanamin in einer Ausbeute von 55% der Theorie. Farblose Kristalle vom Fp. 148-150° C (Acetonitril).

Beispiel 26

D,L-5,11-Dihydro-11-[[[4-[2-[(dimethylamino)methyl]-1-piperidinyl]butyl]methylamino]carbonyl]-6H-pyrido-[2,3-b] [1,4]benzodiazepin-6-on

Hergestellt analog Beispiel 2 aus 11-(Chlorcarbonyl)-5,11-dihydro-6H-pyrido[2,3-b][1,4]benzodiazepin-6-on und 4-[2-[(Dimethylamino)methyl]-1-piperidinyl]-N-methyl-butanamin in einer Ausbeute von 73% der Theorie. Farblose, amorphe Substanz: $R_F$ 0,25 (Macherey-Nagel, Polygram® SIL G/$UV_{254}$, pre-coated plastic sheets for TLC, Fließmittel: Dichlormethan/Essigsäureethylester/Cyclohexan/Methanol/konz. Ammoniak 58/25/8/8/1 v/v)

$C_{26}H_{36}N_6O_2$ (464,61)

| | | | | | | |
|---|---|---|---|---|---|---|
| Ber.: | C | 67,21 | H | 7,81 | N | 18,09 |
| Gef.: | | 67,35 | | 7,85 | | 17,70 |

IR ($CH_2Cl_2$) N-H 3370, C=O 1665 $cm^{-1}$
UV (Ethanol): $\lambda_{max}$ 282, Schulter bei 224 nm
$^1$H-NMR-($CDCl_3/CD_3OD$); 400 MHz): $\delta$ 8,25 (1H-dd, J=5,2 und 2 Hz, α-Pyridyl-H);
8,15 (1H-dd, J=7,6 und 1 Hz, ar. H in 7-Stellung),
7,79 (1H-dd, J=7,6 und 2 Hz, γ-Pyridyl-H);
7,61-7,53 (2H-m, ar. H);
7,38-7,31 (1H-m, ar. H);
7,26 (1H-dd, J=7,6 und 5,2 Hz, ß-Pyridyl-H);
4,27 (1H-s, breit, austauschbarer H);
3,23-3,13 (2H-m, aliph. H);
2,91-2,82 (1H-m, aliph. H);
2,83-2,74 (1H-m, aliph. H);
2,59 (3H-s, $-C(=O)-N-CH_3$);
2,55-2,15 (5H-m, aliph. H);
2,23 (6H-s; $-N(CH_3)_2$);
1,90-1,25 (10H-m, aliph. H).

Beispiel 27

11-[[[2-[3-[(Diethylamino)methyl]-4-morpholinyl]ethyl] amino]carbonyl]-5,11-dihydro-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 2-[3-[(Diethylamino)methyl]-4-morpholinyl]ethanamin in einer Ausbeute von 67% der Theorie. Farblose Kristalle vom Fp. 150-151 °C (Acetonitril).

Beispiel 28

11-[[[4-[2-[(Diethylamino)methyl]-1-piperidinyl]butyl] methylamino]carbonyl]-5,11-dihydro-6H-pyrido[2,3-b]-[1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 4-[2-[(Diethylamino)methyl]-1-piperidinyl]-N-methyl-butanamin in einer Ausbeute von 44% der Theorie. Farblose, amorphe Substanz.

$C_{28}H_{40}N_6O_2$ (492,66)

| | | | | | | |
|---|---|---|---|---|---|---|
| Ber.: | C | 68,26 | H | 8,18 | N | 17,06 |
| Gef.: | | 68,20 | | 8,20 | | 16,87 |

IR ($CH_2Cl_2$): N-H 3375, C=O 1670 $cm^{-1}$
UV (Ethanol): $\lambda_{max}$ 283, Schulter bei 230 nm

$^1$H-NMR (400 MHz, $CDCl_3/CD_3OD$): $\delta$ 8,25 (1H-dd, J=5,2 und 2 Hz, $\alpha$-Pyridyl-H);
8,16 (1H-dd, J=7,6 und 1 Hz, ar H in 7-Stellung),
7,79 (1H-dd, J=7,6 und 2 Hz, $\gamma$ -Pyridyl-H);
7,61-7,53 (2H-m, ar. H);
7,37-7,31 (1H-m, ar. H);
7,25 (1H-dd, J=7,6 und 5,2 Hz, ß-Pyridyl-H);
4,15 (1H-s, breit, austauschbares H);
3,25-3,11 (2H-m, aliph. H);
2,95-2,85 (1H-m, aliph. H);
2,83-2,71 (1H-m, aliph. H);
2,58 (3H-s, -C(=O)-N-$CH_3$);
1,95-1,82 (1H-m, aliph. H);
1,75-1,23 (10H-m, aliph. H);
1,02 (6H-t, J=3,4 HZ; N-C-$CH_3$).

Beispiel 29

5,11-Dihydro-11-[[[2-[2-[(dipropylamino)methyl]-1-piperi dinyl]ethyl]amino]carbonyl]-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 2-[2-[(Dipropylamino)methyl]-1-piperidinyl]ethanamin in einer Ausbeute von 88% der Theorie. Farblose Kristalle vom Fp. 165° C (Acetonitril).

Beispiel 30

5,11-Dihydro-11-[[[2-[2-[(dipropylamino)methyl]-1-piperi dinyl]ethyl]amino]carbonyl]-6H-pyrido[2,3-b][1,4]-benzo diazepin-6-on-methansulfonat

Zu der Suspension von 56,2 g (0,1174 Mol) 5,11-Dihydro-11-[[[2-[2-[(dipropylamino)methyl]-1-piperidinyl]ethyl]amino]carbonyl]-6H-pyrido[2,3-b][1,4]benzodiazepin-6-on in einer Mischung aus 100 ml Acetonitril und 100 ml Essigsäureethylester tropfte man unter Eiskühlung und Rühren eine Lösung von 11,3 g (0,1176 Mol) Methansulfonsäure in 74 ml Acetonitril. Nach Fortnahme des Eisbads erwärmte man auf 70° C, tropfte zu der nunmehr klaren Lösung 574 ml Essigsäure, impfte die Mischung mit einem Kristall des angestrebten Salzes an, ließ auf 40° C erkalten und rührte 2 Stunden bei dieser Temperatur, erwärmte dann nochmals auf 50° C, tropfte nochmals 280 ml Essigsäureethylester langsam zu und rührte nach Entfernung des Heizbades 2 Stunden bei Raumtemperatur, ließ noch 14 Stunden absitzen und nutschte schließlich das entstandene Kristallisat ab. Nach dem Waschen mit Ethylacetat und dem Trocknen im Umlufttrockenschrank erhielt man 64,0 g (95% der Theorie) an farblosen Kristallen vom Fp. 165-167° C.
$C_{27}H_{38}N_6O_2 . CH_3SO_3H$ (574,74)

| | | | | | | | | |
|---|---|---|---|---|---|---|---|---|
| Ber.: | C | 58,52 | H | 7,37 | N | 14,62 | S | 5,58 |
| Gef.: | | 58,62 | | 6,95 | | 14,60 | | 5,73 |
| Gef.: | | 58,53 | | 7,41 | | 14.68 | | 5,69 |

Beispiel 31

5,11-Dihydro-11-[[[2-[2-[(dipropylamino)methyl]-1-piperi dinyl]ethyl]amino]carbonyl]-6H-pyrido[2,3-b][1,4]-benzo diazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 2-[2-[(Dipropylamino)methyl]-1-piperidinyl]ethanamin in einer Ausbeute von 87% der Therorie. Farblose Kristalle vom Fp. 197-199° C, schwer löslich in Wasser.

Beispiel 32

8-Chlor-11-[[[2-[2-[(diethylamino)methyl]-1-piperidinyl] ethyl]amino]carbonyl]-5,11-dihydro-6H-pyrido[2,3-b]-[1,4] benzodiazepin-6-on

Die Mischung aus 3,1 g (0,01 Mol) 8-Chlor-11-(chlorcarbonyl)-5,11-dihydro-6H-pyrido[2,3-b][1,4]-benzodiazepin-6-on, 2,6 g (0,0122 Mol) 2-[2-[(Diethylamino)methyl]-1-piperidinyl]ethanamin und 310 ml 1,2-Dichlorethan wurde 2 1/2 Stunden bei Zimmertemperatur gerührt, anschließend mit verdünnter Salzsäure erschöpfend extrahiert. Die vereinigten wässerigen Auszüge wurden mit gesättigter wässeriger Kaliumcarbonat-Lösung alkalisch gestellt und erschöpfend mit Dichlormethan ausgeschüttelt. Die Dichlormethanphasen wurden vereinigt, über Natriumsulfat unter Zusatz von 1 g Tierkohle getrocknet und im Vakuum eingedampft. Der ölige Rückstand wurde nach dem Verreiben mit Diisopropylether kristallin und wurde aus Acetonitril umkristallisiert. Man erhielt 2,9 g (60% der Theorie) aus farblosen Kristallen vom Fp. 138-140° C.

Beispiel 33

11-[[[2-[2-[(Diethylamino)methyl]-1-piperidinyl]ethyl] amino]carbonyl]-5,11-dihydro-8-ethyl-6H-pyrido[2,3-b] -[1,4]benzodiazepin-6-on

Hergestellt analog Beispiel 2 aus 11-(Chlorcarbonyl)-5,11-dihydro-8-ethyl-6H-pyrido[2,3-b][1,4]-benzodiazepin-6-on und 2-[2-[(Diethylamino)methyl]-1-piperidinyl]ethanamin in einer Ausbeute von 70% der Theorie. Farblose Kristalle vom Fp. 124-126° C (Diisopropylether).

Beispiel 34

8-Brom-11-[[[2-[2-[(diethylamino)methyl]-1-piperidinyl] ethyl]amino]carbonyl]-5,11-dihydro-6H-pyrido[2,3-b]-[1,4] benzodiazepin-6-on

Hergestellt analog Beispiel 2 aus 8-Brom-11-(chlorcarbonyl)-5,11-dihydro-6H-pyrido[2,3-b][1,4]-benzodiazepin-6-on und 2-[2-[(Diethylamino)methyl]-1-piperidinyl]ethanamin in einer Ausbeute von 25% der Theorie. Farblose Kristalle vom Fp. 117-119 °C, $R_F$ 0,3 (Macherey-Nagel, Polygram® SIL G/$UV_{254}$, pre-coated plastic sheets for TLC, Fließmittel: Dichlormethan/Essigsäureethylester/Cyclohexan/Methanol/konz. Ammoniak 58/25/8/8/1 v/v).

Beispiel 35

11-[[[2-[2-[(Diethylamino)methyl]-1-piperidinyl]ethyl] amino]carbonyl]-5,11-dihydro-9-methyl-6H-pyrido[2,3-b] [1,4]benzodiazepin-6-on

Hergestellt analog Beispiel 2 aus 11-(Chlorcarbonyl)-5,11- dihydro-9-methyl-6H-pyrido[2,3-b][1,4]-benzodiazepin-6-on und 2-[2-[(Diethylamino)methyl]-1-piperidinyl]ethanamin in einer Ausbeute von 40% der Theorie. Farblose Kristalle vom Fp. 183-185 °C (Acetonitril).

Beispiel 36

11-[[[2-[2-[4-(Diethylamino)butyl]-1-piperidinyl]ethyl] amino]carbonyl]-5,11-dihydro-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 2-[2-[4-(Diethylamino)butyl]-1-piperidinyl]ethanamin in einer Ausbeute von 55% der Theorie. Farblose Kristalle vom Fp. 155-157 °C (Acetonitril). $R_F$ 0,57 (Macherey-Nagel, Polygram® SIL G/$UV_{254}$, pre-coated plastic sheets for TLC, Fließmittel: Dichlormethan/Cyclohexan/Methanol/konz. Ammoniak 102/23/23/3 v/v).

Beispiel 37

6,11-Dihydro-11-[[[2-[2-[(dipropylamino)methyl]-1-piperi dinyl]ethyl]amino]carbonyl]-5H-pyrido[2,3-b][1,5]-benzo diazepin-5-on

Hergestellt analog Beispiel 2 aus 11-(Chlorcarbonyl)-6,11-dihydro-5H-pyrido[2,3-b][1,5]benzodiazepin-5-on und 2-[2-[(Dipropylamino)methyl]-1-piperidinyl]ethanamin in einer Ausbeute von 66% der Theorie. Farblose Kristalle vom Fp. 149-151 °C (Acetonitril).

Beispiel 38

11-[[[2-[2-[(Diethylamino)methyl]-1-piperidinyl]ethyl] amino]carbonyl]-6,11-dihydro-5H-pyrido[2,3-b][1,5]-benzo diazepin-5-on

Hergestellt analog Beispiel 2 aus 11-(Chlorcarbonyl)-6,11-dihydro-5H-pyrido[2,3-b][1,5]benzodiazepin-5-on und 2-[2-[(Diethylamino)methyl]-1-piperidinyl]ethanamin in einer Ausbeute von 52% der Theorie. Farblose Kristalle vom Fp. 146-147,5 °C (Acetonitril).

Beispiel 39

5,11-Dihydro-11-[[[2-[2-[(1-piperidinyl)methyl]-1-piperi dinyl]ethyl]amino]carbonyl]-6H-pyrido[2,3-b][1,4]-benzodi azepin-6-on-hydrochlorid

Hergestellt analog Beispiel 1 aus 11-(Chlorcarbonyl)-5,11-dihydro-6H-pyrido[2,3-b][1,4]benzodiazepin-6-on und 2-[2-[(1-Piperidinyl)methyl]-1-piperidinyl]ethanamin in einer Ausbeute von 51% der Theorie. Farblose Kristalle vom Fp. 214-216 °C (Acetonitril), löslich in Wasser.

Beispiel 40

11-[[[2-[3-(Diethylamino)-hexahydro-1H-azepin-1-yl]ethyl] amino]carbonyl]-5,11-dihydro-6H-pyrido[2,3-b]-[1,4]benzodi azepin-6-on

Hergestellt analog Beispiel 2 aus 11-(Chlorcarbonyl)-5,11-dihydro-6H-pyrido[2,3-b][1,4]benzodiazepin-6-on und 2-[3-(Diethylamino)hexahydro-1H-azepin-1-yl]ethanamin in einer Ausbeute von 38% der Theorie. Farblose Kristalle vom Fp. 164 °C (Acetonitril).

Beispiel 41

4-[[[2-[2-[(Diethylamino)methyl]-1-piperidinyl]ethyl]amino] carbonyl]-1,3-dimethyl-1,4,9,10-tetrahydropyrazolo[4,3-e] pyrido[2,3-b][1,4]diazepin-10-on

Hergestellt analog Beispiel 2 aus 4-(Chlorcarbonyl)-1,3-dimethyl-1,4,9,10-tetrahydropyrazolo[4,3-e]-pyrido[2,3-b][1,4]diazepin-10-on und 2-[2-[(Diethylamino)methyl]-1-piperidinyl]ethanamin in einer Ausbeute von 17% der Theorie. Farblose Kristalle vom Fp. 138-139 °C ° (Acetonitril).

Beispiel 42

11-[[[2-[4-[(Diethylamino)methyl]-1-piperidinyl]ethyl]amino] carbonyl]-5,11-dihydro-6H-pyrido[2,3-b][1,4]-benzodiazepin-6-on

Hergestellt analog Beispiel 2 aus 11-(Chlorcarbonyl)-5,11-dihydro-6H-pyrido[2,3-b][1,4]benzodiazepin-6-on und 2-[4-[(Diethylamino)methyl]-1-piperidinyl]ethanamin in einer Ausbeute von 78% der Theorie. Farblose Kristalle vom Fp. 171 °C (Acetonitril).

Beispiel 43

5,11-Dihydro-11-[[[2-[4-[2-(1-piperidinyl)ethyl]-1-piperi dinyl]ethyl]amino]carbonyl]-6H-pyrido[2,3-b][1,4]-benzodia zepin-6-on-hydrochlorid

Hergestellt analog Beispiel 1 aus 11-(Chlorcarbonyl)-5,11-dihydro-6H-pyrido[2,3-b][1,4]benzodiazepin-6-on und 2-[4-[2-(1-Piperidinyl)ethyl]-1-piperidinyl]ethanamin in einer Ausbeute von 50% der Theorie. Farblose Kristalle vom Fp. 223 °C (Z.) (Acetonitril).

Beispiel 44

11-[[[2-[4-[2-(Diethylamino)ethyl]-1-piperidinyl]ethyl] amino]carbonyl]-5,11-dihydro-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 2-[4-[2-(Diethylamino)ethyl]-1-piperidinyl]ethanamin in einer Ausbeute von 10% der Theorie. Farblose Kristalle vom Fp. 170-172° C (Essigsäureethylester).

Beispiel 45

11-[[[2-[4-[3-(Diethylamino)propyl]-1-piperidinyl]ethyl] amino]carbonyl]-5,11-dihydro-6H-pyrido[2,3-b][1,4]-benzodia zepin-6-on

Hergestellt analog Beispiel 2 aus 11-(Chlorcarbonyl)-5,11-dihydro-6H-pyrido[2,3-b][1,4]benzodiazepin-6-on und 2-[4-[3-(Diethylamino)propyl]-1-piperidinyl]ethanamin in einer Ausbeute von 92% der Theorie. Farblose Kristalle vom Fp. 165-166° C (Essigsäureethylester).

Beispiel 46

11-[[[2-[2-[2-(Diethylamino)ethyl]-1-pyrrolidinyl]ethyl] amino]carbonyl]-5,11-dihydro-6H-pyrido[2,3-b][1,4]-benzo diazepin-6-on

Zu der Lösung von 1,4 g (0,0051 Mol) 11-(Chlorcarbonyl]-5,11-dihydro-6H-pyrido[2,3-b][1,4]-benzodiazepin-6-on und 0,6 g (0,0059 Mol) Triethylamin in 20 ml wasserfreiem Dimethylformamid tropfte man 1,06 g (0,005 Mol) 2-[2-[2-(Diethylamino)ethyl]-1-pyrrolidinyl]ethanamin und rührte die Mischung anschließend noch 1 Stunde bei Raumtemperatur. Das Dimethylformamid wurde im Vakuum abdestilliert, der verbliebene Rückstand natronalkalisch gestellt und mit Dichlormethan extrahiert. Die vereinigten Dichlormethan-Auszüge wurden getrocknet, eingedampft und schließlich an Kieselgel (70-230 mesh) unter Verwendung von Dichlormethan/Methanol/konz. Ammoniak 70/29/1 v/v) zum Eluieren säulenchromatographisch gereinigt. Die geeigneten Eluate wurden vom Lösungsmittel befreit, der Rückstand mit Diisopropylether verrieben, die entstandenen Kristalle abschließend aus Essigsäureethylester unter Verwendung von Tierkohle umkristallisiert. Man erhielt 0,6 g (27% der Theorie) an farblosen Kristallen vom Fp. 127-128° C.

Beispiel 47

D,L-11-[[[2-[2-[(Diethylamino)methyl]-1-pyrrolidinyl]ethyl] amino]carbonyl]-5,11-dihydro-6H-pyrido[2,3-b]-[1,4]benzodi azepin-6-on

Hergestellt analog Beispiel 46 aus 11-(Chlorcarbonyl)-5,11-dihydro-6H-pyrido[2,3-b][1,4]benzodiazepin-6-on und 2-[2-[(Diethylamino)methyl]-1-pyrrolidinyl]ethanamin in einer Ausbeute von 47% der Theorie. Farblose Kristalle vom Fp. 160° C (Essigsäureethylester/Diisopropylether 1:1 v/v). Das Hydrochlorid schmolz bei 163-165° C.

Beispiel 48

D,L-5-[[[2-[2-[(Diethylamino)methyl]-1-pyrrolidinyl]ethyl] amino]carbonyl]-5,10-dihydro-11H-dibenzo[b,e][1,4]-diazepin-11-on

Hergestellt analog Beispiel 46 aus 5-(Chlorcarbonyl)-5,10-dihydro-11H-dibenzo[b,e][1,4]diazepin-11-on und 2-[2-[(Diethylamino)methyl]-1-pyrrolidinyl]ethanamin in einer Ausbeute von 23% der Theorie. Farblose Kristalle vom Fp. 112-115° C (Essigsäureethylester).

Entsprechend wurden erhalten:

D,L-11-[[[2-[3-[[(Cyclohexyl)methylamino]methyl]-1-pyrrolidinyl]ethyl]amino]carbonyl]-5,11-dihydro-6H-pyrido[2,3-b][1,4]benzodiazepin-6-on;

9-Chlor-11-[[[2-[3-[(diethylamino)methyl]-1-pyrrolidinyl]ethyl]amino]carbonyl]-5,11-dihydro-6H-pyrido[2,3-b][1,4]benzodiazepin-6-on;

4-[[[2-[3-[(Diethylamino)methyl]-1-pyrrolidinyl]ethyl]amino]carbonyl]-4,9-dihydro-1,3-dimethyl-10H-thieno[3,4-b][1,5]benzodiazepin-10-on;

Beispiel 49

4-[[[2-[2-[(Diethylamino)methyl]-1-piperidinyl]ethyl] amino]carbonyl]-1,3-dimethyl-1,4,9,10-tetrahydropyrrolo-[3,2-b][1,5]benzodiazepin-10-on

Hergestellt analog Beispiel 2 aus 4-(Chlorcarbonyl)-1,3-dimethyl-1,4,9,10-tetrahydropyrrolo[3,2-b][1,5]-benzodiazepin-10-on und 2-[2-[(Diethylamino)methyl]-1-piperidinyl]ethanamin in einer Ausbeute von 72% der Theorie.

Beispiel 50

3-Chlor-4-[[[2-[2-[(dipropylamino)methyl]-1-piperidinyl] ethyl]amino]carbonyl]-1-methyl-1,4,9,10-tetrahydro-pyrrolo [3,2-b][1,5]benzodiazepin-10-on

Hergestellt analog Beispiel 2 aus 3-Chlor-4-(chlorcarbonyl)-1-methyl-1,4,9,10-tetrahydropyrrolo[3,2-b]-[1,5]benzodiazepin-10-on und 2-[2-[(Dipropylamino)methyl]-1-piperidinyl]ethanamin in einer Ausbeute von 57% der Theorie.

Beispiel 51

4-[[[2-[2-[(Dipropylamino)methyl]-1-piperidinyl]ethyl] amino]carbonyl]-1-methyl-1,4,9,10-tetrahydropyrrolo-[3,2-b][1,5]benzodiazepin-10-on

4,2 g (8,15 mMol) 3-Chlor-4-[[[2-[2-[(dipropylamino)methyl]-1-piperidinyl]ethyl]amino]carbonyl]-1-methyl-1,4,9,10-tetrahydropyrrolo[3,2-b[[1,5]benzodiazepin-10-on wurden in 350 ml heißem Ethanol gelöst und nach zusatz von 3 g Palladium auf Tierkohle (20proz.) 20 Stunden bei einem Wasserstoffdruck von 50 bar und einer Temperatur von 40 °C hydriert. Man filtrierte vom Katalysator ab, engte das Filtrat im Vakuum ein, nahm den Rückstand in 20 ml Wasser auf, stellte die erhaltene Lösung natronalkalisch und extrahierte erschöpfend mit Dichlormethan. Die vereinigten Extrakte wurden über Natriumsulfat getrocknet und eingedampft und der verbleibende Rückstand einmal aus Ethylacetat und einmal aus Acetonitril umkristallisiert. Man erhielt 2,1 g (54% der Theorie) an farblosen Kristallen vom Fp. 153-155 °C.

Beispiel 52

4-[[[2-[3-[(Dipropylamino)methyl]-1-piperidinyl]ethyl] amino]carbonyl]-1-methyl-1,4,9,10-tetrahydropyrrolo-[3,2-b][1,5]benzodiazepin-10-on

5,02 g (9,7 mMol) 3-Chlor-4-[[[2-[2-[(dipropylamino)methyl]-1-piperidinyl]ethyl]amino]carbonyl]-1-methyl-1,4,9,10-tetrahydropyrrolo[3,2-b][1,5]benzodiazepin-10-on wurden in einem Gemisch aus 5 ml 85proz. Ameisensäure und 25 ml Dimethylformamid gelöst und nach Zusatz von 0,5 g 10proz. Palladium/Aktivkohle 3 Stunden unter Rückfluß gekocht. Man gab 7,0 ml Ameisensäure zu, kochte weitere 6 Stunden unter Rückfluß und erhitzte nach Versetzen mit weiteren 4,0 ml Ameisensäure und 0,8 g 10proz.

Palladium/Aktivkohle abschließend nochmals 8 Stunden unter Rückfluß. Die Mischung wurde heiß filtriert, das Filtrat im Vakuum eingedampft und der Rückstand säulenchromatographisch (Kieselgel; Dichlormethan/Essigsäureethylester/Methanol/konz. Ammoniak 3,5/1,5/0,46/0,06 v/v) gereinigt. Man erhielt 1,8 g (39% der Theorie) an farblosen Kristallen vom Fp. 153-155 °C (Acetonitril), nach Dünnschichtchromatogramm und IR-, UV- und $^1$H-NMR-Spektren identisch mit einem nach Beispiel 51 erhaltenen Präparat.

Beispiel 53

4-[[[2-[2-[(Dipropylamino)methyl]-1-piperidinyl]ethyl] amino]carbonyl]-1-methyl-1,4,9,10-tetrahydropyrrolo-[3,2-b][1,5]benzodiazepin-10-on

Die Mischung aus 5,15 g (0,01 Mol) 3-Chlor-4-[[[2-[(dipropylamino)methyl]-1-piperidinyl]ethyl]amino]-carbonyl]-1-methyl-1,4,9,10-tetrahydropyrrolo[3,2-b][1,5]benzodiazepin-10-on, 83,3 mg (0,001 Mol) 2:1-Tris-(o-tolyl)-phosphin-Palladiumacetat-Katalysator, 2,025 g (0,044 Mol) Ameisensäure und 5,77 g (0,057 Mol) Triethylamin in 200 ml Tetrahydrofuran wurde unter Stickstoff-Atmosphäre im Autoklaven 40 Stunden auf 100 °C erhitzt. Die Mischung wurde filtriert und im Vakuum eingedampft, der Rückstand natronalkalisch gestellt und erschöpfend mit Dichlormethan extrahiert. Die getrockneten und eingedampften organischen Phasen wurden wie in Beispiel 52 säulenchromatographisch gereinigt. Man erhielt 1,75 g (36% der Theorie) an farblosen Kristallen vom Fp. 154-155 °C (Acetonitril), nach Dünnschichtchromatogramm und IR-Spektrum identisch mit einer nach Beipiel 51 erhaltenen Probe.

Beispiel 54

11-[[[2-[2-[(Diethylamino)methyl]-1-piperidinyl]ethyl] methylamino]carbonyl]-5,11-dihydro-6H-pyrido[2,3-b]-[1,4] benzodiazepin-6-on

In eine Mischung, bestehend aus 22,5 ml einer 20proz. Lösung von Phosgen in Toluol, 100 ml Dioxan und 4,75 g (0,045 Mol) wasserfreiem Natriumcarbonat wurden unter äußerer Kühlung mit Eis 9,66 g (0,0425 Mol) 2-[2-[(Diethylamino)methyl]-1-piperidinyl]-N-methyl-ethanamin zugetropft. Man rührte noch 60 Minuten bei Zimmertemperatur, trug dann 9,0 g (0,0428 Mol) 5,11-Dihydro-6H-pyrido[2,3-b][1,4]benzodiazepin-6-on in die Reaktionsmischung ein und kochte anschließend 4 Stunden unter Rückfluß. Man filtrierte, dampfte das Filtrat im Vakuum ein und reinigte das erhaltene Produkt säulenchromatographisch an 500 g Kieselgel unter Verwendung von Essigsäureethylester/Methanol (Volumenverhältnis 10:2) zum Eluieren. Nach dem Umkristallisieren aus Cyclohexan schmolzen die farblosen Kristalle bei 269-270 °C und waren nach Mischschmelzpunkt, Dünnschichtchromatogramm und IR-Spektrum identisch mit einem nach Beispiel 15 hergestellten Präparat. Ausbeute: 6,0 g (30% der Theorie).

Entsprechend erhielt man:

5,11-Dihydro-11-[[[2-[2-[(dimethylamino)methyl]-1-piperidinyl]ethyl]amino]carbonyl]-6H-pyrido[2,3-b]-[1,4]benzodiazepin-6-on, farblose Kristalle vom Fp. 64-67 °C (Petrolether);

trans-5,11-Dihydro-11-[[[2-[2-[[(4-hydroxycyclohexyl]methylamino]methyl]-1-piperidinyl]ethyl]-methylamino]carbonyl]-6H-pyrido[2,3-b][1,4]benzodiazepin-6-on, das nach dem Überführen ins Hydrochlorid bei 211-213 °C (Z.) schmolz (aus Aceton/Isopropanol 1/1 v/v);

11-[[[2-[2-[2-(Diethylamino)ethyl]-1-piperidinyl]ethyl]methylamino]carbonyl]-5,11-dihydro-6H-pyrido[2,3-b][1,4]benzodiazepin-6-on, farblose Kristalle vom Fp. 111-113 °C (Acetonitril);

5,11-Dihydro-11-[[[2-[2-(dimethylamino)ethyl]-1-piperidinyl]ethyl]methylamino]carbonyl]-6H-pyrido[2,3-b][1,4]benzodiazepin-6-on, farblose Kristalle vom Fp. 114-116 °C (Diisopropylether);

5,11-Dihydro-11-[[[4-[2-[(dimethylamino)methyl]-1-piperidinyl]butyl]methylamino]carbonyl]-6H-pyrido-[2,3-b][1,4]benzodiazepin-6-on, nach DC, IR und $^1$H-NMR identisch mit einem nach Beispiel 26 hergestellten Präparat.

Beispiel 55

5,11-Dihydro-11-[[[2-[3-[(1-piperidinyl)methyl]-1-piperi dinyl]ethyl]amino]carbonyl]-6H-pyrido[2,3-b][1,4]-benzo diazepin-6-on

Hergestellt analog Beispiel 46 aus 11-(Chlorcarbonyl)-5,11-dihydro-6H-pyrido[2,3-b][1,4]benzodiazepin-6-on und 2-[3-[(1-Piperidinyl)methyl]-1-piperidinyl]ethanamin in einer Ausbeute von 38% der Theorie. Farblose, amorphe Substanz, $R_F$ 0,16 (Macherey-Nagel, Polygram® SIL G/$UV_{254}$, pre-coated plastic sheets for TLC, Fließmittel: Dichlormethan/Methanol//konz. Ammoniak 90/10/1 v/v).
$C_{26}H_{34}N_6O_2$ . 2 HCl (535,54)

| | | | | | | | | |
|---|---|---|---|---|---|---|---|---|
| Ber.: | C | 58,31 | H | 6,78 | Cl | 13,24 | N | 15,69 |
| Gef.: | | 58,14 | | 6,98 | | 13,10 | | 15,51 |

IR (KBr): N-H 3420 $cm^{-1}$ (breit), C=O 1680 $cm^{-1}$
UV (Ethanol): $\lambda_{max}$ 278, Schulter bei 230 nm
$^1$H-NMR-(400 MHz; DMSO-$d_6$/$CD_3OD$): $\delta$ 8,42-8,35 (1H-m; α-Pyridyl-H);

7,99-7,78 (2H-m; ar. H);
7,71-7,63 (1H-m; ar. H);
7,62-7,43 (3H-m, ar. H);
4,60-4,30 (2H, breit; austauschbarer H);
4,03-3,90 (1H-m; aliph. H);
3,65-3,35 (6H-m; aliph. H);
3,30-3,12 (1H-m, aliph. H);
3,09-2,70 (6H-m, aliph. H);

2,65-2,42 (1H-m, aliph. H);
2,02-1,65 (8H-m; aliph. H);
1,50-1,20 (2H-m; aliph. H).

Beispiel 56

5,11-Dihydro-11-[[[2-[3-[3-(1-piperidinyl)propyl]-1-piperi dinyl]ethyl]amino]carbonyl]-6H-pyrido[2,3-b][1,4]-benzo diazepin-6-on

Hergestellt analog Beispiel 46 aus 11-(Chlorcarbonyl)-5,11-dihydro-6H-pyrido[2,3-b][1,4]benzodiazepin-6-on und 2-[3-[3-(1-Piperidinyl)propyl]-1-piperidinyl]ethanamin in einer Ausbeute von 14% der Theorie. Farblose, amorphe Substanz, $R_F$ 0,13 (Macherey-Nagel, Polygram® SIL G/$UV_{254}$, pre-coated plastic sheets for TLC, Fließmittel: Dichlormethan/Methanol//konz. Ammoniak 90/10/1 v/v).
$C_{26}H_{38}N_6O_2$ (490,66)

| | | | | | | |
|---|---|---|---|---|---|---|
| Ber.: | C | 68,54 | H | 7,81 | N | 17,13 |
| Gef.: | | 68,30 | | 7,96 | | 17,05 |

IR (KBr): N-H 3400 $cm^1$ (breit), C=O 1675 $cm^{-1}$

UV (Ethanol): $\lambda_{max}$ 282, Schulter bei 230 nm

$^1$H-NMR-($CDCl_3$/$CD_3OD$; 400 MHz); $\delta$ 8,32 (1H-dd, J=4,4 Hz und 2 Hz, α-Pyridyl-H);

7,91 (1H-dd, J=8,0 Hz und 1,6 Hz; ar. H in 7-Stellung);
7,67-7,59 (2H-m; ar. H);
7,52 (1H-dd, J=9 und 1 Hz; ar. H);
7,48-7,41 (1H-m; ar. H);
7,37-7,32 (1H-dd; J=8,0 Hz und 4,4 Hz, ß-Pyridyl-H);
4,25 (2H-s, austauschbarer H);
2,83-2,68 (1H-m, aliph. H);
2,55-2,35 (5H-m, aliph. H);
2,35-2,24 (3H-m, aliph. H);

1,98-1,87 (1H-m; aliph. H);
1,87-1,22 (16H-m; aliph. H).
1,22-1,05 (2H-M; aliph. H);
0,93-0,77 (1H-m; aliph. H).

Beispiel 57

11-[[[2-[3-[(Diethylamino)methyl]-1-piperidinyl]ethyl] amino]carbonyl]-5,11-dihydro-6H-pyrido[2,3-b][1,4]-benzo diazepin-6-on

Hergestellt analog Beispiel 46 aus 11-(Chlorcarbonyl)-5,11-dihydro-6H-pyrido[2,3-b][1,4]benzodiazepin-6-on und 2-[3-[(Diethylamino)methyl]-1-piperidinyl]ethanamin in einer Ausbeute von 34% der Theorie. Farblose Kristalle vom Fp. 139-141 °C (Acetonitril), $R_F$ 0,11 (Macherey-Nagel, Polygram® SIL G/$UV_{254}$, pre-coated plastic sheets for TLC, Fließmittel: Dichlormethan/Essigsäureethylester/Cyclohexan/Methanol//konz. Ammoniak 58/25/8/8/1 v/v).

Entsprechend erhielt man:

Aus 4-(Chlorcarbonyl)-1,3-dimethyl-1,4,9,10-tetrahydropyrazolo[4,3-b][1,5]benzodiazepin-10-on und 2-[2-[(Diethylamino)methyl]-1-piperidinyl]ethanamin das 4-[[[2-[2-[(Diethylamino)methyl]-1-piperidinyl]ethyl]-amino]carbonyl-1,3-dimethyl-1,4,9,10-tetrahydropyrazolo[4,3-b][1,5]benzodiazepin-10-on;

aus 4-(Chlorcarbonyl)-1-methyl-1,4,9,10-tetrahydropyrazolo[4,3-b][1,5]benzodiazepin-10-on und 2-[2-[(Diethylamino)methyl]-1-piperidinyl]ethanamin das 4-[[[2-[2-[(Diethylamino)methyl]-1-piperidinyl]ethyl]-amino]carbonyl]-1-methyl-1,4,9,10-tetrahydropyrazolo[4,3-b][1,5]benzodiazepin-10-on.

Beispiel 58

5,11-Dihydro-11-[[[2-[3-[(diproylamino)methyl]-1-piperi dinyl]ethyl]amino]carbonyl]-6H-pyrido[2,3-b][1,4]-benzodi azepin-6-on

Hergestellt analog Beispiel 46 aus 11-(Chlorcarbonyl)-5,11-dihydro-6H-pyrido[2,3-b][1,4]benzodiazepin-6-on und 2-[3-[(Dipropylamino)methyl]-1-piperidinyl]ethanamin in einer Ausbeute von 48% der Theorie. Farblose Kristalle vom Fp. 153-155 °C (Acetonitril).

Beispiel 59

5,11-Dihydro-11-[[[2-[3-[(hexahydro-1H-azepin-1-yl)methyl]-1-piperidinyl]ethyl]amino]carbonyl]-6H-pyrido-[2,3-b][1,4] benzodiazepin-6-on

Hergestellt analog Beispiel 46 aus 11-(Chlorcarbonyl)-5,11-dihydro-6H-pyrido[2,3-b][1,4]benzodiazepin-6-on und 2-[3-[(Hexahydro-1H-azepin-1-yl)methyl]-1-piperidinyl]ethanamin in einer Ausbeute von 69% der Theorie. Farblose Kristalle vom Fp. 135-137 °C (Acetonitril).

Beispiel 60

9-Chlor-11-[[[2-[3-[(diethylamino)methyl]-1-piperidinyl] ethyl]amino]carbonyl]-5,11-dihydro-6H-pyrido[2,3-b]-[1,4] benzodiazepin-6-on

Hergestellt analog Beispiel 46 aus 9-Chlor-11-(chlorcarbonyl)-5,11-dihydro-6H-pyrido[2,3-b][1,4]-benzodiazepin-6-on und 2-[3-[(Diethylamino)methyl]-1-piperidinyl]ethanamin in einer Ausbeute von 73% der Theorie. Farblose Kristalle vom Fp. 125 °C (Z.) (Acetonitril).

Beispiel 61

11-[[[2-[3-[(Diethylamino)methyl]-1-pyrrolidinyl]ethyl] amino]carbonyl]-5,11-dihydro-6H-pyrido[2,3-b][1,4]-benzo diazepin-6-on

Hergestellt analog Beispiel 2 aus 11-(Chlorcarbonyl)-5,11-dihydro-6H-pyrido[2,3-b][1,4]benzodiazepin-6-on und 2-[3-[(Diethylamino)methyl]-1-pyrrolidinyl]ethanamin in einer Ausbeute von 32% der Theorie. Farblose Kristalle vom Fp. 184-185 °C (Acetonitril).

Beispiel 62

5,11-Dihydro-11-[[[2-[3-[(1-piperidinyl)methyl]-1-pyrroli dinyl]ethyl]amino]carbonyl]-6H-pyrido[2,3-b][1,4]-benzodi azepin-6-on

Hergestellt analog Beispiel 2 aus 11-(Chlorcarbonyl)-5,11-dihydro-6H-pyrido[2,3-b][1,4]benzodiazepin-6-on und 2-[3-[(1-Piperidinyl)methyl]-1-pyrrolidinyl]ethanamin in einer Ausbeute von 39% der Theorie. Farblose Kristalle vom Fp. 168-169 °C (Acetonitril).

Beispiel 63

5,11-Dihydro-11-[[[2-[3-[(dipropylamino)methyl]-1-pyrroli dinyl]ethyl]amino]carbonyl]-6H-pyrido[2,3-b][1,4]-benzodi azepin-6-on

Hergestellt analog Beispiel 2 aus 11-(Chlorcarbonyl)-5,11- dihydro-6H-pyrido[2,3-b][1,4]benzodiazepin-6-on und 2-[3-[(Dipropylamino)methyl]-1-pyrrolidinyl]ethanamin in einer Ausbeute von 22% der Theorie. Farblose Kristalle vom Fp. 134-138° C (Acetonitril).

Beispiel 64

9-Chlor-11-[[[2-[3-[(diethylamino)propyl]-1-piperidinyl] ethyl]amino]carbonyl]-5,11-dihydro-6H-pyrido[2,3-b]-[1,4] benzodiazepin-6-on

Hergestellt analog Beispiel 2 aus 9-Chlor-11-(chlorcarbonyl)-5,11-dihydro-6H-pyrido[2,3-b][1,4]-benzodiazepin-6-on und 2-[4-[3-(Diethylamino)propyl]-1-piperidinyl]ethanamin in einer Ausbeute von 41% der Theorie. Farblose Kristalle vom Fp. 168-170° C (Essigsäureethylester).

Beispiel 65

(+)-9-Chlor-11-[[[2-[2-[(diethylamino)methyl]-1-piperidinyl] ethyl]amino]carbonyl]-5,11-dihydro 6H-pyrido-[2,3-b][1,4] benzodiazepin-6-on

Hergestellt analog Beispiel 2 aus 9-Chlor-11-(chlorcarbonyl)-5,11-dihydro-6H-pyrido[2,3-b][1,4]-benzodiazepin-6-on und (+)-2-[2-[(Diethylamino)methyl]-1-piperidinyl]ethanamin in einer Ausbeute von 50% der Theorie. Farblose Kristalle vom Fp. 169-170° C (aus Acetonitril unter Verwendung von Aktivkohle); $[\alpha]_D^{20}$ = + 11,88° (verdünnte Salzsäure).

Beispiel 66

(-)-9-Chlor-11-[[[2-[2-[(diethylamino)methyl]-1-piperidinyl] ethyl]amino]carbonyl]-5,11-dihydro-6H-pyrido[2,3-b][1,4] benzodiazepin-6-on

Hergestellt analog Beispiel 2 aus 9-Chlor-11-(chlorcarbonyl)-5,11-dihydro-6H-pyrido[2,3-b][1,4]-benzodiazepin-6-on und (-)-2-[2-[(Diethylamino)methyl]-1-piperidinyl]ethanamin in einer Ausbeute von 33% der Theorie. Farblose Kristalle vom Fp. 169-170° C (Acetonitril); $[\alpha]_D^{20}$ = - 11,92° (verdünnte Salzsäure).

Beispiel 67

5,11-Dihydro-11-[[[2-[2-[(1-pyrrolidinyl)methyl]-1-piperi dinyl]ethyl]amino]carbonyl]-6H-pyrido[2,3-b][1,4]-benzodi azepin-6-on

Hergestellt analog Beispiel 2 aus 11-Chlorcarbonyl)-5,11-dihydro-6H-pyrido[2,3-b][1,4]benzodiazepin-6-on und 2-[2-[(1-Pyrrolidinyl)methyl]-1-piperidinyl]ethanamin in einer Ausbeute von 44% der Theorie. Farblose Kristalle vom Fp. 198° C (aus Acetonitril unter Verwendung von Bleicherde).

Beispiel 68

11-[[[2-[4-[3-(Diethylamino)propyl]-1-piperidinyl]ethyl] amino]carbonyl]-5,11-dihydro-9-methyl-6H-pyrido[2,3-b][1,4] benzodiazepin-6-on

Hergestellt analog Beispiel 2 aus 11-(Chlorcarbonyl)-5,11-dihydro-9-methyl-6H-pyrido[2,3-b][1,4]-benzodiazepin-6-on und 2-[4-[3-(Diethylamino)propyl]-1-piperidinyl]ethanamin in einer Ausbeute von 39%

der Theorie. Farblose Kristalle vom Fp. 172-173 °C (Acetonitril).

Beispiel 69

11-[[[2-[3-[[(Cyclohexyl)methylamino]methyl]-1-pyrrolidinyl] ethyl]amino]carbonyl]-5,11-dihydro-6H-pyrido-[2,3-b][1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 2-[3-[[(Cyclohexyl)methylamino]methyl]-1-pyrrolidinyl]ethanamin in einer Ausbeute von 19 % der Theorie. Farblose Kristalle vom Fp. 166-167 °C (Essigsäureethylester/Methanol 1/1 v/v).

Beispiel 70

9-Chlor-11-[[[2-[4-[3-(diethylamino)propyl]-1-piperidinyl] ethyl]amino]carbonyl]-5,11-dihydro-6H-pyrido[2,3-b][1,4] benzodiazepin 6-on

Hergestellt analog Beispiel 46 aus 9-Chlor-11-(chlorcarbonyl)-5,11-dihydro-6H-pyrido[2,3-b][1,4]-benzodiazepin-6-on und 2-[4-[3-(Diethylamino)propyl]-1-piperidinyl]ethanamin in einer Ausbeute von 41 % der Theorie. Farblose Kristalle vom Fp. 168-170 °C.

Beispiel 71

5,11-Dihydro-11-[[[2-[4-[4-(1-piperidinyl)butyl]-1-piperi dinyl]ethyl]amino]carbonyl]-6H-pyrido[2,3-b][1,4]-benzodi azepin 6-on-dihydrochlorid

Hergestellt analog Beispiel 46 aus 11-(Chlorcarbonyl)-5,11-dihydro-6H-pyrido[2,3-b][1,4]benzodiazepin-6-on und 2-[4-[4-(1-Piperidinyl)butyl]-1-piperidinyl]ethanamin in einer Ausbeute von 40 % der Theorie. Das farblose Dihydrochlorid schmolz bei 231-233 °C.

Beispiel 72

5,11-Dihydro-11-[[[2-[4-[3-(dipropylamino)propyl]-1-piperi dinyl]ethyl]amino]carbonyl]-9-methyl-6H-pyrido-[2,3-b][1,4] benzodiazepin-6-on-trihydrochlorid-dihydrat

Hergestellt analog Beispiel 1 aus 11-(Chlorcarbonyl)-5,11-dihydro-9-methyl-6H-pyrido[2,3-b][1,4]-benzodiazepin-6-on und 2-[4-[3-(dipropylamino)propyl]-1-piperidinyl]ethanamin in einer Ausbeute von 63 % der Theorie. Farblose Kristalle vom Fp. 195 °C (Z.) $R_F = 0,5$ (Macherey-Nagel, Polygram® SIL G $UV_{254}$, pre-coated plastic sheets for TLC, Fließmittel: Dichlormethan/Cyclohexan/Methanol/konz. Ammoniak 68/15/15/2 v/v/v/v).

Beispiel 73

11-[[[2-[4-[3-[Bis(methylethyl)amino]propyl]-1-piperidinyl] ethyl]amino]carbonyl]-5,11-dihydro-9-methyl-6H-pyrido[2,3-b] [1,4]benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 2-[4-[3-[Bis(methylethyl)amino]propyl]-1-piperidinyl]ethanamin in einer Ausbeute von 46 % der Theorie. Farblose Kristalle vom Fp. 160-162 °C (Essigsäureethylester).

Beispiel 74

5,11-Dihydro-11-[[[2-[4-[3-(1-piperidinyl)propyl]-1-piperi dinyl]ethyl]amino]carbonyl]-6H-pyrido[2,3-b][1,4]-benzodi azepin-6-on

Hergestellt analog Beispiel 1 aus 11-(Chlorcarbonyl)-5,11-dihydro-6H-pyrido[2,3-b][1,4]benzodiazepin-6-on und 2-[4-[3-(1-Piperidinyl)propyl]-1-piperidinyl]ethanamin in einer Ausbeute von 16 % der Theorie. Farblose Kristalle vom Fp. 192-193° C (Acetonitril).

Beispiel 75

5,11-Dihydro-9-methyl-11-[[[2-[4-[3-(1-piperidinyl)propyl]-1-piperidinyl]ethyl]amino]carbonyl]-6H-pyrido[2,3-b][1,4] benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 2-[4-[3-(1-Piperidinyl)propyl]-1-piperidinyl]ethanamin in einer Ausbeute von 20 % der Theorie. Farblose Kristalle vom Fp. 174-175° C (Acetonitril).

Beispiel 76

5,11-Dihydro-11-[[[2-[4-[3-(dipropylamino)propyl]-1-piperi dinyl]ethyl]amino]carbonyl]-6H-pyrido[2,3-b][1,4]-benzodi azepin-6-on

Hergestellt analog Beispiel 1 aus 11-(Chlorcarbonyl)-5,11-dihydro-6H-pyrido[2,3-b][1,4]benzodiazepin-6-on und 2-[4-[3-(Dipropylamino)propyl]-1-piperidinyl]ethanamin in einer Ausbeute von 43 % der Theorie. Farblose Kristalle vom Fp. 169-170° C (Essigsäureethylester).

Beispiel 77

11-[[[2-[4-[3-[Bis(methylethyl)amino]propyl]-1-piperi dinyl]ethyl]amino]carbonyl]-5,11-dihydro-6H-pyrido [2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-[4-[3-[Bis(methylethyl)amino]propyl]-1-piperidinyl]ethanamin in einer Ausbeute von 28 % der Theorie. Farblose Kristalle vom Fp. 184° C (Essigsäureethylester).

Beispiel 78

9-Chlor-5,11-dihydro-11-[[[2-[4-[3-(dipropylamino)propyl]-1-piperidinyl]ethyl]amino]carbonyl]-6H-pyrido[2,3-b][1,4] benzodiazepin-6-on

Hergestellt analog Beispiel 1 aus 9-Chlor-11-(chlorcarbonyl)- 5,11-dihydro-6H-pyrido[2,3-b][1,4]-benzodiazepin-6-on und 2-[4-[3-(Dipropylamino)propyl]-1-piperidinyl]ethanamin in einer Ausbeute von 22 % der Theorie. Farblose Kristalle vom Fp. 174-175° C (Acetonitril).

Beispiel 79

9-Chlor-5,11-dihydro-11-[[[2-[4-[3-(1-piperidinyl)propyl]-1-piperidinyl]ethyl]amino]carbonyl]-6H-pyrido[2,3-b]-[1,4] benzodiazepin-6-on

Hergestellt analog Beispiel 1 aus 9-Chlor-11-(chlorcarbonyl)-5,11-dihydro-6H-pyrido[2,3-b][1,4]-benzodiazepin-6-on und 2-[4-[3-(1-Piperidinyl)propyl]-1-piperidinyl]ethanamin in einer Ausbeute von 10 % der Theorie. Farblose Kristalle vom Fp. 176-177 °C (Acetonitril).

Beispiel 80

11-[[[2-[4-[3-[Bis(methylethyl)amino]propyl]-1-piperidinyl] ethyl]amino]carbonyl]-9-chlor-5,11-dihydro-6H-pyrido[2,3-b] [1,4]benzodiazepin-6-on

Hergestellt analog Beispiel 1 aus 9-Chlor-11-(chlorcarbonyl)-5,11-dihydro-6H-pyrido[2,3-b][1,4]-benzodiazepin-6-on und 2-[4-[3-[Bis(methylethyl)amino]propyl]-1-piperidinyl]ethanamin in einer Ausbeute von 44 % der Theorie. Farblose Kristalle vom Fp. 175-176 °C (Acetonitril).

Beispiel 81

9-Chlor-5,11-dihydro-11-[[[2-[4-[(4-(1-piperidinyl)butyl]-1-piperidinyl]ethyl]amino]carbonyl]-6H-pyrido[2,3-b]-[1,4] benzodiazepin-6-on-hydrochlorid

Hergestellt analog Beispiel 46 aus 9-Chlor-11-(chlorcarbonyl)-5,11-dihydro-6H-pyrido[2,3-b][1,4]-benzodiazepin-6-on und 2-[4-[4-(1-Piperidinyl)butyl]-1-piperidinyl]ethanamin in einer Ausbeute von 68 % der Theorie. Farblose Kristalle vom Fp. 173-175 °C (Essigsäureethylester).

Beispiel 82

4-[[[2-[4-[3-(Dipropylamino)propyl]-1-piperidinyl]ethyl] amino]carbonyl]-4,9-dihydro-3-methyl-10H-thieno[3,4-b][1,5] benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 2-[4-[3-(Dipropylamino)propyl]-1-piperidinyl]ethanamin in einer Ausbeute von 42 % der Theorie. Farblose Kristalle vom Fp. 114-115 °C (Diisopropylether).

Beispiel 83

4-[[[2-[4-[3-(Diethylamino)propyl]-1-piperidinyl]ethyl] amino]carbonyl]-4,9-dihydro-3-methyl-10H-thieno[3,4-b][1,5] benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 2-[4-[(3-(Diethylamino)propyl]-1-piperidinyl]ethanamin in einer Ausbeute von 52 % der Theorie. Farblose Kristalle vom Fp. 131-133 °C (Acetonitril).

Beispiel 84

4,9-Dihydro-3-methyl-4-[[[2-[4-[3-(1-piperidinyl)propyl]-1-piperidinyl]ethyl]amino]carbonyl]-10H-thieno[3,4-b]-[1,5] benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 2-[4-[3-(1-Piperidinyl)propyl]-1-piperidinyl]ethanamin in einer Ausbeute von 9 % der Theorie. Farblose Kristalle vom Fp. 170-171 ° C.

Beispiel 85

4,9-Dihydro-4-[[[2-[4-[3-(dimethylamino)propyl]-1-piperi dinyl]ethyl]amino]carbonyl]-3-methyl-10H-thieno[3,4-b][1,5] benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 2-[4-[3-(Dimethylamino)propyl]-1-piperidinyl]ethanamin in einer Ausbeute von 17 % der Thorie. Farblose Kristalle vom Fp. > 300 ° C (t-Butylmethylether).

Beispiel 86

4,9-Dihydro-3-methyl-4-[[[2-[4-[4-(1-piperidinyl)butyl]-1-piperidinyl]ethyl]amino]carbonyl]-10H-thieno[3,4-b]-[1,5] benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 2-[4-[4-(1-Piperidinyl)butyl]-1-piperidinyl]ethanamin in einer Ausbeute von 20 % der Theorie. Farblose Kristalle vom Fp. 186-187 ° C (Acetonitril).

Beispiel 87

5,11-Dihydro-11-[[[2-[3-[2-(1-piperidinyl)ethyl]-1-piperi dinyl]ethyl]amino]carbonyl]-6H-pyrido[2,3-b][1,4]-benzodi azepin-6-on-dihydrochlorid

Hergestellt analog Beispiel 46 aus 11-(Chlorcarbonyl)-5,11-dihydro-6H-pyrido[2,3-b][1,4]benzodiazepin-6-on und 2-[3-[2-(1-Piperidinyl)ethyl]-1-piperidinyl]ethanamin in einer Ausbeute von 43 % der Theorie. Das Dihydrochlorid schmolz bei 204-208 ° C.

Beispiel 88

5,11-Dihydro-11-[[[2-[3-[4-(dimethylamino)butyl]-1-piperi dinyl]ethyl]amino]carbonyl]-6H-pyrido[2,3-b][1,4]-benzodi azepin-6-on

Hergestellt analog Beispiel 46 aus 11-(Chlorcarbonyl)-5,11-dihydro-6H-pyrido[2,3-b][1,4]benzodiazepin-6-on und 2-[3- [4-(Dimethylamino)butyl]-1-piperidinyl]ethanamin in einer Ausbeute von 53 % der Theorie. Farblose Kristalle vom Fp. 119-120 ° C.

Beispiel 89

5,11-Dihydro-11-[[[2-[3-[4-(1-pyrrolidinyl)butyl]-1-piperi dinyl]ethyl]amino]carbonyl]-6H-pyrido[2,3-b][1,4]-benzodi azepin-6-on-dihydrochlorid

Hergestellt analog Beispiel 46 aus 11-(Chlorcarbonyl)-5,11-dihydro-6H-pyrido[2,3-b][1,4]benzodiazepin-6-on und 2-[3-[4-(1-Pyrrolidinyl)butyl]-1-piperidinyl]ethanamin in einer Ausbeute von 35 % der Theorie. Das farblose Dihydrochlorid schmolz bei 170-172 ° C; $R_F$ 0,69 (HPTLC-Fertigplatten Kieselgel 60 $F_{254}$ für die

Nano-DC, Firma Merck; Fließmittel: Dichlormethan/Ethanol/konz. Ammoniak 65/30/5 v/v/v).

Beispiel 90

11-[[[2-[3-[4-(Diethylamino)butyl]-1-piperidinyl]ethyl] amino]carbonyl]-5,11-dihydro-6H-pyrido[2,3-b][1,4]-benzodi azepin-6-on-dihydrochlorid

Hergestellt analog Beispiel 46 aus 11-(Chlorcarbonyl)-5,11-dihydro-6H-pyrido[2,3-b][1,4]benzodiazepin-6-on und 2-[3-[4-(Diethylamino)butyl]-1-piperidinyl]ethanamin in einer Ausbeute von 41 % der Theorie.

Das Dihydrochlorid, das nur in Form eines partiell kristallinen Schaum erhalten werden konnte, schmolz bei etwa 130 °C; $R_F$ 0,18 (Merck-DC-Fertigplatten Kieselgel 60 $F_{254}$; Fließmittel: Dichlormethan/Methanol/konz. Ammoniak 90/10/1 v/v/v).

IR ($CH_2Cl_2$): C=O 1670 $cm^{-1}$

UV (Ethanol): $\lambda_{max}$ 280, Schulter bei 240 mm

$^1H$-NMR (200 MHz, $CDCl_3/CD_3OD$):

$\delta$ 8,39 (1H-m, α-Pyridyl-H); 7,40-6,93 (6H-m, ar. H); 4,30 (4H-s, austauschbarer H); 3,75-3,55 (4H-m; aliph. H); 3,35-2,95 (8H-m; aliph. H); 2,95-2,40 (2H-m; aliph. H); 2,15-1,65 (7H-m; aliph. H); 1,60-1,25 (10H-m; aliph. H).

Beispiel 91

5,11-Dihydro-11-[[[2-[2-[(dipropylamino)methyl]-1-piperi dinyl]ethyl]amino]carbonyl]-6H-pyrido[2,3-b][1,4]-benzo diazepin-6-on-dihydrochlorid-hydrat

0,479 g (0,001 mol) 5,11-Dihydro-11-[[[2-[2-[(dipropylamino)methyl]-1-piperidinyl]ethyl]amino]carbonyl]-6H-pyrido[2,3-b][1,4]benzodiazepin-6-on wurden in 10 ml wasserfreiem Ethanol gelöst und mit einer Lösung von 0,002 mol Chlorwasserstoff in Isopropanol (0,43 ml einer 0,17 g HCl/ml Isopropanol enthaltenden Lösung) versetzt. Man destillierte die Lösungsmittel im Vakuum ab und verrieb den Rückstand mit trockenem Acetonitril. Der entstandene kristalline Niederschlag wurde abgenutscht, zweimal mit je 5 ml frischem Acetonitril gründlich gewaschen und im Vakuum getrocknet. Fp. 205-207 °C.

$C_{27}H_{38}N_6O_2$.2 HCl.$H_2O$ (569,57)

| | | | | | | | | |
|---|---|---|---|---|---|---|---|---|
| Ber.: | C | 56,94 | H | 7,43 | Cl | 12,45 | N | 14,75 |
| Gef.: | | 56,35 | | 7,29 | | 12,67 | | 14,91 |

Beispiel 92

5,11-Dihydro-11-[[[2-[2-[(dipropylamino)methyl]-1-piperi dinyl]ethyl]amino]carbonyl]-6H-pyrido[2,3-b][1,4]-benzo diazepin-6-on-hydrobromid

Die Lösung von 0,957 g (0,002 mol) 5,11-Dihydro-11-[[[2-[2-[(dipropylamino)methyl]-1-piperidinyl]ethyl]-amino]carbonyl]-6H-pyrido[2,3-b][1,4]benzodiazepin-6-on in 20 ml Ethanol wurde mit 0,135 ml einer 45proz.

wässrigen Bromwasserstofflösung (0,002 mol) versetzt. Aus dem Gemisch kristallisierte langsam das obige farblose Salz aus, das nach 3-stündigem Stehen abgenutscht, dreimal gründlich mit je 3 ml wasserfreiem Ethanol gewaschen und schließlich im Umlufttrockenschrank bei 50 °C getrocknet wurde.
$C_{27}H_{38}N_6O_2$.HBR (559,55)

| | | | | | | | | |
|---|---|---|---|---|---|---|---|---|
| Ber.: | C | 57,96 | H | 7,02 | Br | 14,29 | N | 15,02 |
| Gef.: | | 57,91 | | 7,06 | | 14,27 | | 15,08 |

Beispiel 93

5,11-Dihydro-11-[[[2-[2-[(dipropylamino)methyl]-1-piperidinyl]ethyl]amino]carbonyl]-6H-pyrido[2,3-b][1,4]-benzodiazepin-6-on-sesquimaleinat

Das aus entsprechenden Mengen an 5,11-Dihydro-11-[[[2-[2-[(dipropylamino)methyl]-1-piperidinyl]-ethyl]amino]carbonyl]-6H-pyrido[2,3-b][1,4]benzodiazepin-6-on und Maleinsäure in wasserfreiem Ethanol erhaltene Salz schmolz bei 134-137 °C.
$C_{27}H_{38}N_6O_2$.1,5 $C_4H_4O_4$ (652,75)

| | | | | | | |
|---|---|---|---|---|---|---|
| Ber.: | C | 60,72 | H | 6,79 | N | 12,87 |
| Gef.: | | 60,07 | | 6,88 | | 12,68 |

Beispiel 94

5,11-Dihydro-11-[[[2-[2-[(dipropylamino)methyl]-1-piperidinyl]ethyl]amino]carbonyl]-6H-pyrido[2,3-b][1,4]-benzodiazepin-6-on-sulfat

Hergestellt entsprechend Beispiel 91, jedoch unter Verwendung einer ethanolischen Lösung von Schwefelsäure an Stelle der isopropanolischen Chlorwasserstofflösung. Farblose Kristalle vom Fp. 165-167 °C (aus 2-Propanol/Essigsäureethylester).
$C_{27}H_{38}N_6O_2.H_2SO_4$ (576,71)

| | | | | | | | | |
|---|---|---|---|---|---|---|---|---|
| Ber.: | C | 56,23 | H | 6,99 | N | 14,57 | S | 5,56 |
| Gef.: | | 56,30 | | 7,17 | | 14,52 | | 5,55 |

Beispiel 95

11-[[[2-[2-[(Diethylamino)methyl]-1-piperidinyl]ethyl]amino]carbonyl]-5,11-dihydro-8-ethyl-6H-pyrido[2,3-b]-[1,4]benzodiazepin-6-on

Hergestellt analog Beispiel 2 aus 11-(Chlorcarbonyl)-5,11-dihydro-8-ethyl-6H-pyrido[2,3-b][1,4]-benzodiazepin-6-on und 2-[2-[(Diethylamino)methyl]-1-piperidinyl]ethanamin in einer Ausbeute von 70 % der Theorie. Farblose Kristalle vom Fp. 124-126 °C (Diisopropylether).

Beispiel 96

5,11-Dihydro-11-[[[2-[2-[(4-morpholinyl)methyl]-1-piperidinyl]ethyl]amino]carbonyl]-6H-pyrido[2,3-b][1,4]-benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[(4-Morpholinyl)methyl]-1-piperidinyl]ethanamin in einer Ausbeute von 30 % der Theorie. Farblose Kristalle vom Fp. 167 °C (Acetonitril unter Verwendung von Bleicherde).

Beispiel 97

5,11-Dihydro-11-[[[2-[2-[4-(1-piperidinyl)butyl]-1-piperidinyl]ethyl]amino]carbonyl]-6H-pyrido[2,3-b][1,4]-benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[4-(1-Piperidinyl)butyl]-1-piperidinyl]ethanamin in einer Ausbeute von 36 % der Theorie. Farblose Kristalle vom Fp. 154-155 °C (Acetonitril).

Beispiel 98

11-[[[2-[2-[(Diethylamino)methyl]-1-piperidinyl]-1,1-dimethylethyl]amino]carbonyl]-5,11-dihydro-6H-pyrido-[2,3-b][1,4]benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[(Diethylamino)methyl]-1-piperidinyl]-1,1-dimethylethan amin in einer Ausbeute von 62 % der Theorie. Farblose Kristalle vom Fp. 160-162 °C (Acetonitril und Essigsäureethylester).

Beispiel 99

11-[[[2-[2-[((Cyclopentyl)methylamino)methyl]-1-piperidinyl]ethyl]amino]carbonyl]-5,11-dihydro-6H-pyrido-[2,3-b][1,4]benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[((Cyclopentyl)methylamino)methyl]-1-piperidinyl]ethanamin in einer Ausbeute von 63 % der Theorie. Farblose Kristalle vom Fp. 157-158 °C (Acetonitril unter Verwendung von Aktivkohle).

Beispiel 100

(-)-4-[[[2-[2-[(Diethylamino)methyl]-1-piperidinyl]ethyl]amino]carbonyl]-4,9-dihydro-3-methyl-10H-thieno[3,4-b][1,5]benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und (-)-2-[2-[(Diethylamino)methyl]-1-piperidinyl]ethanamin in einer Ausbeute von 53 % der Theorie. Farblose Kristalle vom Fp. 132-133 °C (Acetonitril); $[\alpha]_D^{20}$ = - 14,00 ° (verdünnte wässerige Salzsäure).

Beispiel 101

(+)-4-[[[2-[2-[(Diethylamino)methyl]-1-piperidinyl]ethyl]amino]carbonyl]-4,9-dihydro-3-methyl-10H-thieno-[3,4-b][1,5]benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und (+)-2-[2-[(Diethylamino)methyl]-1-piperidinyl]ethanamin in einer Ausbeute von 55 % der Theorie. Farblose Kristalle vom Fp. 131-132 °C (Acetonitril); $[\alpha]_D^{20}$ = + 13,66° (verdünnte wässerige Salzsäure).

Beispiel 102

11-[[[2-[2-[[Bis(methylethyl)amino]methyl]-1-piperidinyl] ethyl]amino]carbonyl]-5,11-dihydro-6H-pyrido[2,3-b][1,4] benzodiazepin-6-on

Hergestellt analog Beispiel 1 aus 11-(Chlorcarbonyl)-5,11-dihydro-6H-pyrido[2,3-b][1,4]benzodiazepin-6-on und 2-[2-[[Bis(methylethyl)amino]methyl]-1-piperidinyl]ethanamin in einer Ausbeute von 34 % der Theorie. Farblose Kristalle vom Fp. 181-182 °C (Acetonitril unter Verwendung von Aktivkohle).

Beispiel 103

11-[[[2-[2-[(Butylethylamino)methyl]-1-piperidinyl]ethyl] amino]carbonyl]-5,11-dihydro-6H-pyrido[2,3-b][1,4]-benzo diazepin-6-on

Hergestellt analog Beispiel 1 aus 11-(Chlorcarbonyl)-5,11-dihydro-6H-pyrido[2,3-b][1,4]benzodiazepin-6-on und 2-[2- [(Butylethylamino)methyl]-1-piperidinyl]ethanamin in einer Ausbeute von 16 % der Theorie. Farblose Kristalle vom Fp. 156-157 °C (Acetonitril).

Beispiel 104

5,11-Dihydro-11-[[[2-[2-[(hexahydro-1H-azepin-1-yl)methyl]-1-piperidinyl]ethyl]amino]carbonyl]-6H-pyrido-[2,3-b][1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[(Hexahydro-1H-azepin-1-yl)methyl]-1-piperidinyl]ethanamin in einer Ausbeute von 39 % der Theorie. Farblose Kristalle vom Fp. 156-158 °C (Acetonitril).

Beispiel 105

6-Chlor-5,10-dihydro-5-[[[2-[2-[(dipropylamino)methyl]-1-piperidinyl]ethyl]amino]carbonyl]-11H-dibenzo[b,e]-[1,4] diazepin-11-on

Hergestellt analog Beispiel 1 aus 6-Chlor-5-(chlorcarbonyl)-5,10-dihydro-11H-dibenzo[b,e][1,4]diazepin-11-on und 2-[2-[(Dipropylamino)methyl]-1-piperidinyl]ethanamin in einer Ausbeute von 2,6 % der Theorie. Farblose Kristalle vom Fp. 71-78 °C, vermutlich Gemisch von 2 Diastereomeren.
$C_{28}H_{38}ClN_5O_2$ (512,09)

| | | | | | | |
|---|---|---|---|---|---|---|
| Ber.: | C | 65,67 | H | 7,48 | N | 13,66 |
| Gef.: | | 65,69 | | 7,74 | | 13,60 |

Beispiel 106

5,11-Dihydro-11-[[[2-[2-[(ethylpropylamino)methyl]-1-piperidinyl]ethyl]amino]carbonyl]-6H-pyrido[2,3-b][1,4] benzodiazepin-6-on

Hergestellt analog Beispiel 2 aus 11-(Chlorcarbonyl)-5,11-dihydro-6H-pyrido[2,3-b][1,4]benzodiazepin-6-on und 2-[2-[(Ethylpropylamino)methyl]-1-piperidinyl]ethanamin in einer Ausbeute von 75 % der Theorie. Farblose Kristalle vom Fp. 160-162 °C (Acetonitril).

Beispiel 107

(-)-5,11-Dihydro-11-[[[2-[2-[(dipropylamino)methyl]-1-piperidinyl]ethyl]amino]carbonyl]-6H-pyrido[2,3-b][1,4] benzodiazepin-6-on

Hergestellt analog Beispiel 1 aus 11-(Chlorcarbonyl)-5,11-dihydro-6H-pyrido[2,3-b][1,4]benzodiazepin-6-on und (-)-2-[2-[(Dipropylamino)methyl]-1-piperidinyl]ethanamin in einer Ausbeute von 90 % der Theorie. Farblose Kristalle vom Fp. 164-165 °C (Acetonitril); $[\alpha]_D^{20}$ = - 7,62° (verdünnte wässerige Salzsäure).

Beispiel 108

(+)-5,11-Dihydro-11-[[[2-[2-[(dipropylamino)methyl]-1-piperidinyl]ethyl]amino]carbonyl]-6H-pyrido[2,3-b]-[1,4] benzodiazepin-6-on

Hergestellt analog Beispiel 2 aus 11-(Chlorcarbonyl)-5,11-dihydro-6H-pyrido[2,3-b][1,4]benzodiazepin-6-on und (+)-2- [2-[(Dipropylamino)methyl]-1-piperidinyl]ethanamin in einer Ausbeute von 60 % der Theorie. Farblose Kristalle vom Fp. 164-165 °C (Acetonitril); $[\alpha]_D^{20}$ = + 7,24° (verdünnte wässerige Salzsäure).

Beispiel 109

5,11-Dihydro-11-[[[2-[2-[(dipropylamino)methyl]-1-piperidinyl]ethyl]amino]carbonyl]-6H-pyrido[2,3-b][1,4]-benzodiazepin-6-on

Die abgedunkelte Mischung aus 1,806 g (0,005 mol) 11-[[[2-Bromethyl]amino]carbonyl]-5,11-dihydro-6H-pyrido[2,3-b][1,4]benzodiazepin-6-on, 0,992 g (0,005 mol) 2-[(Dipropylamino)methyl]piperidin, 20 ml wasserfreiem Acetonitril und 0,75 g (0,005 mol) Natriumiodid wurde unter Rühren und unter Stickstoff-Atmosphäre 4 Stunden unter Rückfluß gekocht. Man ließ erkalten, versetzte mit 50 ml 10proz. wässeriger Kaliumcarbonatlösung und trennte die organische Phase ab. Die wässerige Phase wurde insgesamt 5mal mit je 10 ml Dichlormethan extrahiert. Die vereinigten organischen Extrakte wurden über Natriumsulfat unter Zusatz von Tierkohle getrocknet und im Vakuum eingedampft. Der Rückstand wurde an Kieselgel (30-60 mμ) und unter Verwendung von Dichlormethan/Cyclohexan/Methanol/konz. Ammoniak 68/15/15/1 (v/v/v/v) zum Eluieren säulenchromatographisch gereinigt. Die entsprechenden Eluate wurden eingeengt, der Rückstand einmal aus Acetonitril umkristallisiert und im Vakuumtrockenschrank bei 50 °C bis zur Gewichtskonstanz getrocknet. Man erhielt 0,7 g (29 % der Theorie) an farblosen Kristallen vom Fp. 163-164 °C, die nach Mischschmelzpunkt, Dünnschichtchromatogramm und IR-Spektrum identisch waren mit einem nach Beispiel 29 hergestellten Präparat.

Beispiel 110

4-[[[2-[2-[4-(Diethylamino)butyl]-1-piperidinyl]ethyl]amino] carbonyl]-4,9-dihydro-3-methyl-10H-thieno[3,4-b]-[1,5]benzo diazepin-10-on

Hergestellt analog Beispiel 109 aus 4-[[[2-Bromethyl]amino]carbonyl]-4,9-dihydro-3-methyl-10H-thieno-

[3,4-b][1,5]benzodiazepin-10-on und 4-[4-(Diethylamino)butyl]piperidin in einer Ausbeute von 51 % der Theorie. Farblose Kristalle vom Fp. 130-131 °C.

Beispiel 111

4,9-Dihydro-4-[[[2-[4-[4-(1-piperidinyl)butyl]-1-piperidinyl]ethyl]amino]carbonyl]-3-methyl-10H-thieno[3,4-b]-[1,5] benzodiazepin-10-on

Hergestellt analog Beispiel 109 aus 4-[[[2-Bromethyl]amino]carbonyl]-4,9-dihydro-3-methyl-10H-thieno-[3,4-b][1,5]benzodiazepin-10-on und 4-[4-(1-Piperidinyl)butyl]piperidin in einer Ausbeute von 20 % der Theorie. Farblose Kristalle vom Fp. 186-187 °C.

Beispiel 112

11-[[[2-[4-[4-(Diethylamino)butyl]-1-piperidinyl]ethyl]amino]carbonyl]-5,11-dihydro-6H-pyrido[2,3-b][1,4]-benzo diazepin-6-on

Hergestellt analog Beispiel 109 aus 11-[[[2-Bromethyl]amino]carbonyl]-5,11-dihydro-6H-pyrido[2,3-b]-[1,4]benzodiazepin-6-on und 4-[4-(Diethylamino)butyl]piperidin, jedoch unter Verwendung von Dimethylformamid an Stelle von Acetonitril, in einer Ausbeute von 60 % der Theorie. Farblose Kristalle vom Fp. 167 °C (Essigsäureethylester).

Beispiel 113

9-Chlor-11-[[[2-[4-[4-(diethylamino)butyl]-1-piperidinyl]ethyl]amino]carbonyl]-5,11-dihydro-6H-pyrido[2,3-b]-[1,4] benzodiazepin-6-on

Hergestellt analog Beispiel 109 aus 11-[[[2-Bromethyl]amino]carbonyl]-9-chlor-5,11-dihydro-6H-pyrido-[2,3-b][1,4]benzodiazepin-6-on und 4-[4-(Diethylamino)butyl]piperidin, jedoch unter Verwendung von Dimethylformamid an Stelle von Acetonitril, in einer Ausbeute von 19 % der Theorie. Farblose Kristalle vom Fp. 157 °C.

Beispiel 114

4,9-Dihydro-4-[[[2-[4-[3-(hexahydro-1H-azepin-1-yl)propyl]-1-piperidinyl]ethyl]amino]carbonyl]-3-methyl-10H-thieno [3,4-b][1,5]benzodiazpein-10-on

Hergestellt analog Beispiel 109 aus 4-[[[2-Bromethyl]amino]carbonyl]-4,9-dihydro-3-methyl-10H-thieno-[3,4-b][1,5]benzodiazepin-10-on und 4-[3-(Hexahydro-1H-azepin-1-yl)propyl]piperidin in einer Ausbeute von 29 % der Theorie. Farblose Kristalle vom Fp. 144-145 °C. $R_F$ 0,5 (Macherey-Nagel, Polygram® SIL G/$UV_{254}$, pre-coated plastic sheets for TLC; Fließmittel: Dichlormethan/Cyclohexan/Methanol/konz. Ammoniak 68/15/15/2 v/v/v/v).

Beispiel 115

4,9-Dihydro-3-methyl-4-[[[2-[4-[3-(1-methyl-2-pyrrolidinyl)propyl]-1-piperidinyl]ethyl]amino]carbonyl]-10H-thieno [3,4-b][1,5]benzodiazpein-10-on

Hergestellt analog Beispiel 109 aus 4-[[[2-Bromethyl]amino]carbonyl]-4,9-dihydro-3-methyl-10H-thieno-[3,4-b][1,5]benzodiazepin-10-on und 4-[3-(1-Methyl-2-pyrrolidinyl)propyl]piperidin in einer Ausbeute von 18 % der Theorie. Farblose Kristalle vom Fp. 145-146° C. (aus Essigsäurethylester/Ethanol). $R_F$ 0,5 (DC-Untersuchung wie im Beispiel 114).

Im folgenden wird die Herstellung pharmazeutischer Anwendungsformen anhand einiger Beispiele beschrieben:

Beispiel I

Tabletten mit 5 mg 5,11-Dihydro-11-[[[2-[2-[(dipropyl amino)methyl]-1-piperidinyl]ethyl]amino]carbonyl]-6H-pyrido[2,3-b][1,4]benzodiazepin-6-on-methansulfonat

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzukker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.

Tablettengewicht: 220 mg

Stempel: 9 mm

Beispiel II

Dragées mit 5 mg 5,11-Dihydro-11-[[[2-[2-[(dipropyl amino)methyl]-1-piperidinyl]ethyl]amino]carbonyl]-6H-pyrido[2,3-b][1,4]benzodiazepin-6-on-methansulfonat

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg 5,11-Dihydro-11-[[[2-[2-[(dipropyl amino)methyl]-1-piperidinyl]ethyl]amino]carbonyl]-6H-pyrido[2,3-b][1,4]benzodiazepin-6-on-methansulfonat

Zusammensetzung:
1 Ampulle enthält:

| | | | |
|---|---|---|---|
| Wirkstoff | | 10,0 | mg |
| Natriumchlorid | | 8,0 | mg |
| Dest. Wasser | ad | 1 | ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation:20 Minuten bei 120 °C.

Beispiel IV

Suppositorien mit 20 mg 5,11-Dihydro-11-[[[2-[2-[(dipropyl amino)methyl]-1-piperidinyl]ethyl]amino]-carbonyl]-6H-pyrido[2,3-b][1,4]benzodiazepin-6-on-methansulfonat

Zusammensetzung:
1 Zäpfchen enthält:

| | |
|---|---|
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.
Zäpfchengewicht 1,7 g

Beispiel V

Tropfen mit 5,11-Dihydro-11-[[[2-[2-[(dipropylamino) methyl]-1-piperidinyl]ethyl]amino]carbonyl]-6H-pyrido-[2,3-b][1,4]benzodiazepin-6-on-methansulfonat

Zusammensetzung:

100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| | | |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

## Ansprüche

1. Neue kondensierte Diazepinone der allgemeinen Formel I

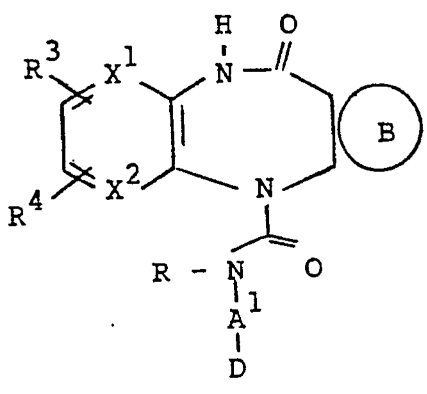

(I)

in der

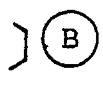

einen der zweiwertigen Reste

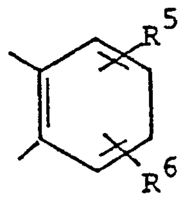, 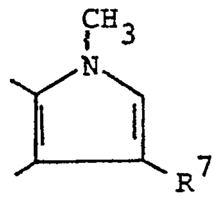, 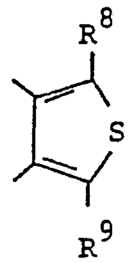, 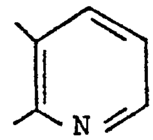 oder 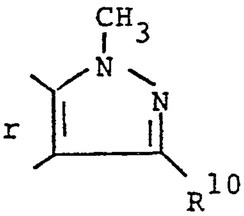

(S) (T) (U) (V) (W)

und D die Gruppen

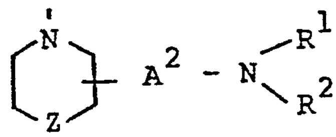, 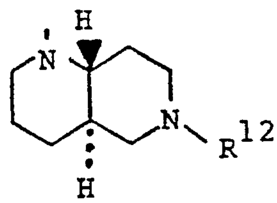, 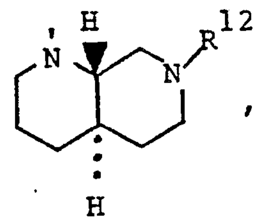,

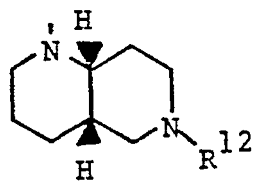 und 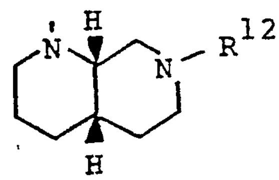

darstellen und

$X^1$, $X^2$, $A^1$, $A^2$, R, $R^1$ bis $R^{10}$, $R^{12}$ und Z die folgenden Bedeutungen besitzen:

$X^1$ und $X^2$ stellen eine =CH-Gruppe dar oder, sofern

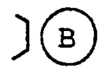

die Bedeutungen der oben genannten, zweiwertigen Reste S, U oder W annimmt, können beide oder nur $X^1$ oder nur $X^2$ auch ein Stickstoffatom sein;

$A^1$ ist ein geradkettiger oder verzweigter gesättigter Alkylenrest mit zwei bis sieben Kohlenstoffatomen;

$A^2$ ein geradkettiger oder verzweigter gesättigter Alkylenrest mit 1 bis 5 Kohlenstoffatomen oder, wenn er sich in 3-Stellung zum Stickstoff des gesättigten heterocyclischen Ringes befindet, auch eine Einfachbindung;

Z eine Einfachbindung, ein Sauerstoff- oder Schwefelatom, die Methylen- oder 1,2-Ethylengruppe;

R ein Wasserstoffatom oder eine Methylgruppe;

$R^1$ ein verzweigter oder unverzweigter Alkylrest mit 1 bis 4 Kohlenstoffatomen;

$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;

$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder durch die $N-CH_3$- Gruppe unterbrochen sein kann;

$R^3$ ist eine Alkylgruppe mit 1 bis 4 C-Atomen, ein Chloratom oder ein Wasserstoffatom;

$R^4$ ein Wasserstoffatom oder eine Methylgruppe;

$R^5$ und $R^6$ bedeuten jeweils ein Wasserstoffatom, ein Fluor-, Chlor- oder Bromatom oder eine Alkylgruppe mit 1 bis 4 C-Atomen;

$R^7$ ein Wasserstoff- oder Chloratom oder eine Methylgruppe;

$R^8$ ein Wasserstoffatom oder einen Alkylrest mit 1 bis 4 Kohlenstoffatomen;

$R^9$ ein Wasserstoff- oder Halogenatom oder eine Alkylgruppe mit 1 bis 4 Kohlenstoffatomen; und
$R^{10}$ ein Wasserstoffatom oder eine Methylgruppe und
$R^{12}$ einen verzweigten oder unverzweigten Alkylrest mit 1 bis 6 Kohlenstoffatomen,
wobei für den Fall, daß

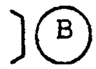

den zweiwertigen Rest T darstellt und $R^7$ ein Wasserstoffatom ist, $R^3$ kein Chloratom und Z kein Schwefelatom bedeuten können,

ihre Diastereomeren und Enantiomeren, und ihre physiologisch verträglichen Salze mit anorganischen oder organischen Säuren.

2. Neue kondensierte Diazepinone der allgemeinen Formel I gemäß Anspruch 1, in der
$X^1$ eine =CH-Gruppe,
$X^2$ entweder ein Stickstoffatom, sofern

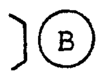

den zweiwertigen Rest S darstellt, mit der Maßgabe, daß $R^3$, $R^4$ und $R^5$ Wasserstoffatome sind und $R^6$ ein Wasserstoffatom, ein Chlor- oder Bromatom oder eine Methyl- oder Ethylgruppe in 8- oder 9-Stellung des Heterocyclus ist, oder eine =CH-Gruppe ist, wenn

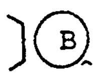

die Bedeutung des zweiwertigen Restes U annimmt, wobei $R^8$ ein Wasserstoffatom und $R^9$ eine Methylgruppe ist;
$A^1$ eine 1,2-Ethylengruppe;
$A^2$ eine geradkettige Alkylengruppe mit 1 bis 5 Kohlenstoffatomen;
Z eine Methylengruppe;
R ein Wasserstoffatom und
$R^1$ und $R^2$ Alkylgruppen mit 1 bis 3 Kohlenstoffatomen oder zusammen mit dem dazwischenliegenden Stickstoffatom den Piperidinylrest darstellen,

ihre Diastereomeren und Enantiomeren und ihre physiologisch verträglichen Salze mit anorganischen oder organischen Säuren.

3. Neue kondensierte Diazepinone der allgemeinen Formel I gemäß Anspruch 1, in der
$X^1$ die Gruppe =CH-,
$X^2$ ein Stickstoffatom oder die Gruppe =CH-,

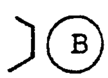

einen zweiwertigen Rest S oder U,
$A^1$ die Ethylengruppe
$A^2$ die Methylen- oder 1,3-Propylengruppe,
Z die Methylengruppe,
R ein Wasserstoffatom oder die Methylgruppe,
$R^1$ und $R^2$, die gleich oder verschieden sein können, einen Alkylrest mit 1 bis 3 Kohlenstoffatomen,
$R^3$ und $R^4$, die gleich oder verschieden sein können, Wasserstoffatome oder die Methylgruppe,
$R^5$, $R^6$ Wasserstoffatome,
$R^8$ und $R^9$ Wasserstoffatome oder einer dieser Reste eine Methylgruppe bedeuten,

ihre Diastereomeren und Enantiomeren und ihre physiologisch verträglichen Salze mit anorganischen oder organischen Säuren.

4. Als neue Verbindungen der allgemeinen Formel I gemäß Anspruch 1
5,11-Dihydro-11-[[[2-[2-[(dipropylamino)methyl]-1-piperi-dinyl]ethyl]amino]carbonyl]-6H-pyrido[2,3-b][1,4]benzo-diazepin-6-on,
4-[[[2-[2-[(Diethylamino)methyl]-1-piperidinyl]ethyl]amino]carbonyl]-4,9-dihydro-3-methyl-10H-thieno[3,4-b][1,5]benzodiazepin-10-on,
9-Chlor-11-[[[2-[2-[(diethylamino)methyl]-1-piperidinyl]ethyl]amino]carbonyl]-5,11-dihydro-6H-pyrido-[2,3-b][1,4]benzodiazepin-6-on,
ihre Diastereomeren und Enantiomeren und ihre physiologisch verträglichen Salze mit anorganischen oder organischen Säuren.

5. Arzneimittel, enthaltend eine Verbindung gemäß den Ansprüchen 1 bis 4 neben einem oder mehreren inerten Träger-und/oder Hilfsstoffen.

6. Verwendung von Verbindungen gemäß den Ansprüchen 1 bis 4 zur Herstellung von Arzneimitteln zur Behandlung von Bradycardien und Bradyarrhythmien.

7. Verfahren zur Herstellung von neuen kondensierten Diazepinonen der allgemeinen Formel I

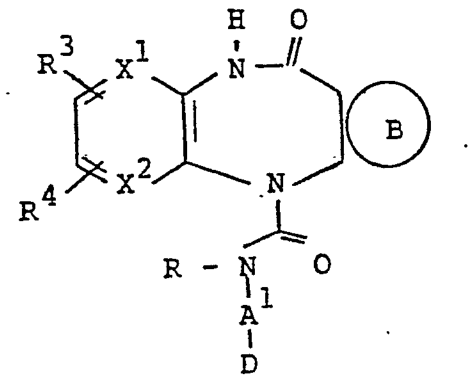

(I)

in der

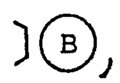

D, $X^1$, $X^2$, $A^1$, $A^2$, R, $R^1$ bis $R^{10}$, $R^{12}$ bis Z wie in Anspruch 1 angegeben definiert sind,
und von ihren Diastereomeren oder Enantiomeren und ihren physiologisch verträglichen Salzen mit anorganischen oder organischen Säuren, dadurch gekennzeichnet, daß
a.) zur Herstellung von kondensierten Diazepinonen der allgemeinen Formel Ia

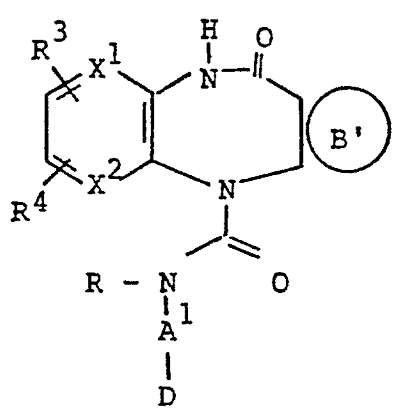

(Ia)

in der

R, $R^3$, $R^4$, $X^1$, $X^2$, $A^1$ und D die oben angegebenen Bedeutungen haben und

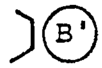

einen der zweiwertigen Reste S, U, V, W oder T'

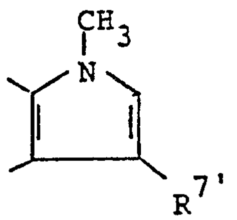

(T')

darstellt, wobei $R^{7'}$ ein Chloratom oder eine Methylgruppe ist, ein Kohlensäurederivat der allgemeinen Formel II

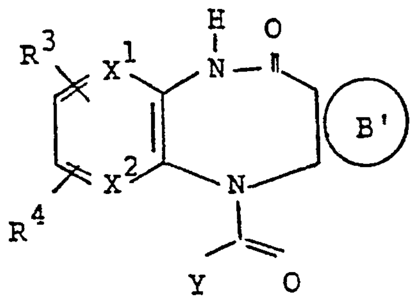

(II)

in der
$R^3$, $R^4$,

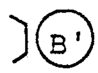

, $X^1$, $X^2$ wie oben definiert sind, und Y ein Halogenatom oder den Rest $OR^{11}$ bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit einer Verbindung der allgemeinen Formel III oder IIIa,

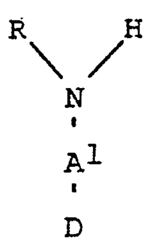

(III) oder

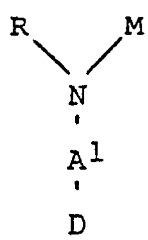

(IIIa)

in welchen
R, $A^1$ und D wie eingangs erwähnt definiert sind und M ein Alkalimetallatom oder 1 Äquivalent eines Erdalkalimetallatoms darstellt, gegebenenfalls in Gegenwart eines Lösungsmittels bei Temperaturen zwischen -10 °C und dem Siedepunkt des Reaktionsgemisches umgesetzt wird, oder

b.) zur Herstellung derselben kondensierten Diazepinone der allgemeinen Formel Ia mit den oben angegebenen Bedeutungen für

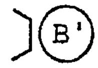

und die Substituenten R, $R^3$, $R^4$, $X^1$, $X^2$, $A^1$ und D, eine tricyclische Verbindung der allgemeinen Formel IV

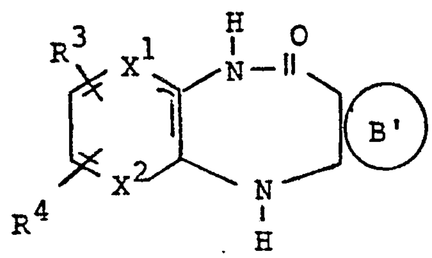

(IV)

in der die Reste $R^3$, $R^4$, $X^1$, $X^2$ und

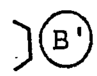

wie oben definiert sind, mit einem Chlorkohlensäurederivat der allgemeinen Formel V

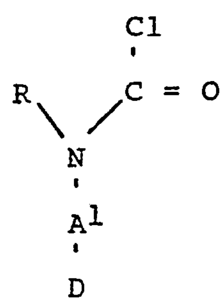

(V)

oder - sofern in Verbindungen der allgemeinen Formel Ia R ein Wasserstoffatom sein soll - auch mit einem Isocyanat der allgemeinen Formel Va

$$O = C = N - A^1 - D \qquad (Va)$$

in welchen die Reste R, $A^1$ und D die oben erwähnten Bedeutungen haben, vorzugsweise in Lösungsmitteln bei Temperaturen bis zum Siedepunkt des Reaktionsgemisches umgesetzt wird oder

c.) zur Herstellung von unter die allgemeine Formel I fallenden Pyrrolo-kondensierten Diazepinonen der allgemeinen Formel Ib

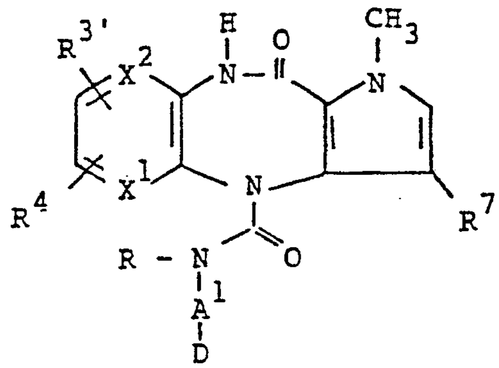

(Ib)

worin

$R^4$, $X^1$, $X^2$, $A^1$, und D die eingangs erwähnten Bedeutungen haben,

$R^{3'}$ eine Alkylgruppe mit 1 bis 4 C-Atomen oder ein Wasserstoffatom, $R^7$ ein Wasserstoffatom und für die Gruppe

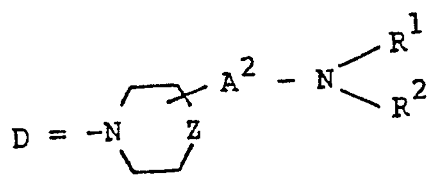

Z die eingangs genannten Bedeutungen mit Ausnahme eines Schwefelatoms hat, wobei $R^1$, $R^2$ und $A^2$ wie oben definiert sind,

eine Verbindung der allgemeinen Formel Ib, in der $R^7$ ein Chloratom bedeutet, einer Hydrogenolyse entweder mit Wasserstoff in Gegenwart von Katalysatoren von Metallen der VIII. Nebengruppe des Periodensystems der Elemente bei einem Wasserstoffdruck von 1 bis 300 bar, oder mit Ameisensäure oder Trialkylammoniumformiat in Gegenwart von Palladium auf Kohle oder Palladiumacetat und Triarylphosphinen bei Temperaturen zwischen 0 °C bis 130 °C und in Gegenwart eines Lösungsmittels unterworfen wird,

d.) zur Herstellung der unter a) beschriebenen kondensierten Diazepinone der allgemeinen Formel Ia mit den oben angegebenen Bedeutungen für

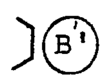

und für die Substituenten R, $R^3$, $R^4$, $X^1$, $X^2$, $A^1$ und D, kondensierte Diazepinone der allgemeinen Formel VIII

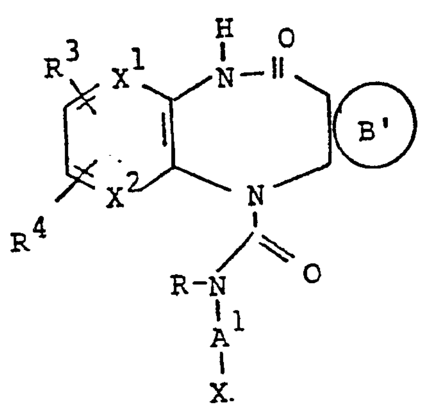

(VIII)

in der

R, $R^3$, $R^4$, $X^1$, $X^2$ und $A^1$ die angegebenen Bedeutungen haben und

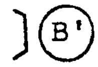

wie vorstehend definiert ist und X eine nucleofuge Gruppe bzw. Austrittsgruppe ist, mit Diaminen der allgemeinen Formel IIIc

$$H - D \qquad (IIIc)$$

in der
D die eingangs genannten Bedeutungen hat, in einer inerten Lösungsmittel bei Temperaturen zwischen -10° C und den Siedepunkt des Reaktionsgemisches, gebenenfalls in Gegenwart einer Hilfsbase, und falls X in der Verbindung der allgemeinen Formel VIII die Hydroxygruppe bedeutet, in Gegenwart von Katalysatoren der VIII. Nebengruppe des Periodensystems vorzugsweise in Anwesenheit aprotischer, inerter Lösungsmittel, umgesetzt werden,

und, falls erwünscht, die so erhaltenen Verbindungen der allgemeinen Formel I in ihre Enantiomeren aufgetrennt, und, gegebenenfalls, die erhaltenen Verbindungen der allgemeinen Formel I, falls sie in Salzform vorliegen, in ihre freie Basen oder, falls sie als freie Basen vorliegen, in ihre physiologisch verträglichen Salze mit anorganischen oder organischen Säuren übergeführt werden.

**8.** Verfahren nach Anspruch 7a, dadurch gekennzeichnet, daß die Umsetzung in Gegenwart aprotischer polarer Lösungsmittel und in Gegenwart zusätzlicher anorganischer oder organischer Basen oder in Gegenwart eines Überschusses einer Verbindung der allgemeinen Formel III durchgeführt wird, wobei, falls eine Metallverbindung der allgemeinen Formel IIIa verwendet wird, diese aus einer Verbindung der allgemeinen Formel III in situ erzeugt wird.

**9.** Verfahren nach Anspruch 7b, dadurch gekennzeichnet, daß die Umsetzung in einem inerten organischen Lösungsmittel, in Gegenwart tertiärer organischer Basen und bei Temperaturen zwischen 30 und 100° C erfolgt.

**10.** Verfahren nach Anspruch 7c, dadurch gekennzeichnet, daß die Hydrogenolyse drucklos mit Ameisensäure in Gegenwart von Dimethylformamid und Palladium auf Kohle bei 70 bis 110° C, mit Trimethylammoniumformiat in Gegenwart von überschüssigem Triethylamin und von Palladium auf Kohle, oder mit Palladiumacetat und Triarylphosphinen bei Temperaturen zwischen 40 und 110° C erfolgt.

Patentansprüche für folgende Vertragsstaaten : GR, ES

**1.** Verfahren zur Herstellung von neuen kondensierten Diazepinonen der allgemeinen Formel I

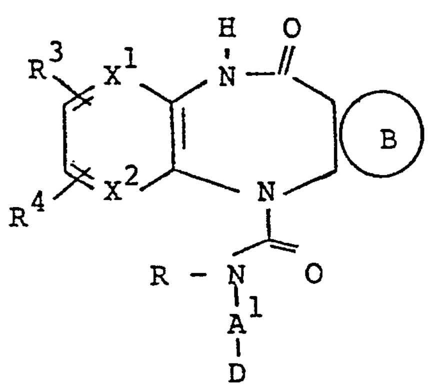

(I)

in der

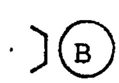

einen der zweiwertigen Reste

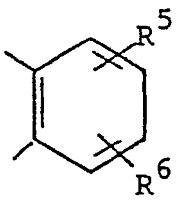 , 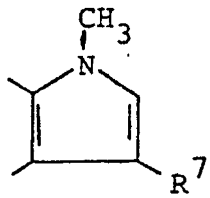 , 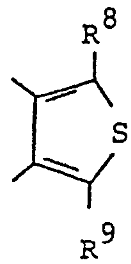 , 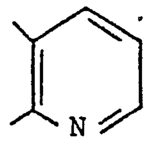 oder 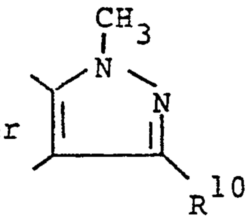

(S) (T) (U) (V) (W)

und D die Gruppen

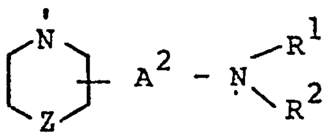 , 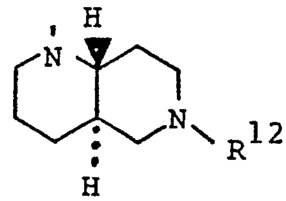 , 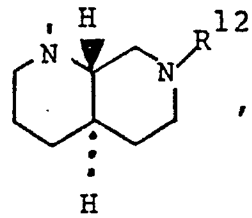 ,

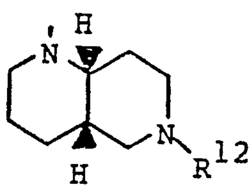 und 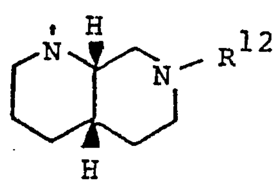

darstellen und

$X^1$, $X^2$, $A^1$, $A^2$, R, $R^1$ bis $R^{10}$, $R^{12}$ und Z die folgenden Bedeutungen besitzen:

$X^1$ und $X^2$ stellen eine =CH-Gruppe dar oder, sofern

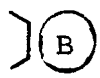

die Bedeutungen der oben genannten, zweiwertigen Reste S, U oder W annimmt, können beide oder nur $X^1$ oder nur $X^2$ auch ein Stickstoffatom sein;

$A^1$ ist ein geradkettiger oder verzweigter gesättigter Alkylenrest mit zwei bis sieben Kohlenstoffatomen;

$A^2$ ein geradkettiger oder verzweigter gesättigter Alkylenrest mit 1 bis 5 Kohlenstoffatomen oder, wenn er sich in 3-Stellung zum Stickstoff des gesättigten heterocyclischen Ringes befindet, auch eine Einfachbindung,

Z eine Einfachbindung, ein Sauerstoff- oder Schwefelatom, die Methylen- oder 1,2-Ethylengruppe;

R ein Wasserstoffatom oder eine Methylgruppe;

$R^1$ ein verzweigter oder unverzweigter Alkylrest mit 1 bis 4 Kohlenstoffatomen;

$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;

$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder durch die $N-CH_3$-Gruppe unterbrochen sein kann;

$R^3$ ist eine Alkylgruppe mit 1 bis 4 C-Atomen, ein Chloratom oder ein Wasserstoffatom;

$R^4$ ein Wasserstoffatom oder eine Methylgruppe;

$R^5$ und $R^6$ bedeuten jeweils ein Wasserstoffatom, ein Fluor-, Chlor- oder Bromatom oder eine

Alkylgruppe mit 1 bis 4 C-Atomen;

$R^7$ ein Wasserstoff- oder Chloratom oder eine Methylgruppe;

$R^8$ ein Wasserstoffatom oder einen Alkylrest mit 1 bis 4 Kohlenstoffatomen;

$R^9$ ein Wasserstoff- oder Halogenatom oder eine Alkylgruppe mit 1 bis 4 Kohlenstoffatomen;

$R^{10}$ ein Wasserstoffatom oder eine Methylgruppe und

$R^{12}$ ein verzweigter oder unverzweigter Alkylrest mit 1 bis 6 Kohlenstoffatomen

wobei für den Fall, daß

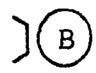

den zweiwertigen Rest T darstellt und $R^7$ ein Wasserstoffatom ist, $R^3$ kein Chloratom und Z kein Schwefelatom bedeuten können,

und von ihren Diastereomeren oder Enantiomeren und ihren physiologisch verträglichen Salzen mit anorganischen oder organsichen Säuren, dadurch gekennzeichnet, daß

a.) zur Herstellung von kondensierten Diazepinonen der allgemeinen Formel Ia

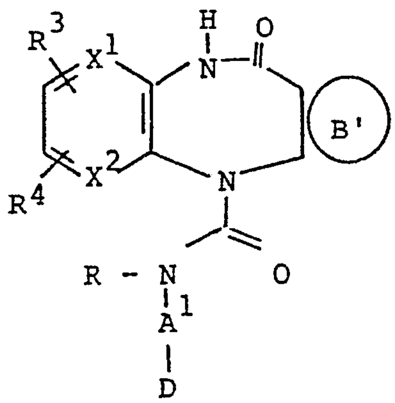

(Ia)

in der

R, $R^3$, $R^4$, $X^1$, $X^2$, $A^1$ und D die oben angegebenen Bedeutungen haben und

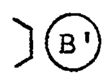

einen der zweiwertigen Reste S, U, V, W oder T'

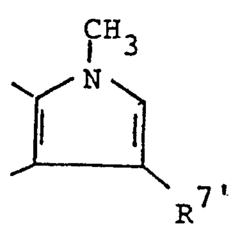

(T')

darstellt, wobei $R^{7'}$ ein Chloratom oder eine Methylgruppe ist,

ein Kohlensäurederivat der allgemeinen Formel II

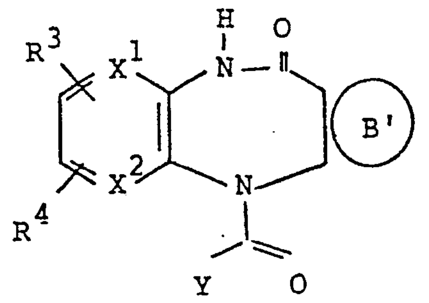

(II)

in der
$R^3$, $R^4$,

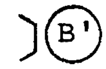

, $X^1$, $X^2$ wie oben definiert sind, und Y ein Halogenatom oder den Rest $OR^{11}$ bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit einer Verbindung der allgemeinen Formel III oder IIIa,

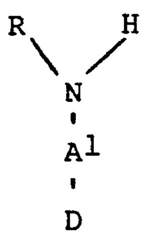

(III) oder

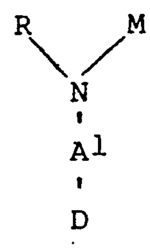

(IIIa)

in welchen
R, $A^1$ und D wie eingangs erwähnt definiert sind und M ein Alkalimetallatom oder 1 Äquivalent eines Erdalkalimetallatoms darstellt, gegebenenfalls in Gegenwart eines Lösungsmittels bei Temperaturen zwischen -10° C und dem Siedepunkt des Reaktionsgemisches umgesetzt wird, oder

b.) zur Herstellung derselben kondensierten Diazepinone der allgemeinen Formel Ia mit den oben angegebenen Bedeutungen für

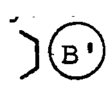

und die Substituenten R, $R^3$, $R^4$, $X^1$, $X^2$,
$A^1$ und D, eine tricyclische Verbindung der allgemeinen Formel IV

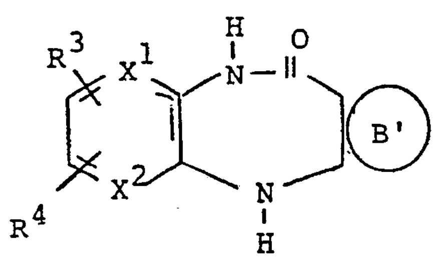

(IV)

in der
die Reste $R^3$, $R^4$, $X^1$, $X^2$ und

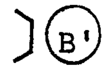

wie oben definiert sind, mit einem Chlorkohlensäurederivat der allgemeinen Formel V

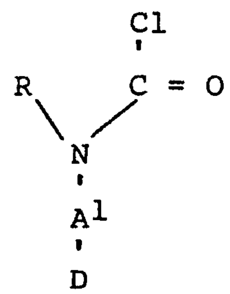

(V)

oder - sofern in Verbindungen der allgemeinen Formel Ia R ein Wasserstoffatom sein soll - auch mit einem Isocyanat der allgemeinen Formel Va

$$O = C = N - A^1 - D \qquad (Va)$$

in welchen die Reste R, $A^1$ und D die oben erwähnten Bedeutungen haben, vorzugsweise in Lösungsmitteln bei Temperaturen bis zum Siedepunkt des Reaktionsgemisches umgesetzt wird oder

c.) zur Herstellung von unter die allgemeine Formel I fallenden Pyrrolo-kondensierten Diazepinonen der alllgemeinen Formel Ib

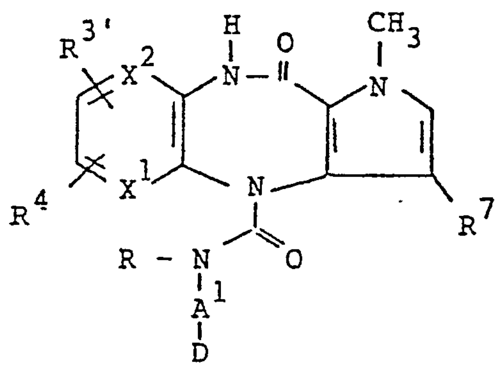

(Ib)

worin

$R^4$, $X^1$, $X^2$, $A^1$, und D die eingangs erwähnten Bedeutungen haben,

$R^{3'}$ eine Alkylgruppe mit 1 bis 4 C-Atomen oder ein Wasserstoffatom, $R^7$ ein Wasserstoffatom und für die Gruppe

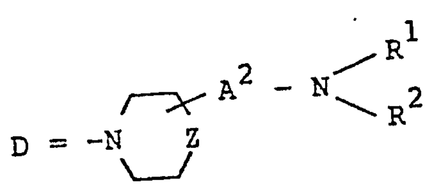

Z die eingangs genannten Bedeutungen mit Ausnahme eines Schwefelatoms hat, wobei $R^1$, $R^2$ und $A^2$ wie oben definiert sind,

eine Verbindung der allgemeinen Formel Ib, in der $R^7$ ein Chloratom bedeutet, einer Hydrogenolyse entweder mit Wasserstoff in Gegenwart von Katalysatoren von Metallen der VIII. Nebengruppe des Periodensystems der Elemente bei einem Wasserstoffdruck von 1 bis 300 bar, oder mit Ameisensäure

oder Trialkylammoniumformiat in Gegenwart von Palladium auf Kohle oder Palladiumacetat und Triarylphosphinen bei Temperaturen zwischen 0 ° C bis 130 ° C und in Gegenwart eines Lösungsmittels unterworfen wird,

d.) zur Herstellung der unter a) beschriebenen kondensierten Diazepinone der allgemeinen Formel Ia mit den oben angegebenen Bedeutungen für

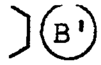

und für die Substituenten R, $R^3$, $R^4$, $X^1$, $X^2$, $A^1$ und D, kondensierte Diazepinone der allgemeinen Formel VIII

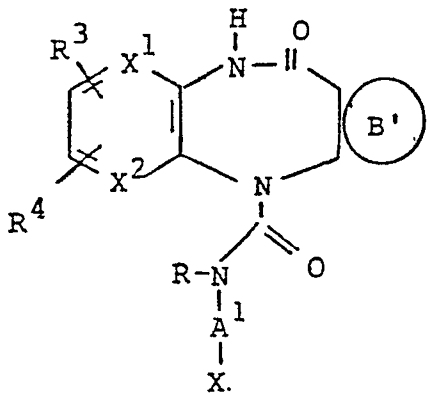

(VIII)

in der
R, $R^3$, $R^4$, $X^1$, $X^2$ und $A^1$ die angegebenen Bedeutungen haben und

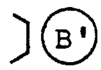

wie vorstehend definiert ist und X eine nucleofuge Gruppe bzw. Austrittsgruppe ist, mit Diaminen der allgemeinen Formel IIIc

$$H - D \qquad (IIIc)$$

in der
D die eingangs genannten Bedeutungen hat, in einem inerten Lösungsmittel bei Temperaturen zwischen -10 ° C und dem Siedepunkt des Reaktionsgemisches, gegebenenfalls in Gegenwart einer Hilfsbase, und falls X in der Verbindung der allgemeinen Formel VIII die Hydroxygruppe bedeutet, in Gegenwart von Katalysatoren der VIII. Nebengruppe des Periodensystems vor zugsweise in Anwesenheit aprotischer, inerter Lösungsmittel, umgesetzt werden,

und, falls erwünscht, die so erhaltenen Verbindungen der allgemeinen Formel I in ihre Enantiomeren aufgetrennt, und, gegebenenfalls, die erhaltenen Verbindungen der allgemeinen Formel I, falls sie in Salzform vorliegen, in ihre freie Basen oder, falls sie als freie Basen vorliegen, in ihre physiologisch verträglichen Salze mit anorganischen oder organischen Säuren übergeführt werden.

2. Verfahren nach Anspruch 1a, dadurch gekennzeichnet, daß die Umsetzung in Gegenwart aprotischer polarer Lösungsmittel und in Gegenwart zusätzlicher anorganischer oder organischer Basen oder in Gegenwart eines Überschusses einer Verbindung der allgemeinen Formel III durchgeführt wird, wobei, falls eine Metallverbindung der allgemeinen Formel IIIa verwendet wird, diese aus einer Verbindung der allgemeinen Formel III in situ erzeugt wird.

3. Verfahren nach Anspruch 1b, dadurch gekennzeichnet, daß die Umsetzung in einem inerten organischen Lösungsmittel, in Gegenwart tertiärer organischer Basen und bei Temperaturen zwischen 30 und 100 ° C erfolgt.

4. Verfahren nach Anspruch 1c, dadurch gekennzeichnet, daß die Hydrogenolyse drucklos mit Ameisensäure in Gegenwart von Dimethylformamid und Palladium auf Kohle bei 70 bis 110 °C, mit Trimethylammoniumformiat in Gegenwart von überschüssigem Triethylamin und von Palladium auf Kohle, oder mit Palladiumacetat und Triarylphosphinen bei Temperaturen zwischen 40 und 110 °C erfolgt.

## Claims

1. New condensed diazepinones of general formula I

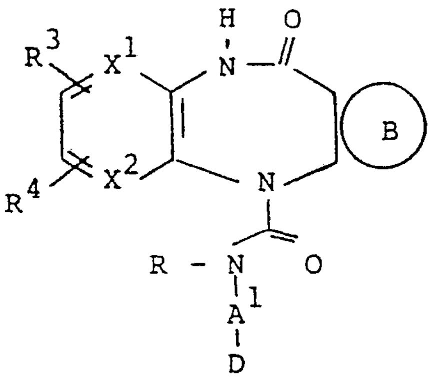

(I)

wherein

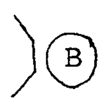

represents one of the divalent groups

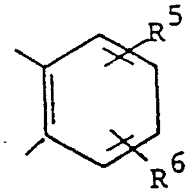 , 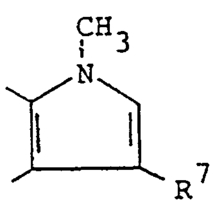 , 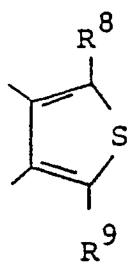 , 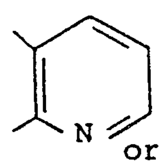 or 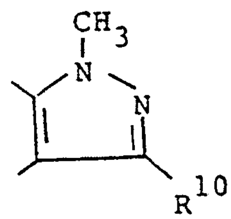

(S) (T) (U) (V) (W)

and D represents the groups

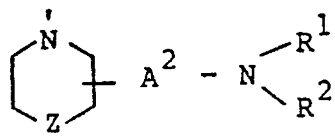 , 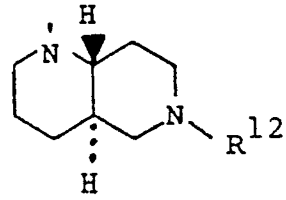 , 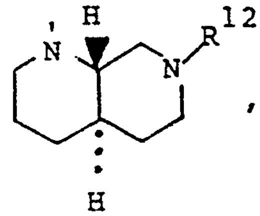 ,

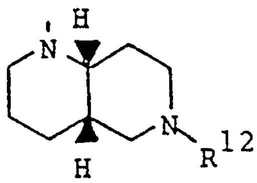 , and 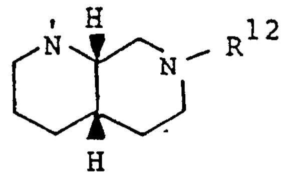

and $X^1$, $X^2$, $A^1$, $A^2$, R, $R^1$ to $R^{10}$, $R^{12}$ and Z have the following meanings:

$X^1$ and $X^2$ represent a =CH- group or, if

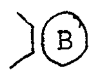

assumes the meanings of the above-mentioned divalent groups S, U or W, both or only $X^1$ or only $X^2$ may also represent a nitrogen atom;

$A^1$ is a straight-chained or branched saturated alkylene group with two to seven carbon atoms;

$A^2$ is a straight-chained or branched saturated alkylene group with 1 to 5 carbon atoms or, if it is in the 3-position relative to the nitrogen of the saturated heterocyclic ring, it may also represent a single bond;

Z represents a single bond, an oxygen or sulphur atom, a methylene or 1,2-ethylene group;

R represents a hydrogen atom or a methyl group;

$R^1$ represents a branched or unbranched alkyl group with 1 to 4 carbon atoms;

$R^2$ represents a branched or unbranched alkyl group with 1 to 7 carbon atoms which may optionally also be substituted by a hydroxy group at its second to seventh carbon atoms, or a cycloalkyl or cycloalkylmethyl group with 3 to 7 carbon atoms in the ring, whilst the cycloalkyl ring may optionally also be substituted by a hydroxy group;

$R^1$ and $R^2$ may, however, also form, together with the nitrogen atom between them, a 4- to 7-membered saturated, monocyclic, heterocyclic ring which may optionally be interrupted by an oxygen atom or by the $N\text{-}CH_3$ group;

$R^3$ is an alkyl group with 1 to 4 carbon atoms, a chlorine atom or a hydrogen atom;

$R^4$ represents a hydrogen atom or a methyl group;

$R^5$ and $R^6$ each represent a hydrogen atom, a fluorine, chlorine or bromine atom or an alkyl group with 1 to 4 carbon atoms;

$R^7$ represents a hydrogen or chlorine atom or a methyl group;

$R^8$ represents a hydrogen atom or an alkyl group with 1 to 4 carbon atoms;

$R^9$ represents a hydrogen or halogen atom or an alkyl group with 1 to 4 carbon atoms;

$R^{10}$ represents a hydrogen atom or a methyl group and

$R^{12}$ represents a branched or unbranched alkyl group with 1 to 6 carbon atoms,

whilst if

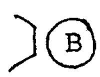

represents the divalent group T and R is a hydrogen atom, $R^3$ cannot represent a chlorine atom and Z cannot represent a sulphur atom,

the diastereomers and enantiomers thereof and their physiologically acceptable salts with inorganic or organic acids.

2. New condensed diazepinones of general formula I as claimed in claim 1, wherein

$X^1$ represents a =CH- group,

$X^2$ either represents a nitrogen atom and

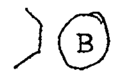

represents the divalent group S, with the proviso that $R^3$ $R^4$ and $R^5$ are hydrogen atoms and $R^6$ is a hydrogen atom, a chlorine or bromine atom or a methyl or ethyl group in the 8 or 9 position of the heterocycle,

or it is a =CH- group, if

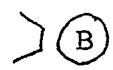

assumes the meaning of the divalent group U, whilst $R^8$ is a hydrogen atom and $R^9$ is a methyl group;

$A^1$ is a 1,2-ethylene group;

$A^2$ is a straight-chained alkylene group with 1 to 5 carbon atoms;

Z is a methylene group;

R is a hydrogen atom; and

$R^1$ and $R^2$ represent alkyl groups with 1 to 3 carbon atoms or together with the nitrogen atom between them they represent the piperidinyl group;

the diastereomers and enantiomers thereof and their physiologically acceptable salts with inorganic or organic acids.

3. New condensed diazepinones of general formula I as claimed in claim 1, wherein

$X^1$ represents the group =CH-,

$X^2$ represents a nitrogen atom or the group =CH-,

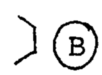

represents a divalent group S or U,

$A^1$ represents an ethylene group,

$A^2$ represents a methylene or 1,3-propylene group,

Z represents a methylene group,

R represents a hydrogen atom or a methyl group,

$R^1$ and $R^2$, which may be identical or different, represents an alkyl group with 1 to 3 carbon atoms,

$R^3$ and $R^4$, which may be identical or different, represent hydrogen atoms or a methyl group,

$R^5$ and $R^6$ represent hydrogen atoms,

$R^8$ and $R^9$ represent hydrogen atoms or one of these groups represents a methyl group,

the diastereomers and enantiomers thereof and their physiologically acceptable salts with inorganic or organic acids.

4. As new compounds of general formula I as claimed in claim 1:

5,11-dihydro-11-[[[2-12-[ (dipropylamino)methyl]-piperidin-1-yl]ethyl]amino]carbonyl]-6H-pyrido[2,3-b]-[1,4]benzodiazepin-6-one,

4-[[[2-[2-[ (diethylamino)methyl]-piperidin-1-yl]ethyl]-amino]carbonyl]-4,9-dihydro-3-methyl-10H-thieno[3,4-b]-[1,5]benzodiazepin-10-one,

9-chloro-11-[[[2-[2-[(diethylamino)methyl]-piperidin-1-yl]ethyl]amino]carbonyl]-5,11-dihydro-6H-pyrido[2,3-b][1,4]benzodiazepin-6-one,

the diastereomers and enantiomers thereof and their physiologically acceptable salts with inorganic or organic acids.

5. Pharmaceutical compositions containing a compound as claimed in claims 1 to 4 together with one or more inert carriers and/or excipients.

6. Use of compounds as claimed in claims 1 to 4 for the preparation of pharmaceutical compositions for the treatment of bradycardia and bradyarrhythmia.

7. Process for preparing new condensed diazepinones of general formula I

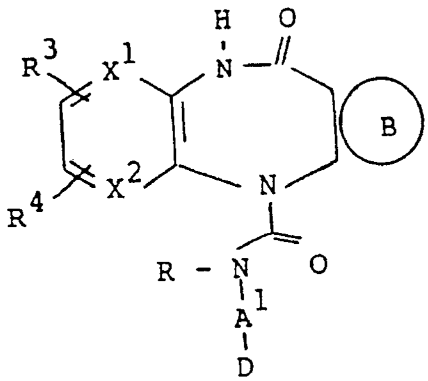

(I)

wherein

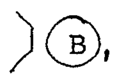

D, $X^1$, $X^2$, $A^1$, $A^2$, R, $R^1$ to $R^{10}$, $R^{12}$ to Z are defined as in claim 1,

and the diastereomers or enantiomers thereof and their physiologically acceptable salts with inorganic or organic acids, characterised in that

a) in order to prepare condensed diazepinones of general formula Ia

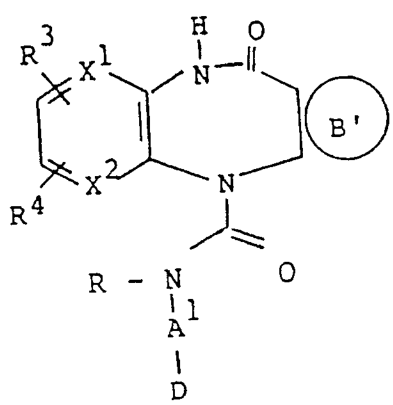

(Ia)

wherein R, $R^3$, $R^4$, $X^1$, $X^2$, $A^1$ and D are defined as hereinbefore and

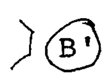

represents one of the divalent groups S, U, V, W or T'

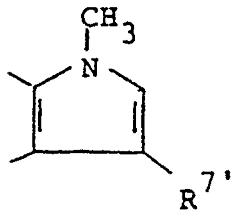

(T')

wherein $R^{7'}$ is a chlorine atom or a methyl group, a carbonic acid derivative of general formula II

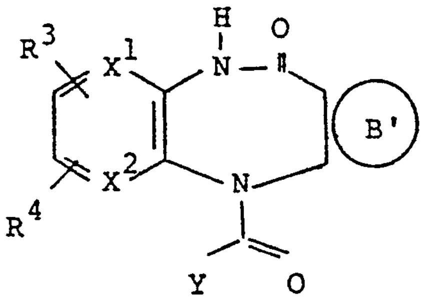

(II)

wherein
$R^3$, $R^4$,

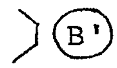

$X^1$ and $X^2$ are defined as hereinbefore and Y represents a halogen atom or the group $OR^{11}$, wherein $R^{11}$ represents an optionally halogen substituted alkyl group with 1 to 5 carbon atoms, a phenyl group optionally substituted by halogen atoms or nitro groups or an aralkyl group with 7 to 15 carbon atoms, is reacted with a compound of general formula III or IIIa

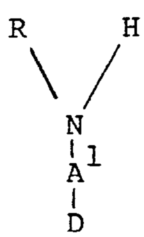

(III) or

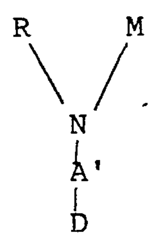

(IIIa)

wherein
R, $A^1$ and D are defined as hereinbefore, and M represents an alkali metal atom or 1 equivalent of an alkaline earth metal atom, optionally in the presence of a solvent at temperatures between -10° C and the boiling point of the reaction mixture, or

b) in order to prepare the same condensed diazepinones of general formula Ia with the above definitions for

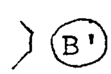

and the substituents R, $R^3$, $R^4$, $X^1$, $X^2$, $A^1$ and D, a tricyclic compound of general formula IV

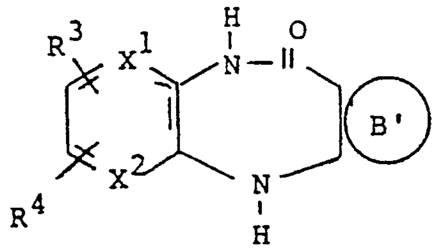

(IV)

wherein the groups $R^3$, $R^4$, $X^1$, $X^2$ and

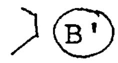

are defined as hereinbefore is reacted with a chlorocarbonic acid derivative of general formula V

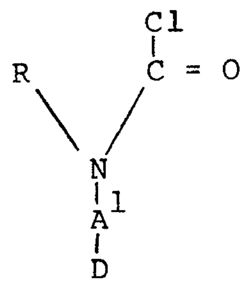

(V)

or- if in the compounds of general formula Ia R represents a hydrogen atom - an isocyanate of general formula Va

$$O = C = N - A^1 - D \quad (Va)$$

wherein the groups R, $A^1$ and D are defined as hereinbefore, preferably in solvents at temperatures up to the boiling point of the reaction mixture, or

c) in order to prepare the pyrrolo-condensed diazepinones of general formula Ib which come under general formula I

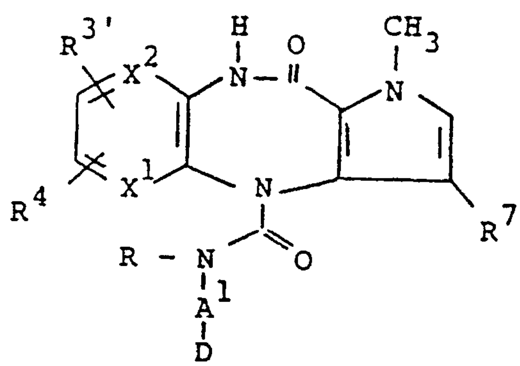

(Ib)

wherein

$R^4$, $X^1$, $X^2$, $A^1$ and D are defined as hereinbefore,

$R^{3'}$ represents an alkyl group with 1 to 4 carbon atoms or a hydrogen atom and

$R^7$ represents a hydrogen atom and for the group

D = 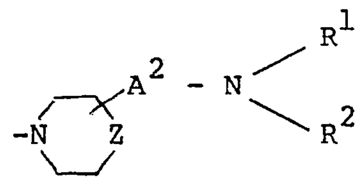

Z has the meanings given before with the exception of a sulphur atom and $R^1$, $R^2$ and $A^2$ are defined as hereinbefore,

a compound of general formula Ib wherein $R^7$ represents a chlorine atom is subjected to hydrogenolysis either with hydrogen in the presence of catalysts of metals of the VIIIth subsidiary group of the periodic table of elements under a hydrogen pressure of 1 to 300 bar, or with formic acid or trialkylammonium formate in the presence of palladium on charcoal or palladium acetate and triarylphosphines at temperatures between 0° C and 130° C and in the presence of a solvent,

d) in order to prepare the condensed diazepinones of general Ia described in a) with the above meanings for

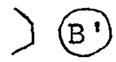

and for the substituents R, $R^3$, $R^4$, $X^1$, $X^2$, $A^1$ and D,
condensed diazepinones of general formula VIII

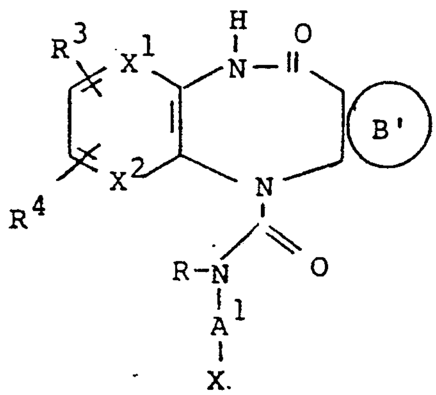

(VIII)

wherein
R, $R^3$, $R^4$, $X^1$, $X^2$ and $A^1$ are defined as hereinbefore and

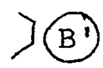

is defined as hereinbefore and X represents a nucleofugal group or leaving group, are reacted with diamines of general formula IIIc

$$H - D \qquad (IIIc)$$

wherein
D is defined as hereinbefore, in an inert solvent at temperatures between -10° C and the boiling point of the reaction mixture, optionally in the presence of an auxiliary base, and if X in the compound of general formula VIII represents the hydroxy group, in the presence of the catalysts of the VIIIth subsidiary group of the periodic table, preferably in the presence of aprotic inert solvents,

and if desired the resulting compounds of general formula I are resolved into their enantiomers and optionally the compounds of general formula I, if they are obtained in salt form, are converted into their free bases or, if they are obtained as free bases, are converted into their physiologically acceptable salts with inorganic or organic acids.

8. Process as claimed in claim 7a, characterised in that the reaction is carried out in the presence of aprotic polar solvents and in the presence of additional inorganic or organic bases or in the presence of an excess of a compound of general formula VIII whilst, if a metal compound of general formula IIIa is used, this is produced in situ from a compound of general formula III.

9. Process as claimed in claim 7b, characterised in that the reaction is carried out in an inert organic solvent, in the presence of tertiary organic bases and at temperatures of between 30 and l00° C.

10. Process as claimed in claim 7c, characterised in that the hydrogenolysis is carried out under unpressurized conditions with formic acid in the presence of dimethylformamide and palladium on charcoal at 70 to 110° C, with trimethylammonium formate in the presence of excess triethylamine and palladium on charcoal, or with palladium acetate and triarylphosphines at temperatures of between 40 and 110° C.

Claims for the following Ccntracting States : GR, ES

1. Process for preparing new condensed diazepinones of general formula I

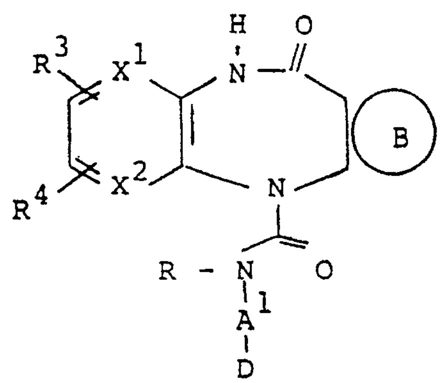

(I)

wherein

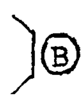

represents one of the divalent groups

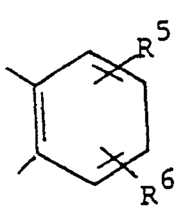 , 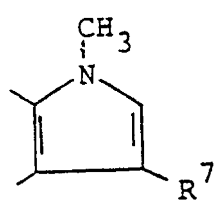 , 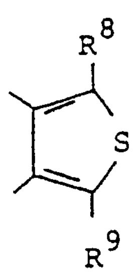 , 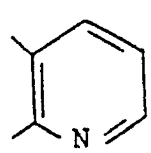 or 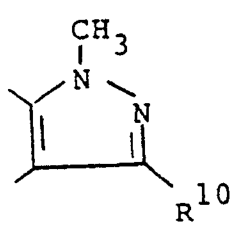

(S) (T) (U) (V) (W)

and D represents the groups

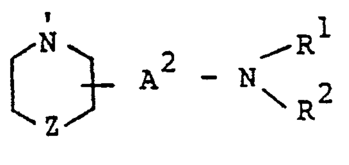,
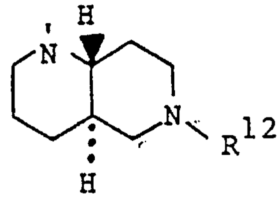,
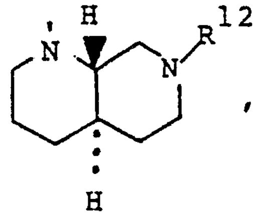,

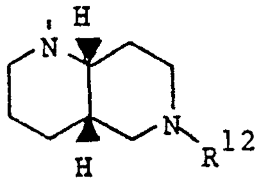
and
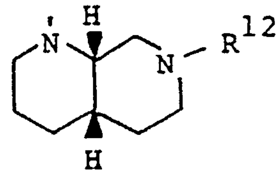

and $X^1$, $X^2$, $A^1$, $A^2$, R, $R^1$ to $R^{10}$, $R^{12}$ and Z have the following meanings:

$X^1$ and $X^2$ represent a =CH- group or, if

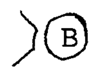

assumes the meanings of the above-mentioned divalent groups S, U or W, both or only $X^1$ or only $X^2$ may also represent a nitrogen atom;

$A^1$ is a straight-chained or branched saturated alkylene group with two to seven carbon atoms;

$A^2$ is a straight-chained or branched saturated alkylene group with 1 to 5 carbon atoms or, if it is in the 3-position relative to the nitrogen of the saturated heterocyclic ring, it may also represent a single bond;

Z represents a single bond, an oxygen or sulphur atom, a methylene or 1,2-ethylene group;

R represents a hydrogen atom or a methyl group;

$R^1$ represents a branched or unbranched alkyl group with 1 to 4 carbon atoms;

$R^2$ represents a branched or unbranched alkyl group with 1 to 7 carbon atoms which may optionally also be substituted by a hydroxy group at its second to seventh carbon atoms, or a cycloalkyl or cycloalkylmethyl group with 3 to 7 carbon atoms in the ring, whilst the cycloalkyl ring may optionally also be substituted by a hydroxy group;

$R^1$ and $R^2$ may, however, also form, together with the nitrogen atom between them, a 4- to 7-membered saturated, monocyclic, heterocyclic ring which may optionally be interrupted by an oxygen atom or by the $N-CH_3$ group;

$R^3$ is an alkyl group with 1 to 4 carbon atoms, a chlorine atom or a hydrogen atom;

$R^4$ represents a hydrogen atom or a methyl group;

$R^5$ and $R^6$ each represent a hydrogen atom, a fluorine, chlorine or bromine atom or an alkyl group with 1 to 4 carbon atoms;

$R^7$ represents a hydrogen or chlorine atom or a methyl group;

$R^8$ represents a hydrogen atom or an alkyl group with 1 to 4 carbon atoms;

$R^9$ represents a hydrogen or halogen atom or an alkyl group with 1 to 4 carbon atoms;

$R^{10}$ represents a hydrogen atom or a methyl group and

$R^{12}$ represents a branched or unbranched alkyl group with 1 to 6 carbon atoms,

whilst if

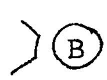

represents the divalent group T and $R^7$ is a hydrogen atom, $R^3$ cannot represent a chlorine atom and Z cannot represent a sulphur atom,

the diastereomers and enantiomers thereof and their physiologically acceptable salts with inorganic or organic acids,characterised in that

a) in order to prepare condensed diazepinones of general formula Ia

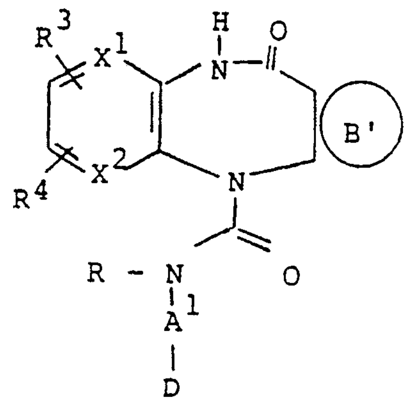

(Ia)

wherein R, $R^3$, $R^4$, $X^1$, $X^2$, $A^1$ and D are defined as hereinbefore and

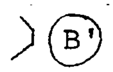

represents one of the divalent groups S, U, V, W or T'

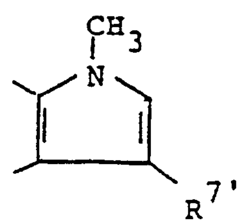

(T')

wherein $R^{7'}$ is a chlorine atom or a methyl group, a carbonic acid derivative of general formula II

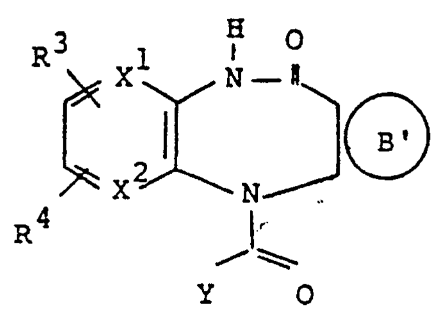

(II)

wherein
$R^3$, $R^4$,

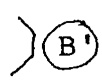

, $X^1$ and $X^2$ are defined as hereinbefore and Y represents a halogen atom or the group $OR^{11}$, wherein $R^{11}$ represents an optionally halogen substituted alkyl group with 1 to 5 carbon atoms, a phenyl group optionally substituted by halogen atoms or nitro groups or an aralkyl group with 7 to 15 carbon atoms, is reacted with a compound of general formula III or IIIa

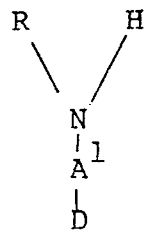

(III) or

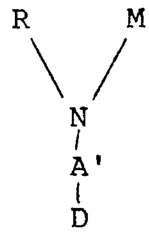

(IIIa)

wherein
R, $A^1$ and D are defined as hereinbefore, and M represents an alkali metal atom or 1 equivalent of an alkaline earth metal atom, optionally in the presence of a solvent at temperatures between -10° C and the boiling point of the reaction mixture, or

b) in order to prepare the same condensed diazepinones of general formula Ia with the above definitions for

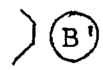

and the substituents R, $R^3$, $R^4$, $X^1$, $X^2$, $A^1$ and D, a tricyclic compound of general formula IV

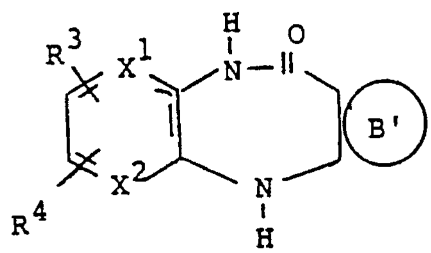

(IV)

wherein the groups $R^3$ , $R^4$ , $X^1$ , $X^2$ and

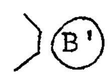

are defined as hereinbefore is reacted with a chlorocarbonic acid derivative of general formula V

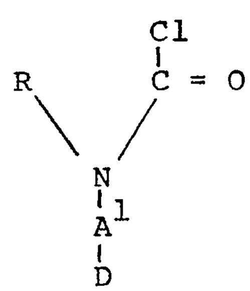

(V)

or- if in the compounds of general formula Ia R represents a hydrogen atom - an isocyanate of general formula Va

$$O = C = N - A^1 - D \qquad (Va)$$

wherein the groups R, $A^1$ and D are defined as hereinbefore, preferably in solvents at temperatures up to the boiling point of the reaction mixture, or

c) in order to prepare the pyrrolo-condensed diazepinones of general formula Ib which come under general formula I

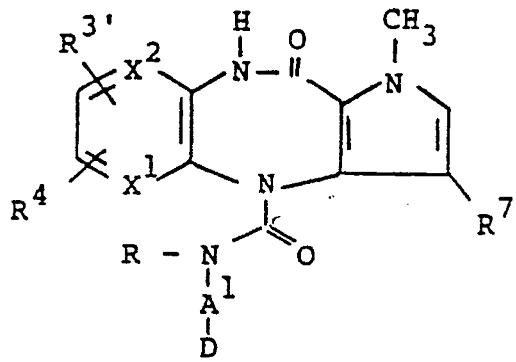

(Ib)

wherein

$R^4$, $X^1$, $X^2$, $A^1$ and D are defined as hereinbefore,

$R^{3'}$ represents an alkyl group with 1 to 4 carbon atoms or a hydrogen atom and

$R^7$ represents a hydrogen atom and for the group

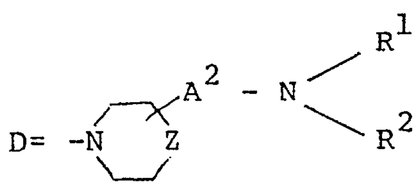

Z has the meanings given before with the exception of a sulphur atom and $R^1$, $R^2$ and $A^2$ are defined as hereinbefore,

a compound of general formula Ib wherein $R^7$ represents a chlorine atom is subjected to hydrogenolysis either with hydrogen in the presence of catalysts of metals of the VIIIth subsidiary group of the periodic table

of elements under a hydrogen pressure of 1 to 300 bar, or with formic acid or trialkylammonium formate in the presence of palladium on charcoal or palladium acetate and triarylphosphines at temperatures between 0° C and 130° C and in the presence of a solvent,

d) in order to prepare the condensed diazepinones of general Ia described in a) with the above meanings for

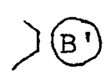

and for the substituents R, $R^3$, $R^4$, $X^1$, $X^2$, $A^1$ and D,

condensed diazepinones of general formula VIII

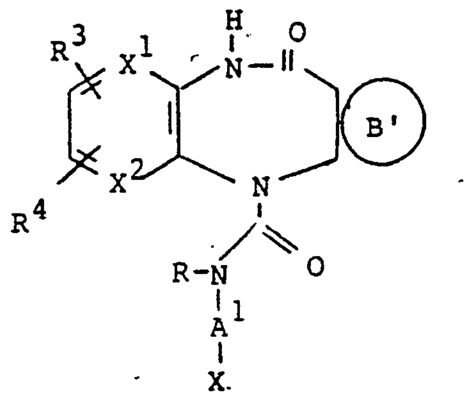

(VIII)

wherein
R, $R^3$, $R^4$, $X^1$, $X^2$ and $A^1$ are defined as hereinbefore and

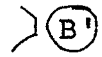

is defined as hereinbefore and X represents a nucleofugal group or leaving group, are reacted with diamines of general formula IIIc

H - D (IIIc)

wherein
D is defined as hereinbefore, in an inert solvent at temperatures between -10° C and the boiling point of the reaction mixture, optionally in the presence of an auxiliary base, and if X in the compound of general formula VIII represents the hydroxy group, in the presence of the catalysts of the VIIIth subsidiary group of the periodic table, preferably in the presence of aprotic inert solvents,

and if desired the resulting compounds of general formula I are resolved into their enantiomers and optionally the compounds of general formula I, if they are obtained in salt form, are converted into their free bases or, if they are obtained as free bases, are converted into their physiologically acceptable salts with inorganic or organic acids.

2. Process as claimed in claim 1a, characterised in that the reaction is carried out in the presence of aprotic polar solvents and in the presence of additional inorganic or organic bases or in the presence of an excess of a compound of general formula VIII whilst, if a metal compound of general formula IIIa is used, this is produced in situ from a compound of general formula III.

3. Process as claimed in claim 1b, characterised in that the reaction is carried out in an inert organic solvent, in the presence of tertiary organic bases and at temperatures of between 30 and 100° C.

4. Process as claimed in claim 1c, characterised in that the hydrogenolysis is carried out under unpressurized conditions with formic acid in the presence of dimethylformamide and palladium on charcoal at 70 to 110° C, with trimethylammonium formate in the presence of excess triethylamine and palladium on charcoal, or with palladium acetate and triarylphosphines at temperatures of between 40 and 110° C.

## Revendications

1. Nouvelles diazépinones condensées de formule générale I

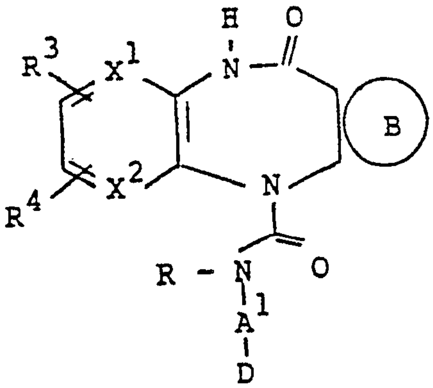

(I)

dans laquelle

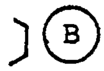

représente l'un des radicaux bivalents

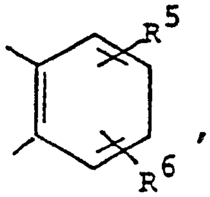

, 

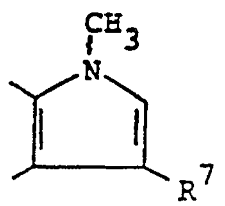

, 

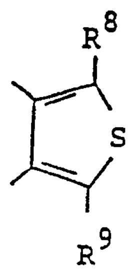

, 

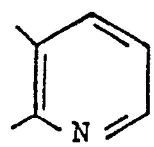

ou

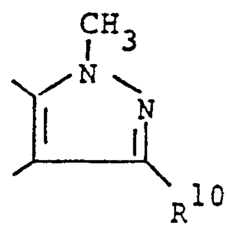

(S) (T) (U) (V) (W)

et D les groupes

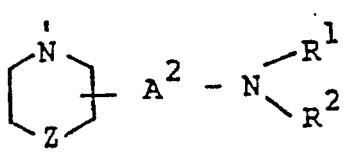

, 

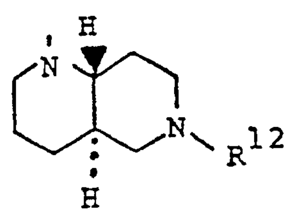

, 

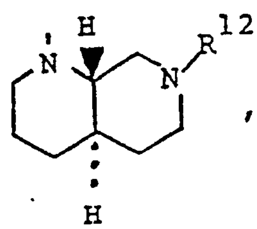

, 

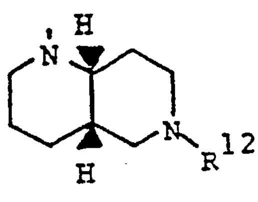

et

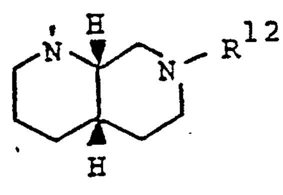

et

$X^1$, $X^2$, $A^1$, $A^2$, R, $R^1$ à $R^{10}$, $R^{12}$ et Z possèdent les significations suivantes

$X^1$ et $X^2$ représentent un groupe =CH- ou, dans la mesure où

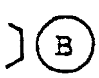

prend les significations des radicaux bivalents S, U ou W susmentionnés, peuvent l'un et l'autre ou seulement $X^1$ ou seulement $X^2$ être également un atome d'azote;

$A^1$ est un radical alcoylène saturé, rectiligne ou ramifié avec deux jusqu'à sept atomes de carbone;

$A^2$ est un radical alcoylène saturé, rectiligne ou ramifié, avec 1 à 5 atomes de carbone ou, lorsqu'il se trouve en position 3 par rapport à l'azote du cycle hétérocyclique saturé, également une liaison simple;

Z est une liaison simple, un atome d'oxygène ou de soufre, le groupe méthylène ou 1,2-éthylène;

R est un atome d'hydrogène ou un groupe méthyle;

$R^1$ est un radical alcoyle ramifié ou non ramifié avec 1 à 4 atomes de carbone;

$R^2$ est un radical alcoyle ramifié ou non ramifié avec 1 à 7 atomes de carbone, qui peut éventuellement être encore substitué sur son 2ème à 7ème atome de carbone par un groupe hydroxy, ou est un radical cycloalcoyle ou un radical cycloalcoylméthyle avec 3 à 7 atomes de carbone dans le cycle, le cycle cycloalcoyle pouvant éventuellement être encore substitué par un groupe hydroxy;

$R^1$ et $R^2$ peuvent toutefois aussi, ensemble avec l'atome d'azote intermédiaire, former un cycle hétérocyclique, monocyclique, saturé à 4 à 7 membres, qui peut éventuellemnt être interrompu par un atome d'oxygène ou par le groupe $N-CH_3$;

$R^3$ est un groupe alcoyle avec 1 à 4 atomes de C, un atome de chlore ou un atome d'hydrogène;

$R^4$ est un atome d'hydrogène ou un groupe méthyle;

$R^5$ et $R^6$ représentent chacun un atome d'hydrogène, un atome de fluor, de chlore ou de brome ou un groupe alcoyle avec 1 à 4 atomes de C;

$R^7$ représente un atome d'hydrogène ou de chlore ou un groupe méthyle;

$R^8$ représente un atome d'hydrogène ou un radical alcoyle avec 1 à 4 atomes de carbone;

$R^9$ représente un atome d'hydrogène ou d'halogène ou un groupe alcoyle avec 1 à 4 atomes de carbone; et

$R^{10}$ représente un atome d'hydrogène ou un groupe méthyle; et

$R^{12}$ représente un radical alcoyle ramifié ou non ramifié avec 1 à 6 atomes de carbone,

dans le cas où

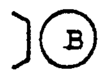

représente le radical bivalent T et $R^7$ est un atome d'hydrogène, $R^3$ ne peut pas représenter un atome de chlore et Z ne peut pas représenter un atome de soufre, leurs diastéréoisomères et énantiomères, et leurs sels physiologiquement supportables avec des acides non organiques ou organiques.

**2.** Nouvelles diazépinones condensées de formule générale I selon la revendication 1, dans laquelle

$X^1$ est un groupe =CH-,

$X^2$ est soit un atome d'azote, dans la mesure où

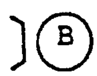

représente le radical bivalent S, étant entendu que $R^3$, $R^4$ et $R^5$ sont des atomes d'hydrogène et $R^6$ est un atome d'hydrogène, un atome de chlore ou de brome ou un groupe méthyle ou éthyle en position 8 ou 9 de l'hétérocycle,

soit un groupe =CH-, lorsque

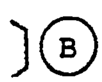

prend la signification du radical bivalent U, $R^8$ étant un atome d'hydrogène et $R^9$ un groupe méthyle;

$A^1$ représente un groupe 1,2-éthylène;

$A^2$ représente un groupe alcoylène rectiligne avec 1 à 5 atomes de carbone;

Z représente un groupe méthylène;

R représente un atome d'hydrogène et

$R^1$ et $R^2$ représentent des groupes alcoyle avec 1 à 3 atomes de carbone ou, ensemble avec l'atome d'azote intermédiaire, le radical pipéridinyle,

leurs diastéréoisomères et énantiomères, et leurs sels physiologiquement supportables avec des acides non organiques ou organiques.

3. Nouvelles diazépinones condensées de formule générale I selon la revendication 1, dans laquelle
$X^1$ représente =CH-,
$X^2$ représente un atome d'azote ou le groupe =CH-,

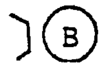

représente un radical S ou U bivalent,
$A^1$ représente le groupe éthylène,
$A^2$ représente le groupe méthylène ou 1,3-propylène,
Z représente le groupe méthylène,
R représente un atome d'hydrogène ou le groupe méthyle,
$R^1$ et $R^2$, qui peuvent être identiques ou différents, représentent un radical alcoyle avec 1 à 3 atomes de carbone,
$R^3$ et $R^4$ qui peuvent être identiques ou différents, représentent des atomes d'hydrogène ou le groupe méthyle,
$R^5$, $R^6$ représentent des atomes d'hydrogène,
$R^8$ et $R^9$ représentent des atomes d'hydrogène ou l'un de ces radicaux représente un groupe méthyle,
leurs diastéréoisomères et énantiomères, et leurs sels physiologiquement supportables avec des acides non organiques ou organiques.

4. En tant que nouveaux composés de formule générale I selon la revendication 1
la 5,11-dihydro-11-[[[2-[2-[(dipropylamino)méthyl]-1-pipéridinyl]éthyl]amino]carbonyl]-6H-pyrido[2,3-b][1,4]benzodiazépine-6-one,
la 4-[[[2-[2-[(diéthylamino)méthyl]-1-pipéridinyl]éthyl]amino]carbonyl]-4,9-dihydro-3-méthyl-10H-thiéno[3,4-b][1,5]benzodiazépine-10-one,
la 9-chloro-11-[[[2-[2-[(diéthylamino)méthyl]-1-pipéridinyl] éthyl]amino]carbonyl]-5,11-dihydro-6H-pyrido[2,3-b] [1,4]benzodiazépine-6-one,
leurs diastéréoisomères et énantiomères, et leurs sels physiologiquement supportables avec des acides non organiques ou organiques.

5. Médicament contenant un composé selon les revendications 1 à 4, conjointement à un ou plusieurs excipients et/ou adjuvants inertes.

6. Utilisation des composés selon les revendications 1 à 4 pour la fabrication de médicaments pour le traitement des bradycardies et des bradyarythmies.

7. Procédé pour la fabrication des nouvelles diazépinones condensées de formule générale I

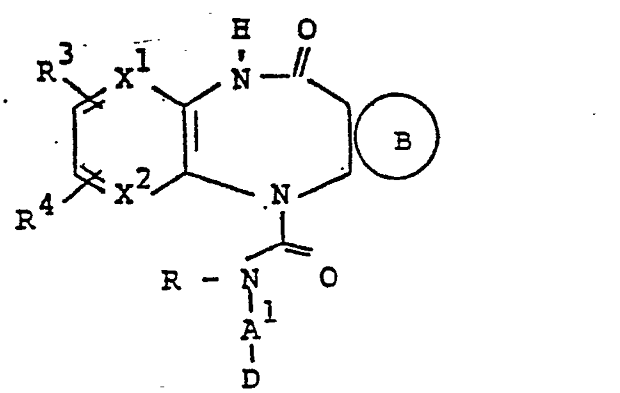

(I)

dans laquelle

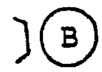

, D, $X^1$, $X^2$, $A^1$, $A^2$, R, $R^1$ à $R^{10}$, $R^{12}$ á Z sont définis comme indiqué à la revendication 1,

et de leurs diastéréoisomères ou énantiomères, et de leurs sels physiologiquement supportables avec des acides non organiques ou organiques, caractérisé en ce que

a.) pour la fabrication de diazépinones condensées de formule générale Ia

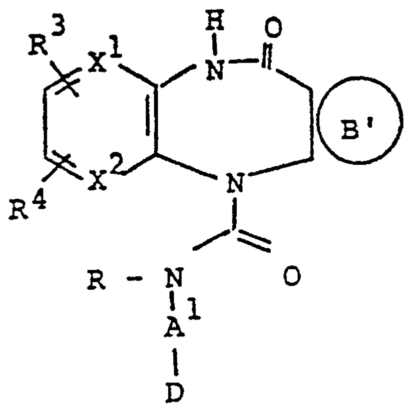

(Ia)

dans laquelle

R, $R^3$, $R^4$, $X^1$, $X^2$, $A^1$ et D ont les significations indiquées plus haut et

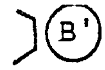

représente l'un des radicaux bivalents S, U, V, W ou T'

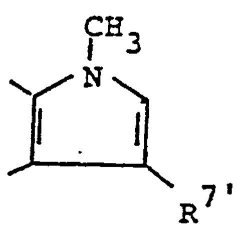

(T')

où $R^{7'}$ est un atome de chlore ou un groupe méthyle, on fait réagir un dérivé d'acide carboxylique de formule générale II

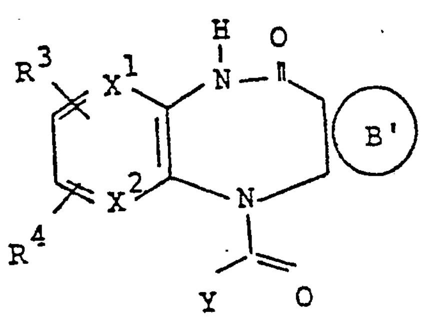

(II)

dans laquelle

$R^3$, $R^4$,

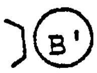

, $X^1$, $X^2$ sont définis comme plus haut, et Y représente un atome d'halogène ou le radical $OR^{11}$, $R^{11}$ représentant un radical alcoyle avec 1 à 5 atomes de carbone, éventuellement halogèno-substitué, un radical phényle éventuellement substitué par des atomes d'halogène ou des grcupes nitro ou un radical aralcoyle avec 7 à 15 atomes de carbone, avec un composé de formule genérale III ou IIIa,

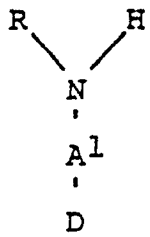 (III) ou 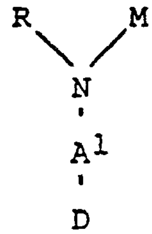 (IIIa)

dans lesquelles
A, $A^1$ et D sont définis comme au début et M représente un atome de métal alcalin ou un équivalent d'un atome de métal alcalino-terreux, éventuellement en présence d'un solvant à des températures entre -10 °C et la température d'ébullition du mélange réactionnel, ou

b.) pour la fabrication des mêmes diazépinones condensées de formule générale Ia avec les significations indiquées plus haut pour

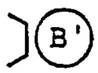

et les substituants R, $R^3$, $R^4$, $X^1$, $X^2$, $A^1$ et D, on fait réagir un composé tricyclique de formule générale IV

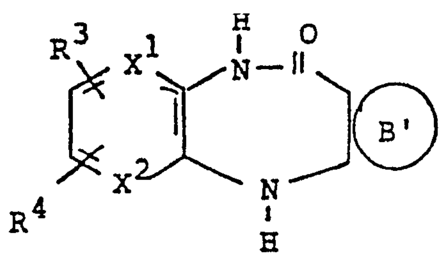 (IV)

dans laquelle
les radicaux $R^3$, $R^4$, $X^1$, $X^2$ et

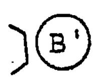

sont définis comme plus haut, avec un dérivé d'acide chlorocarboxylique de formule générale V

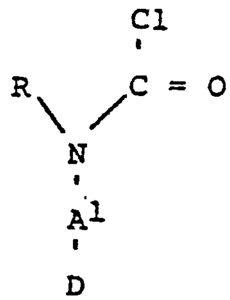

(V)

ou-dans la mesure où dans les composés de formule générale Ia, R doit être un atome d'hydrogène - également avec un isocyanate de formule générale Va

$$O = C = N - A^1 - D \qquad (Va)$$

dans lesquelles les radicaux R, $A^1$ et D ont les significations mentionnées plus haut, de préférence dans des solvants à des températures allant jusqu'à la température d'ébullition du mélange réactionnel, ou

c.) pour la fabrication de diazépinones pyrrolo-condensées de formule générale Ib entrant dans le cadre de la formule générale Ib

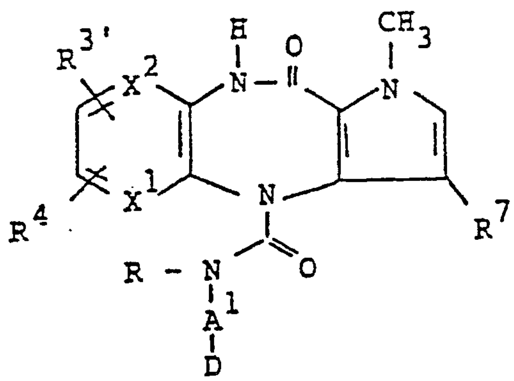

(Ib)

dans laquelle
$R^4$, $X^1$, $X^2$, $A^1$ et D ont les significations mentionnées au début, $R^{3'}$ représente un groupe alcoyle avec 1 à 4 atomes de C ou un atome d'hydrogène, $R^7$ représente un atome d'hydrogène et pour le groupe

D = 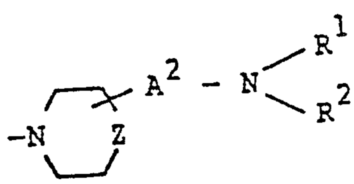

Z a les significations mentionnées au début, à l'exception d'un atome de soufre, $R^1$, $R^2$ et $A^2$ étant définis comme plus haut, on soumet un composé de formule générale Ib, dans laquelle $R^7$ représente un atome de chlore, à une hydrogénolyse soit au moyen d'hydrogène en présence de catalyseurs de métaux du VIIIème sous-groupe du système périodique des éléments sous une pression d'hydrogène de 1 à 300 bars, soit au moyen d'acide formique ou de formiate de trialcoylammonium en présence de palladium sur charbon ou d'acétate de palladium et de triarylphosphines à des températures entre 0 °C jusqu'à 130 °C et en présence d'un solvant,

d.) pour la fabrication des diazépinones condensées décrites sous a) , de formule générale Ia avec les significations indiquées ci-dessus pour

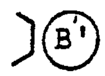

et pour les substituants R, $R^3$, $R^4$, $X^1$, $X^2$, $A^1$ et D, on fait réagir des diazépinones condensées de formule générale VIII

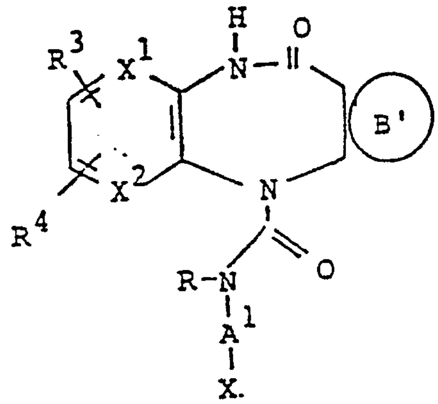

(VIII)

dans laquelle
R, $R^3$, $R^4$, $X^1$, $X^2$ et $A^1$ ont les significations indiquées et

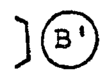

est défini comme plus haut et X est un groupe nucléofuge ou respectivement un groupe partant, avec des diamines de formule générale IIIc

$$H - D \qquad (IIIc)$$

dans laquelle
D a les significations mentionnées au début, dans un solvant inerte à des températures entre -10 ° C et la température d'ébullition du mélange réactionnel, éventuellement en présence d'une base adjuvante, et pour le cas où X dans le composé de formule générale VIII représente le groupe hydroxy, en présence de catalyseurs du VIIIème sous-groupe du système périodique, de préférence en présence de solvants aprotiques, inertes, et, si on le désire, on sépare les composés de formule générale I ainsi obtenus en leurs énantiomères, et, éventuellement, on transforme les composés obtenus de formule générale I, lorsqu'ils se présentent sous forme de sels, en leurs bases libres ou, pour le cas où ils se présentent sous forme de bases libres, en leurs sels physiologiquement supportables avec des acides non organiques ou organiques.

**8.** Procédé selon la revendication 7a, caractérisé en ce que la réaction est effectuée en présence de solvants aprotiques polaires et en présence de bases non organiques ou organiques supplémentaires ou en présence d'un excès d'un composé de formule générale III, dans le cas où un composé métallique de formule générale IIIa est utilisé, celui-ci étant produit in situ à partir d'un composé de formule générale III.

**9.** Procédé selon la revendication 7b, caractérisé en ce que la réaction a lieu dans un solvant organique inerte, en présence de bases organiques tertiaires et à des températures entre 30 et 100 ° C.

**10.** Procédé selon la revendication 7c, caractérisé en ce que l'hydrogénolyse est effectuée sans pression au moyen d'acide formique en présence de diméthylformamide et de palladium sur charbon à 70 jusqu'à 110 ° C, au moyen de formiate de triméthylammonium en présence de triéthylamine en excès et de palladium sur charbon, ou au moyen d'acétate de palladium et de triarylphosphines à des températures entre 40 et 110 ° C.

Revendications pour les Etats contractants suivants : GR, ES

1. Procédé pour la fabrication de nouvelles diazépinones condensées de formule générale I

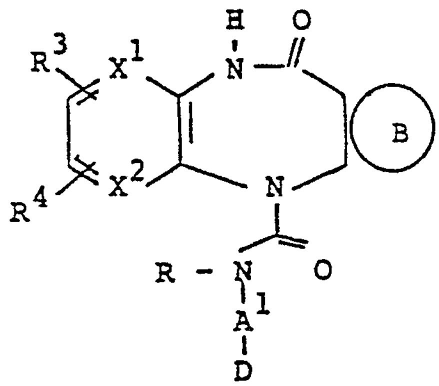

(I)

dans laquelle

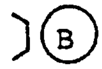

représente un radical bivalent

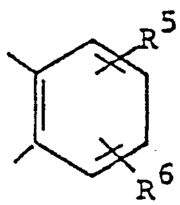 , 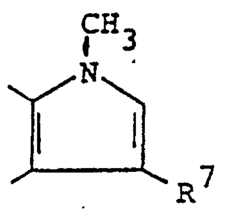 , 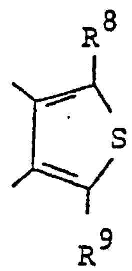 , 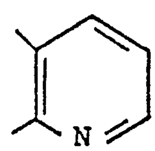 ou 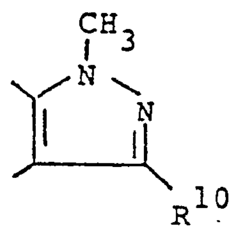

(S) (T) (U) (V) (W)

et D représente les groupes

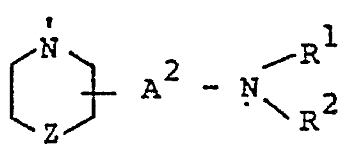 , 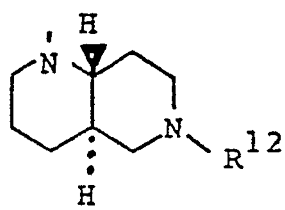 , 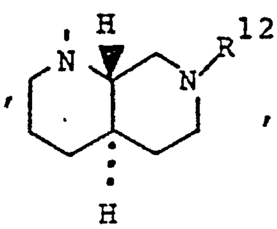 ,

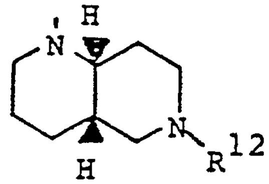 et 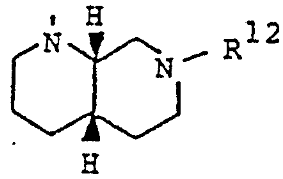

et

$X^1$, $X^2$, $A^1$, $A^2$, R, $R^1$ à $R^{10}$, $R^{12}$ et Z possèdent les significations suivantes:

$X^1$ et $X^2$ représentent un groupe =CH- ou, dans la mesure où

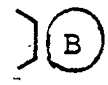

prend les significations des radicaux S, U ou W bivalents mentionnés plus haut, peuvent l'un et l'autre ou seulement $X^1$ ou seulement $X^2$ être également un atome d'azote;

$A^1$ est un radical alcoylène saturé, rectiligne ou ramifié avec deux jusqu'à sept atomes de carbone;

$A^2$ est un radical alcoylène saturé, rectiligne ou ramifié, avec 1 à 5 atomes de carbone ou, lorsqu'il se trouve en position 3 par rapport à l'atome d'azote du cycle hétérocyclique saturé, également une liaison simple;

Z est une liaison simple, un atome d'oxygène ou de soufre, le groupe méthylène ou 1,2-éthylène;

R est un atome d'hydrogène ou un groupe méthyle;

$R^1$ est un radical alcoyle ramifié ou non ramifié avec 1 à 4 atomes de carbone;

$R^2$ est un radical alcoyle ramifié ou non ramifié avec 1 à 7 atomes de carbone, qui peut éventuellement être encore substitué sur son atome de carbone 2 à 7 par un groupe hydroxy, ou est un radical cycloalcoyle ou un radical cycloalcoylméthyle avec 3 à 7 atomes de carbone dans le cycle, le cycle cycloalcoyle pouvant éventuellement être encore substitué par un groupe hydroxy;

$R^1$ et $R^2$ peuvent cependant aussi, ensemble avec l'atome d'azote intermédiaire, former un cycle hétérocyclique, monocyclique, saturé à 4 à 7 membres, qui peut éventuellemnt être interrompu par un atome d'oxygène ou par le groupe $N\text{-}CH_3$;

$R^3$ est un groupe alcoyle avec 1 à 4 atomes de C, un atome de chlore ou un atome d'hydrogène;

$R^4$ est un atome d'hydrogène ou un groupe méthyle;

$R^5$ et $R^6$ signifient chacun un atome d'hydrogène, un atome de fluor, de chlore ou de brome ou un groupe alcoyle avec 1 à 4 atomes de C;

$R^7$ représente un atome d'hydrogène ou de chlore ou un groupe méthyle;

$R^8$ représente un atome d'hydrogène ou un radical alcoyle avec 1 à 4 atomes de carbone;

$R^9$ représente un atome d'hydrogène ou d'halogène ou un groupe alcoyle avec 1 à 4 atomes de carbone;

$R^{10}$ représente un atome d'hydrogène ou un groupe méthyle; et

$R^{12}$ représente un radical alcoyle ramifié ou non ramifié avec 1 à 6 atomes de carbone, dans le cas où

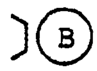

représente le radical bivalent T et $R^7$ est un atome d'hydrogène, $R^3$ ne pouvant signifier un atome de chlore et Z ne pouvant signifier un atome de soufre,

et de leurs diastéréoisomères ou énantiomères, et de leurs sels physiologiquement supportables avec des acides non organiques ou organiques, caractérisé en ce que

a.) pour la fabrication de diazépinones condensées de formule générale Ia

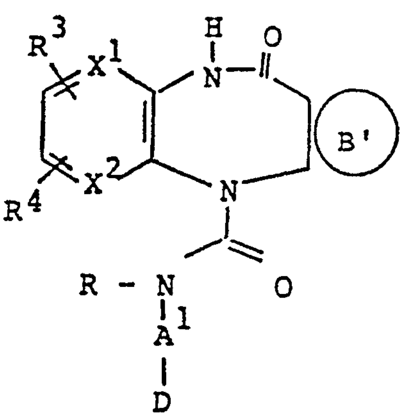

(Ia)

dans laquelle

$R$, $R^3$, $R^4$, $X^1$, $X^2$, $A^1$ et D ont les significations indiquées plus haut et

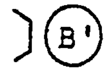

représente l'un des radicaux bivalents S, U, V, W ou T'

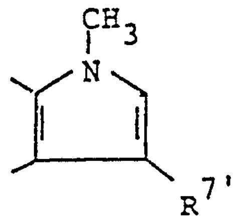

(T')

où $R^{7'}$ est un atome de chlore ou un groupe méthyle, on fait réagir un dérivé d'acide carboxylique de formule générale II

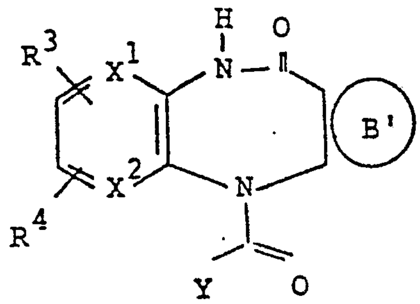

(II)

dans laquelle
$R^3$, $R^4$,

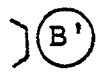

, $X^1$, $X^2$ sont définis comme plus haut, et Y représente un atome d'halogène ou le radical $OR^{11}$, $R^{11}$ représentant un radical alcoyle avec 1 à 5 atomes de carbone, éventuellement halogèno-substitué, un radical phényle éventuellement substitué par des atomes d'halogène ou des groupes nitro ou un radical aralcoyle avec 7 à 15 atomes de carbone, avec un composé de formule générale III ou IIIa,

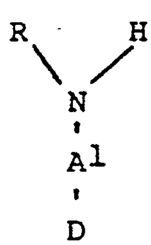

(III) ou

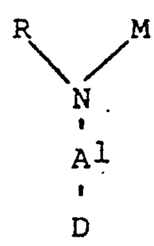

(IIIa)

dans lesquelles
R, $A^1$ et D sont définis comme au début et M représente un atome de métal alcalin ou un équivalent d'un atome de métal alcalino-terreux, éventuellement en présence d'un solvant à des températures entre -10 °C et la température d'ébullition du mélange réactionnel, ou

b.) pour la fabrication des mêmes diazépinones condensées de formule générale Ia avec les significations indiquées plus haut pour

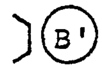

et les substituants R, $R^3$, $R^4$, $X^1$, $X^2$, $A^1$ et D, on fait réagir un composé tricyclique de formule générale IV

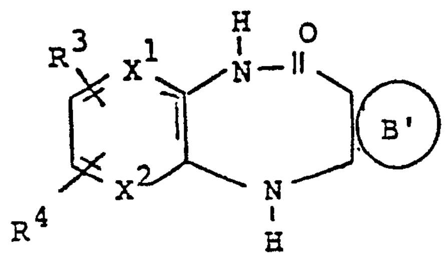

(IV)

dans laquelle
les radicaux $R^3$, $R^4$, $X^1$, $X^2$ et

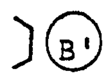

sont définis comme plus haut, avec un dérivé d'acide chlorocarboxylique de formule générale V

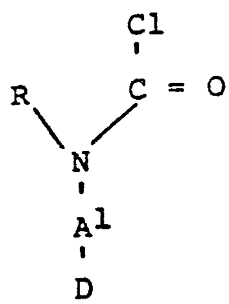

(V)

ou - dans la mesure où dans les composés de formule générale Ia, R doit être un atome d'hydrogène - également avec un isocyanate de formule générale Va

$$O = C = N - A^1 - D \qquad (Va)$$

dans lesquelles les radicaux R, $A^1$ et D ont les significations mentionnées plus haut, de préférence dans des solvants à des températures allant jusqu'à la température d'ébullition du mélange réactionnel, ou

c.) pour la fabrication de diazépinones pyrrolo-condensées de formule générale Ib entrant dans le cadre de la formule générale Ib

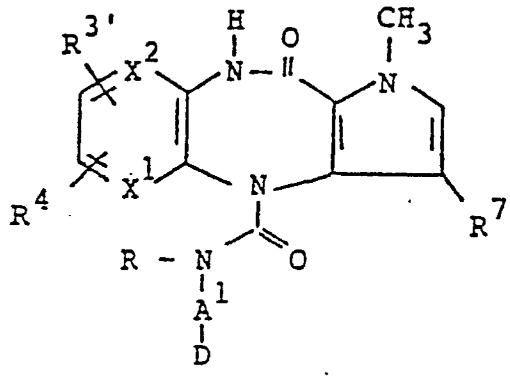

(Ib)

dans laquelle
$R^4$, $X^1$, $X^2$, $A^1$ et D ont les significations mentionnées au début, $R^{3'}$ représente un groupe alcoyle avec 1 à 4 atomes de C ou un atome d'hydrogène, $R^7$ représente un atome d'hydrogène et pour le groupe

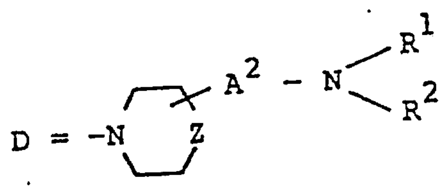

Z a les significations mentionnées au début, à l'exception d'un atome de soufre, $R^1$, $R^2$ et $A^2$ étant définis comme plus haut, on soumet un composé de formule générale Ib, dans laquelle $R^7$ représente un atome de chlore, à une hydrogénolyse soit au moyen d'hydrogène en présence de catalyseurs de métaux du VIIIème sous-groupe du système périodique des éléments sous une pression d'hydrogène de 1 à 300 bars, soit au moyen d'acide formique ou de formiate de trialcoylammonium en présence de palladium sur charbon ou d'acétate de palladium et de triarylphosphines à des températures entre 0° C jusqu'à 130° C et en présence d'un solvant,

d.) pour la fabrication des diazépinones condensées décrites sous a), de formule générale Ia avec les significations indiquées ci-dessus pour

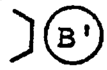

et pour les substituants R, $R^3$, $R^4$, $X^1$, $X^2$, $A^1$ et D, on fait réagir des diazépinones condensées de formule générale VIII

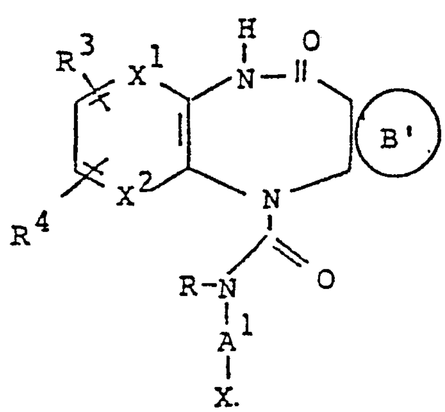

(VIII)

dans laquelle
R, $R^3$, $R^4$, $X^1$, $X^2$, et $A^1$ ont les significations indiquées et

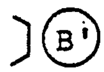

est défini comme plus haut et X est un groupe nucléofuge ou respectivement un groupe partant, avec des diamines de formule générale IIIc

$$H - D \qquad (IIIc)$$

dans laquelle
D a les significations mentionnées au début, dans un solvant inerte à des températures entre -10° C et la température d'ébullition du mélange réactionnel, éventuellement en présence d'une base adjuvante, et pour le cas où X dans le composé de formule générale VIII représente le groupe hydroxy, en présence de catalyseurs du VIIIème sous-groupe du système périodique, de préférence en présence de solvants aprotiques, inertes, et, si on le désire, on sépare les composés de formule générale I ainsi obtenus en leurs énantiomères, et, éventuellement, on transforme les composés obtenus de formule générale I, lorsqu'ils se présentent sous forme de sels, en leurs bases libres ou, pour le cas où ils se présentent sous forme de bases libres, en leurs sels physiologiquement supportables avec des acides non organiques ou organiques.

2. Procédé selon la revendication 1a, caractérisé en ce que la réaction est effectuée en présence de solvants aprotiques polaires et en présence de bases non organiques ou organiques supplémentaires ou en présence d'un excès d'un composé de formule générale III, dans le cas où un composé métallique de formule générale IIIa est utilisé, celui-ci étant produit in situ à partir d'un composé de formule générale III.

3. Procédé selon la revendication 1b, caractérisé en ce que la réaction a lieu dans un solvant organique inerte, en présence de bases organiques tertiaires et à des températures entre 30 et 100° C.

4. Procédé selon la revendication 1c, caractérisé en ce que l'hydrogénolyse est effectuée sans pression au moyen d'acide formique en présence de diméthylformamide et de palladium sur charbon à 70 jusqu'à 110° C, au moyen de formiate de triméthylammonium en présence de triéthylamine en excès et de palladium sur charbon, ou au moyen d'acétate de palladium et de triarylphosphines à des températures entre 40 et 110° C.